# FASCICULE DE BREVET EUROPEEN

(11) **EP 1 856 043 B1**
(45) Date de publication et mention de la délivrance du brevet: **08.06.2011**
(21) Numéro de dépôt: 06709213.0
(22) Date de dépôt: 01.02.2006
(51) Int. Cl.: C07D 207/34, C07D 233/90, A61K 31/4164, A61K 31/40, A61P 35/00

(54) **1-H-PYROLE-2-CARBOXAMIDES ET IMIDAZOLE-5-CARBOXAMIDES ET LEUR UTILISATION EN TANT MODULATEURS DES KINASES FAK, KDR ET TIE2 POUR TRAITER LE CANCER**
1H-PYRROL-2-CARBONSÄUREAMIDE UND IMIDAZOL-5-CARBONSÄUREAMIDE UND DEREN VERWENDUNG ALS FAK-, KDR- UND TIE2-KINASE-MODULATOREN ZUR BEHANDLUNG VON KREBS
1-H-PYRROLE-2-CARBOXAMIDES AND IMIDAZOLE-5-CARBOXAMIDES AND USE THEREOF AS FAK, KDR AND TIE2 KINASE MODULATORS FOR THE TREATMENT OF CANCER

(30) Priorité: 03.02.2005 FR 0501092
(43) Date de publication de la demande: 21.11.2007
(73) Titulaire: Aventis Pharma S.A., 92160 Antony (FR)
(72) Inventeur: RONAN, Baptiste, F-92140 Clamart (FR); HALLEY, Frank, F-92310 Chaville (FR); SOUAILLE, Catherine, F-94600 CHOISY LE ROI (FR); VIVIANI, Fabrice, F-95380 Louvres (FR); EL-AHMAD, Youssef, F-94000 Créteil (FR); BACQUE, Eric, F-91190 Gif sur Yvette (FR); TABART, Michel, F-91290 La Norville (FR)
(74) Mandataire: Domenego, Bertrand
(86) Numéro de dépôt international: PCT/FR2006/000220
(87) Numéro de publication internationale: WO 2006/082309

(56) Documents cités:
- WO-A-03/072541
- WO-A-2005/049603
- US-A1- 2004 138 269

## Description

La présente invention concerne notamment de nouveaux composés chimiques, particulièrement de nouveaux pyroles et imidazoles substitués, les compositions les contenant, et leur utilisation comme médicaments.

Plus particulièrement, l'invention concerne de nouveaux pyroles et imidazoles spécifiques présentant une activité anticancéreuse, via la modulation de l'activité de protéines, en particulier des kinases.

A ce jour, la plupart des composés commerciaux utilisés en chimiothérapie posent des problèmes importants d'effets secondaires et de tolérance par les patients. Ces effets pourraient être limités dans la mesure où les médicaments utilisés agissent sélectivement sur les cellules cancéreuses, à l'exclusion des cellules saines. Une des solutions pour limiter les effets indésirables d'une chimiothérapie peut donc consister en l'utilisation de médicaments agissant sur des voies métaboliques ou des éléments constitutifs de ces voies, exprimés majoritairement dans les cellules cancéreuses, et qui ne seraient pas ou peu exprimés dans les cellules saines.

Les protéines kinases sont une famille d'enzymes qui catalysent la phosphorylation de groupes hydroxyles de résidus spécifiques de protéines tels que des résidus tyrosine, sérine ou thréonine. De telles phosphorylations peuvent largement modifier la fonction des protéines ; ainsi, les protéines kinases jouent un rôle important dans la régulation d'une grande variété de processus cellulaires, incluant notamment le métabolisme, la prolifération cellulaire, la différentiation cellulaire, la migration cellulaire ou la survie cellulaire. Parmi les différentes fonctions cellulaires dans lesquelles l'activité d'une protéine kinase est impliquée, certains processus représentent des cibles attractives pour traiter les maladies cancéreuses ainsi que d'autres maladies.

La demande de brevet US 2004/138269 décrit des pyrroles substitués de formule générale suivante

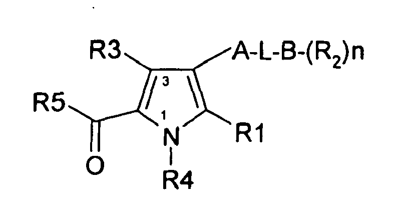

Où le pyrrole n'est jamais substitué en position 3 (R3) par un groupe aryle mais uniquement par H ou alkyle. Les radicaux R1 pouvant être H, R5 pouvant représenter NR2, et A-L-B pouvant représenter un groupe diarylaminourée.

La demande de brevet WO03/072541 décrit des pyrazoles de formule générale suivante 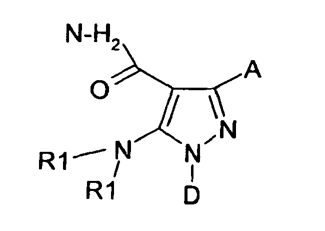 ne comportant jamais directement lié sur le noyau une diarylurée.

Ainsi, un des objets de la présente invention est de proposer des compositions ayant une activité anticancéreuse, en agissant en particulier vis-à-vis de kinases. Parmi les kinases pour lesquelles une modulation de l'activité est recherchée, FAK, KDR et Tie2 sont préférées.

Ces produits répondent à la formule (I) suivante : 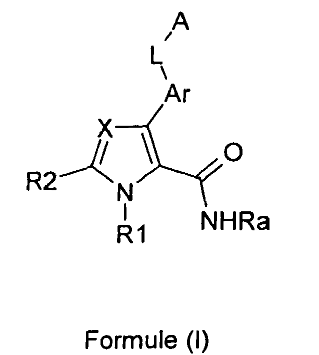 dans laquelle :
1) Ar-L-A est: 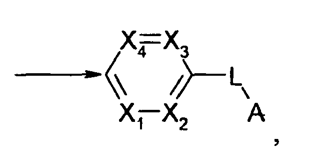 dans lequel chaque X1, X2, X3 et X4 est N ou CH ;
2) L est NH-CO-NH;
3) A est phényle, pyrazole ou isoxazolyl éventuellement substitué ;
4) Ra est H ;
5) R 1 est H ;
6) X est choisi parmi CR3 et N ;
7) R2 est un atome d'hydrogène
8) R3 est sélectionné dans le groupe constitué par : H, halogène, R'2, CN, O(R'2), OC(O)(R'2), OC(O)N(R'2)(R'3), OS(O₂)(R'2), N(R'2)(R'3), N=C(R'2)(R'3), N(R'2)C(O)(R'3), N(R'2)C(O)O(R'3), N(R'4)C(O)N(R'2)(R'3), N(R'4)C(S)N(R'2)(R'3), N(R'2)S(O₂)(R'3), C(O)(R'2), C(O)O(R'2), C(O)N(R'2)(R'3), C(=N(R'3))(R'2), C(=N(OR'3))(R'2), S(R'2), S(O)(R'2), S(O₂)(R'2), S(O₂)O(R'2), S(O₂)N(R'2)(R'3) ; dans lequel chaque R'2, R'3, R'4 est indépendamment sélectionné dans le groupe constitué par H, alkyle, alkylène, alkynyle, aryle, hétéroaryle, cycloalkyle, hétérocyclyle, alkyle substitué, alkylène substitué, alkynyle substitué, aryle substitué, hétéroaryle substitué, cycloalkyle substitué, hétérocyclyle substitué; dans lequel, lorsque R'2 et R'3 sont chacun différents de H et simultanément présents sur R2 ou sur R3, ils peuvent être liés entre eux pour former un cycle contenant de 0 à 3 hétéroatomes choisis parmi O, S et N et caracterisé en ce qu'au moins l'un des R₂ est R3 est un halogéne.

Des produits préférés selon l'invention sont ceux pour lesquels X est CR3.

R3 est aussi préférentiellement sélectionné dans le groupe constitué par H, halogène, NH₂, N(R'2)C(O)(R'3), N(R'2)C(O)O(R'3), N(R'4)C(O)N(R'2)(R'3), C(O)O(R'2), C(O)N(R'2)(R'3); dans lequel chaque R'2, R'3 est indépendamment sélectionné dans le groupe constitué par H, alkyle, alkylène, alkynyle, aryle, hétéroaryle, cycloalkyle, hétérocyclyle, alkyle substitué, alkylène substitué, alkynyle substitué, aryle substitué, hétéroaryle substitué, cycloalkyle substitué, hétérocyclyle substitué; dans lequel, lorsque R'2 et R'3 sont chacun différents de H et simultanément présents sur R2 ou sur R3, ils peuvent être liés entre eux pour former un cycle contenant de 0 à 3 hétéroatomes choisis parmi O, S et N.

R3 est plus préférentiellement sélectionné dans le groupe constitué constitué par H, halogène, NH₂, NH-COO-alkyle, -NH-CO-NH-aryle où l'aryle est non substitué ou substitué par un ou plusieurs halogène ou alkyle halogéné, -NH-CO-alkyle, -NH-CO-alkyle-N(alkyle)(alkyle'), -COOH, -COO-alkyle, -COO-alkyle-N(alkyle)(alkyle'), -CO-NH-alkyle-aryle où l'aryle est non substitué ou substitué par un ou plusieurs alcoxy, -CONH₂, -CO-hétérocyclyle où l'hétérocyclycle est non substitué ou substitué par un ou plusieurs alkyles.

Le substituant A est très avantageusement substitué par un premier substituant sélectionné dans le groupe constitué par alkyle, alkyle substitué, alkylène, alkynyle, aryle, hétéroaryle, O-alkyle, O-alkyle substitué, O-Aryle, O-hétéroaryle, S-alkyle, S-alkyle substitué, S-Aryle, S-hétéroaryle, chacun étant éventuellement substitué par un substituant choisi parmi (C1-C3)alkyle, halogène, O-(C1-C3)alkyle.

Le substituant A est préférentiellement substitué par un deuxième substituant sélectionné dans le groupe constitué par F, Cl, Br, I, OH, SH, SO₃M, COOM, CN, NO₂, CON(R8)(R9), N(R8)CO(R9), (C1-C3)alkyle-OH, (C1-C3)alkyleN(R8)(R9), (C1-C3)alkyle-(R10), (C1-C3)alkyle-COOH, N(R8)(R9); dans lequel R8 et R9 sont indépendamment choisis parmi H, (C1-C3)alkyle, (C1-C3)alkyle halogéné, (C1-C3)alkyleOH, (C1-C3)alkyleNH₂, (C1-C3)alkyleCOOM, (C1-C3)alkyleSO₃M ; dans lequel lorsque R8 et R9 sont simultanément différents de H, ils peuvent être liés pour former un cycle de 5 à 7 chaînons contenant éventuellement de 1 à 3 hétéroatomes ; dans lequel M est H ou un cation de métal alcalin choisi parmi Li, Na et K ; et dans lequel R10 est H ou un hétérocycle non aromatique éventuellement substitué, comprenant 2 à 7 atomes de carbone, et 1 à 3 hétéroatomes choisis parmi N, OetS.

Des substituants A particulièrement préférés sont choisis parmi phényle, pyrazolyle et isoxazolyle ; lesdits substituants A pouvant être substitués par halogène, (C1-C4)alkyle, (C1-C3)alkyle halogéné, O-(C1-C4)alkyle, S-(C1-C4)alkyle, O-(C1-C4)alkyle halogéné, et S-(C1-C4)alkyle halogéné. Lorsque A est disubstitué, les deux substituants de A peuvent former un cycle de 5 à 7 chaînons contenant éventuellement de 1 à 3 hétéroatomes.

D'autres substituants A préférés sont substitués par un ou plusieurs substituants, identiques ou différents, indépendamment sélectionnés dans le groupe constitué par F, Cl, Br, I, OH, SH, SO₃M, COOM, CN, NO₂, CON(R8)(R9), N(R8)CO(R9), (C1-C3)alkyle-OH, (C1-C3)alkyle-N(R8)(R9), (C1-C3)alkyle-(R10), (C1-C3)alkyle-COOH, N(R8)(R9), (C1-C6)alkyle, (C2-C6)alkylène, (C2-C6)alkynyle, aryle, hétéroaryle, O-(C1-C6)alkyle, O-Aryle, O-hétéroaryle, S-(C1-C6)alkyle, S-Aryle, S-hétéroaryle, chacun étant éventuellement substitué par un ou plusieurs substituants choisis parmi (C1-C3)alkyle, halogène, O-(C1-C3)alkyle; dans lequel R8 et R9 sont indépendamment choisis parmi H, (C1-C3)alkyle, (C1-C3)alkyleOH, (C1-C3)alkyleNH₂, (C1-C3)alkyleCOOM, (C1-C3)alkyleSO₃M; dans lequel lorsque R8 et R9 sont simultanément différents de H, ils peuvent être liés pour former un cycle de 5 à 7 chaînons comportant de 0 à 3 hétéroatomes choisis parmi O, N et S; dans lequel M est H ou un cation de métal alcalin choisi parmi Li, Na et K ; et dans lequel R10 est H ou un hétérocycle non aromatique éventuellement substitué, comprenant 2 à 7 atomes de carbone, et 1 à 3 hétéroatomes choisis parmi N, O et S.

Des produits préférés selon la présente invention sont choisis parmi :
3-{4-[3-(2-Fluoro-5-trifluorométhyl-phényl)-uréido]-phényl}-1H-pyrrole-2-carboxamide,
4-*tert*-Butyloxycarbonylamino-3-{4-[3-(2-fluoro-5-trifluorométhyl-phényl)-uréido]-phényl}-1H-pyrrole-2-carboxamide,
Chlorhydrate du 4-amino-3-{4-[3-(2-fluoro-5-trifluorométhyl-phényl)-uréido]-phényl}-1H-pyrrole-2-carboxamide,
4-[3-(2-Fluoro-5-trifluorométhyl-phényl)-uréido]-3-{4-[3-(2-fluoro-5-trifluorométhyl-phényl)-uréido]-phényl}-1H-pyrrole-2-carboxamide,
4-Acétylamino-3-{4-[3-(2-fluoro-5-trifluorométhyl-phényl)-uréido]-phényl}-1H-pyrrole-2-carboxamide,
4-(2-Diméthylamino-acétylamino)-3-{4-[3-(2-fluoro-5-trifluorométhyl-phényl)-uréido]-phényl}-1H-pyrrole-2-carboxamide,
4-Benzoylamino-3-{4-[3-(2-fluoro-5-trifluorométhyl-phényl)-uréido]-phényl}-1H-pyrrole-2-carboxamide,
4-Nicotinoylamino-3-{4-[3-(2-fluoro-5-trifluorométhyl-phényl)-uréido]-phényl}-1H-pyrrole-2-carboxamide,
5-Carbamoyl-4-{4-[3-(2-fluoro-5-trifluorométhyl-phényl)-uréido]-phényl}-1H-pyrrole-3-carboxylate d'éthyle,
5-Carbamoyl-4-{4-[3-(2-fluoro-phényl)-uréido]-phényl}-1H-pyrrole-3-carboxylate d'éthyle,
5-Carbamoyl-4-{4-[3-(2-méthoxy-phényl)uréido]-phényl}-1H-pyrrole-3-carboxylate d'éthyle,
5-Carbamoyl-4-{4-[3-(2-trifluorométhyl-phényl)-uréido]-phényl}-1H-pyrrole-3-carboxylate d'éthyle,
5-Carbamoyl-4-[4-(3-o-tolyl-uréido)-phényl]-1H-pyrrole-3-carboxylate d'éthyle,
5-Carbamoyl-4-{4-[3-(3-fluoro-phényl)-uréido]-phényl}-1H-pyrrole-3-carboxylate d'éthyle,
5-Carbamoyl-4-{4-[3-(3-méthoxy-phényl)-uréido]-phényl}-1H-pyrrole-3-carboxylate d'éthyle,
5-Carbamoyl-4-{4-[3-(3-trifluorométhyl-phényl)-uréido]-phényl}-1H-pyrrole-3-carboxylate d'éthyle,
5-Carbamoyl-4-[4-(3-m-tolyl-uréido)-phényl]-1H-pyrrole-3-carboxylate d'éthyle,
5-Carbamoyl-4-{4-[3-(4-fluoro-phényl)-uréido]-phényl}-1H-pyrrole-3-carboxylate d'éthyle,
5-Carbamoyl-4-{4-[3-(4-méthoxy-phényl)-uréido]-phényl}-1H-pyrrole-3-carboxylate d'éthyle,
5-Carbamoyl-4-{4-[3-(4-trifluorométhyl-phényl)-uréido]-phényl}-1H-pyrrole-3-carboxylate d'éthyle,
5-Carbamoyl-4-[4-(3-p-tolyl-uréido)-phényl]-1H-pyrrole-3-carboxylate d'éthyle,
5-Carbamoyl-4-{4-[3-(4-chloro-3-trifluorométhyl-phényl)-uréido]-phényl}-1H-pyrrole-3-carboxylate d'éthyle,
5-Carbamoyl-4-{4-[3-(2-chloro-5-trifluorométhyl-phényl)-uréido]-phényl}-1H-pyrrole-3-carboxylate d'éthyle,
5-Carbamoyl-4-{4-[3-(2-fluoro-3-trifluorométhyl-phényl)-uréido]-phényl}-1H-pyrrole-3-carboxylate d'éthyle,
5-Carbamoyl-4-{4-[3-(4-fluoro-3-trifluorométhyl-phényl)-uréido]-phényl}-1H-pyrrole-3-carboxylate d'éthyle,
5-Carbamoyl-4-{4-[3-(3-fluoro-5-trifluorométhyl-phényl)-uréido]-phényl}-1H-pyrrole-3-carboxylate d'éthyle,
5-Carbamoyl-4-{4-[3-(4-méthyl-3-trifluorométhyl-phényl)-uréido]-phényl}-1H-pyrrole-3-carboxylate d'éthyle,
5-Carbamoyl-4-{4-[3-(4-trifluorométhoxy-phényl)-uréido]-phényl}-1H-pyrrole-3-carboxylate d'éthyle,
5-Carbamoyl-4-{4-[3-(4-difluorométhoxy-phényl)-uréido]-phényl}-1H-pyrrole-3-carboxylate d'éthyle,
5-Carbamoyl-4-{4-[3-(3,4-diméthyl-phényl)-uréido]-phényl}-1H-pyrrole-3-carboxylate d'éthyle,
5-Carbamoyl-4-{4-[3-(3,4-diméthoxy-phényl)-uréido]-phényl}-1H-pyrrole-3-carboxylate d'éthyle,
5-Carbamoyl-4-{4-[3-(3,5-diméthoxy-phényl)-uréido]-phényl}-1H-pyrrole-3-carboxylate d'éthyle,
5-Carbamoyl-4-{4-[3-(2,5-diméthyl-phényl)-uréido]-phényl}-1H-pyrrole-3-carboxylate d'éthyle,
5-Carbamoyl-4-{4-[3-(2-méthoxy-5-méthyl-phényl)-uréido]-phényl}-1H-pyrrole-3-carboxylate d'éthyle,
5-Carbamoyl-4-{4-[3-(2,5-diméthoxy-phényl)-uréido]-phényl}-1H-pyrrole-3-carboxylate d'éthyle,
5-Carbamoyl-4-{4-[3-(3-chloro-4-difluorométhoxy-phényl)-uréido]-phényl}-1H-pyrrole-3-carboxylate d'éthyle,
5-Carbamoyl-4-{4-[3-(3,5-diméthyl-phényl)-uréido]-phényl}-1H-pyrrole-3-carboxylate d'éthyle,
Acide 5-carbamoyl-4-{4-[3-(2-fluoro-5-trifluorométhyl-phényl)-uréido]-phényl}-1H-pyrrole-3-carboxylique,
4-(2,4-Diméthoxy-benzylamino)carbonyl-3-{4-[3-(2-fluoro-5-trifluorométhylphényl)-uréido]-phényl}-1H-pyrrole-2-carboxamide,
3-{4-[3-(2-Fluoro-5-trifluorométhyl-phényl)-uréido]-phényl}-1H-pyrrole-2,4-dicarboxamide,
3-{4-[3-(2-Fluoro-5-trifluorométhyl-phényl)-uréido]-phényl}-4-(4-méthyl-pipérazine-1-carbonyl)-1H-pyrrole-2-carboxamide,
3-{4-[3-(2-Fluoro-5-trifluorométhyl-phényl)-uréido]-phényl}-4-(pyrrolidine-1-carbonyl)-1H-pyrrole-2-carboxamide,
5-Carbamoyl-4-{4-[3-(2-fluoro-5-trifluorométhyl-phényl)-uréido]-phényl}-1H-pyrrole-3-carboxylate de 2-diméthylamino-éthyle,
4-Chloro-3-{4-[3-(2-fluoro-5-trifluorométhyl-phényl)-uréido]-phényl}-1H-pyrrole-2-carboxamide,
5-Chloro-3-{4-[3-(2-fluoro-5-trifluorométhyl-phényl)-uréido]-phényl}-1H-pyrrole-2-carboxamide,
5-{4-[3-(2-Fluoro-5-trifluorométhyl-phényl)-uréido]-phényl}-3H-imidazole-4-carboxamide.

Un produit conforme à l'invention pourra se présenter sous forme :
1) non chirale, ou
2) racémique, ou
3) enrichie en un stéréoisomère, ou
4) enrichie en un énantiomère ;
et pourra être éventuellement salifié.

Un produit conforme à l'invention pourra être utilisé pour la fabrication d'un médicament utile pour traiter un état pathologique, en particulier un cancer.

La présente invention concerne aussi les compositions thérapeutiques comprenant un produit selon l'invention, en combinaison avec un excipient pharmaceutiquement acceptable selon le mode d'administration choisi. La composition pharmaceutique peut se présenter sous forme solide, liquide ou de liposomes.

Parmi les compositions solides on peut citer les poudres, les gélules, les comprimés. Parmi les formes orales on peut aussi inclure les formes solides protégées vis-à-vis du milieu acide de l'estomac. Les supports utilisés pour les formes solides sont constitués notamment de supports minéraux comme les phosphates, les carbonates ou de supports organiques comme le lactose, les celluloses, l'amidon ou les polymères. Les formes liquides sont constituées de solutions de suspensions ou de dispersions. Elles contiennent comme support dispersif soit l'eau, soit un solvant organique (éthanol, NMP ou autres) ou de mélanges d'agents tensioactifs et de solvants ou d'agents complexants et de solvants.

Les formes liquides seront, de préférence, injectables et de ce fait auront une formulation acceptable pour une telle utilisation.

Des voies d'administration par injection acceptables incluent les voies intraveineuse, intra-péritonéale, intramusculaire, et sous cutanée, la voie intraveineuse étant habituellement préférée.

La dose administrée des composés de l'invention sera adaptée par le praticien en fonction de la voie d'administration au patient et de l'état de ce dernier.

Les composés de la présente invention peuvent être administrés seuls ou en mélange avec d'autres anticancéreux. Parmi les associations possibles on peut citer:
● les agents alkylants et notamment le cyclophosphamide, le melphalan, l'ifosfamide, le chlorambucil, le busulfan, le thiotepa, la prednimustine, la carmustine, la lomustine, la semustine, la steptozotocine, la decarbazine, la témozolomide, la procarbazine et l'hexaméthylmélamine
● les dérivés du platine comme notamment le cisplatine, le carboplatine ou l'oxaliplatine
● les agents antibiotiques comme notamment la bléomycine, la mitomycine, la dactinomycine
● les agents antimicrotubules comme notamment la vinblastine, la vincristine, la vindésine, la vinorelbine, les taxoïdes (paclitaxel et docétaxel)
● les anthracyclines comme notamment la doxorubicine, la daunorubicine, l'idarubicine, l'épirubicine, la mitoxantrone, la losoxantrone
● les inhibiteurs de topoisomérases des groupes I et II telles que l'étoposide, le teniposide, l'amsacrine, l'irinotecan, le topotecan et le tomudex
● les fluoropyrimidines telles que la 5-fluorouracile, l'UFT, la floxuridine
● les analogues de cytidine telles que la 5-azacytidine, la cytarabine, la gemcitabine, la 6-mercaptomurine, la 6-thioguanine
● les analogues d'adénosine tels que la pentostatine, la cytarabine ou le phosphate de fludarabine
● le méthotrexate et l'acide folinique
● les enzymes et composés divers tels que la L-asparaginase, l'hydroxyurée, l'acide trans-rétinoique, la suramine, la dexrazoxane, l'amifostine, l'herceptine ainsi que les hormones oestrogéniques, androgéniques
● les agents antivasculaires tels que les dérivés de la combretastatine, par exemple la CA4P, des chalcones ou de la colchicine, par exemple le ZD6126, et leurs prodrogues.

Il est également possible d'associer aux composés de la présente invention un traitement par des radiations. Ces traitements peuvent être administrés simultanément, séparément, séquentiellement. Le traitement sera adapté par le praticien en fonction du malade à traiter.

Les produits de l'invention sont utiles comme agents inhibiteurs d'une réaction catalysée par une kinase. FAK , KDR et Tie2 sont des kinases pour lesquelles les produits de l'invention seront particulièrement utiles en tant qu'inhibiteurs.

Les raisons pour lesquelles ces kinases sont choisies sont données ci-après :

### FAK

FAK est une tyrosine kinase cytoplasmique jouant un rôle important dans la transduction du signal transmis par les intégrines, famille de récepteurs hétérodimériques de l'adhésion cellulaire. FAK et les intégrines sont colocalisés dans des structures périmembranaires appelées plaques d'adhérence. Il a été montré dans de nombreux types cellulaires que l'activation de FAK ainsi que sa phosphorylation sur des résidus tyrosine et en particulier son autophosphorylation sur la tyrosine 397 étaient dépendantes de la liaison des intégrines à leurs ligands extracellulaires et donc induites lors de l'adhésion cellulaire [Kornberg L, et al. J. Biol. Chem. 267(33): 23439-442. (1992)]. L'autophosphorylation sur la tyrosine 397 de FAK représente un site de liaison pour une autre tyrosine kinase, Src, via son domaine SH2 [Schaller et al. Mol. Cell. Biol. 14 :1680-1688. 1994; Xing et al. Mol. Cell. Biol. 5 :413-421. 1994]. Src peut alors phosphoryler FAK sur la tyrosine 925, recrutant ainsi la protéine adaptatrice Grb2 et induisant dans certaines cellules l'activation de la voie ras et MAP Kinase impliquée dans le contrôle de la prolifération cellulaire [Schlaepfer et al. Nature; 372:786-791. 1994; Schlaepfer et al. Prog. Biophy. Mol. Biol. 71:435-478. 1999; Schlaepfer and Hunter, J. Biol. Chem. 272:13189-13195. 1997]. L'activation de FAK peut aussi induire la voie de signalisation jun NH2-terminal kinase (JNK) et résulter dans la progression des cellules vers la phase G1 du cycle cellulaire [Oktay et al., J. Cell. Biol.145 :1461-1469. 1999]. Phosphatidylinositol-3-OH kinase (PI3-kinase) se lie aussi à FAK sur la tyrosine 397 et cette interaction pourrait être nécessaire à l'activation de PI3-kinase [Chen and Guan, Proc. Nat. Acad. Sci. USA. 91: 10148-10152. 1994; Ling et al. J. Cell. Biochem. 73 :533-544. 1999]. Le complexe FAK/Src phosphoryle différents substrats comme la paxilline et p130CAS dans les fibroblastes [Vuori et al. Mol. Cell. Biol. 16: 2606-2613. 1996].

Les résultats de nombreuses études soutiennent l'hypothèse que les inhibiteurs de FAK pourraient être utiles dans le traitement du cancer. Des études ont suggéré que FAK pourrait jouer un rôle important dans la prolifération et/ou la survie cellulaires *in vitro.* Par exemple, dans les cellules CHO, certains auteurs ont démontré que la surexpression de p125FAK conduit à une accélération de la transition G1 à S, suggérant que p125FAK favorise la prolifération cellulaire [Zhao J.-H et al. J. Cell Biol. 143:1997-2008. 1998]. D'autres auteurs ont montré que des cellules tumorales traitées avec des oligonucleotides anti-sens de FAK perdent leur adhésion et entrent en apoptose (Xu et al, Cell Growth Differ. 4:413-418. 1996). Il a également été démontré que FAK promeut la migration des cellules *in vitro.* Ainsi, des fibroblastes déficients pour l'expression de FAK (souris « knockout » pour FAK) présentent une morphologie arrondie, des déficiences de migration cellulaire en réponse à des signaux chimiotactiques et ces défauts sont supprimés par une réexpression de FAK [DJ. Sieg et al., J. Cell Science. 112:2677-91. 1999]. La surexpression du domaine C-terminal de FAK (FRNK) bloque l'étirement des cellules adhérentes et réduit la migration cellulaire *in vitro* [Richardson A. and Parsons J.T. Nature. 380:538-540. 1996]. La surexpression de FAK dans des cellules CHO, COS ou dans des cellules d'astrocytome humain favorise la migration des cellules. L'implication de FAK dans la promotion de la prolifération et de la migration des cellules dans de nombreux types cellulaires *in vitro,* suggère le rôle potentiel de FAK dans les processus néoplasiques. Une étude récente a effectivement démontré l'augmentation de la prolifération des cellules tumorales *in vivo* après induction de l'expression de FAK dans des cellules d'astrocytome humain [Cary L.A. et al. J. Cell Sci. 109:1787-94. 1996; Wang D et al. J. Cell Sci. 113:4221-4230. 2000]. De plus, des études immunohistochimiques de biopsies humaines ont démontré que FAK était surexprimé dans les cancers de la prostate, du sein, de la thyroïde, du colon, du mélanome, du cerveau et du poumon, le niveau d'expression de FAK étant directement corrélé aux tumeurs présentant le phénotype le plus agressif [Weiner TM, et al. Lancet. 342(8878):1024-1025. 1993 ; Owens et al. Cancer Research. 55:2752-2755. 1995; Maung K. et al. Oncogene. 18:6824-6828. 1999; Wang D et al. J. Cell Sci. 113:4221-4230. 2000].

### KDR

KDR (Kinase insert Domain Receptor) aussi appelée VEGF-R2 (Vascular Endothelial Growth Factor Receptor 2), est exprimé uniquement dans les cellules endothéliales. Ce récepteur se fixe au facteur de croissance angiogénique VEGF, et sert ainsi de médiateur à un signal transductionnel via l'activation de son domaine kinase intracellulaire. L'inhibition directe de l'activité kinase de VEGF-R2 permet de réduire le phénomène d'angiogénèse en présence de VEGF exogène (Vascular Endothelial Growth Factor : facteur de croissance vasculaire endothélial) (Strawn et al., Cancer Research, 1996, vol. 56, p.3540-3545). Ce processus a été démontré notamment à l'aide de mutants VEGF-R2 (Millauer et al., Cancer Research, 1996, vol. 56, p.1615-1620). Le récepteur VEGF-R2 semble n'avoir aucune autre fonction chez l'adulte que celle liée à l'activité angiogénique du VEGF. Par conséquent, un inhibiteur sélectif de l'activité kinase du VEGF-R2 ne devrait démontrer que peu de toxicité.

En plus de ce rôle central dans le processus dynamique angiogénique, des résultats récents suggèrent que l'expression de VEGF contribue à la survie des cellules tumorales après des chimio- et radio-thérapies, soulignant la synergie potentielle d'inhibiteurs de KDR avec d'autres agents (Lee et al. Cancer Research, 2000, vol. 60, p.5565-5570).

### Tie2

Tie-2 (TEK) est un membre d'une famille de récepteurs à tyrosine kinase, spécifique des cellules endothéliales. Tie2 est le premier récepteur à activité tyrosine kinase dont on connaît à la fois l'agoniste (angiopoïetine 1 ou Ang1) qui stimule l'autophosphorylation du récepteur et la signalisation cellulaire [S. Davis et al (1996) Cell 87, 1161-1169] et l'antagoniste (angiopoïetine 2 ou Ang2) [P.C. Maisonpierre et al. (1997) Science 277, 55-60]. L'angiopoïetine 1 peut synergiser avec le VEGF dans les derniers stades de la néo-angiogénèse [AsaharaT. Circ. Res.(1998) 233-240]. Les expériences de knock-out et les manipulations transgéniques de l'expression de Tie2 ou de Ang1 conduisent à des animaux qui présentent des défauts de vascularisation [D.J. Dumont et al (1994) Genes Dev. 8, 1897-1909 *et* C. Suri (1996) Cell 87, 1171-1180]*.* La liaison d'Ang1 à son récepteur conduit à l'autophosphorylation du domaine kinase de Tie2 qui est essentielle pour la néovascularisation ainsi que pour le recrutement et l'interaction des vaisseaux avec les péricytes et les cellules musculaires lisses ; ces phénomènes contribuent à la maturation et la stabilité des vaisseaux nouvellement formés [P.C. Maisonpierre et al (1997) Science 277, 55-60]. Lin et al (1997) J. Clin. Invest. 100, 8: 2072-2078 *et* Lin P. (1998) PNAS 95, 8829-8834*,* ont montré une inhibition de la croissance et de la vascularisation tumorale, ainsi qu'une diminution des métastases de poumon, lors d'infections adénovirales ou d'injections du domaine extracellulaire de Tie-2 (Tek) dans des modèles de xénographes de tumeur du sein et de mélanome.

Les inhibiteurs de Tie2 peuvent être utilisés dans les situations où une néovascularisation se fait de façon inappropriée (c'est-à-dire dans la rétinopathie diabétique, l'inflammation chronique, le psoriasis, le sarcome de Kaposi, la néovascularisation chronique due à la dégénérescence maculaire, l'arthrite rhumatoïde, l'hémoangiome infantile et les cancers).

### Définitions

Le terme « halogène » fait référence à un élément choisi parmi F, Cl, Br, et I.

Le terme «alkyle» fait référence à un substituant hydrocarboné saturé, linéaire ou ramifié, ayant de 1 à 12 atomes de carbone. Les substituants méthyle, éthyle, propyle, 1-méthyléthyl, butyle, 1-méthylpropyl, 2-méthylpropyle, 1,1-diméthyléthyle, pentyle, 1-méthylbutyle, 2-méthylbutyle, 3-méthylbutyle, 1,1-diméthylpropyle, 1,2-diméthylpropyle, 2,2-diméthylpropyle, 1-éthylpropyle, hexyle, 1-méthylpentyle, 2-méthylpentyle, 1-éthylbutyle, 2-éthylbutyle, 3,3-diméthylbutyle, heptyle, 1-éthylpentyle, octyle, nonyle, décyle, undécyle, et dodécyle sont des exemples de substituant alkyle.

Le terme « alkylène » fait référence à un substituant hydrocarboné linéaire ou ramifié ayant une ou plusieurs insaturations, ayant de 2 à 12 atomes de carbone. Les substituants éthylènyle, 1-méthyléthylènyle, prop-1-ènyle, prop-2-ènyle, Z-1-méthylprop-1-ènyle, E-1-méthylprop-1-ènyle, Z-1,2-diméthyl-prop-1-ènyle, E-1,2-diméthylprop-1-ènyle, but-1,3-diényle, 1-méthylidènyl-prop-2-ènyle, Z-2-méthylbut-1,3-diényle, E-2-méthylbut-1,3-diényle, 2-méthyl-1-méthylidènylprop-2-ènyle, undéc-1-ènyle et undéc-10-ènyle sont des exemples de substituant alkylène.

Le terme « alkynyle » fait référence à un substituant hydrocarboné linéaire ou ramifié ayant au moins deux insaturations portées par une paire d'atomes de carbone vicinaux, ayant de 2 à 12 atomes de carbone. Les substituants éthynyle; prop-1-ynyle; prop-2-ynyle; et but-1-ynyle sont des exemples de substituant alkynyle.

Le terme « aryle » fait référence à un substituant aromatique mono- ou polycyclique ayant de 6 à 14 atomes de carbone. Les substituants phényle, napht-1-yle ; napht-2-yle ; anthracen-9-yl ; 1,2,3,4-tétrahydronapht-5-yle ; et 1,2,3,4-tétrahydronapht-6-yle sont des exemples de substituant aryle.

Le terme « hétéroaryle » fait référence à un substituant hétéroaromatique mono- ou polycyclique ayant de 1 à 13 atomes de carbone et de 1 à 4 hétéroatomes. Les substituants pyrrol-1-yle ; pyrrol2-yle ; pyrrol3-yle ; furyle ; thienyle ; imidazolyle ; oxazolyle ; thiazolyle ; isoxazolyle ; isothiazolyle ; 1,2,4-triazolyle; oxadiazolyle ; thiadiazolyle ; tétrazolyle ; pyridyle ; pyrimidyle ; pyrazinyle ; 1 ,3,5-triazinyle ; indolyle ; benzo[b]furyle ; benzo[b]thiényle; indazolyle ; benzimidazolyle ; azaindolyle ; quinoléyle ; isoquinoléyle ; carbazolyle ; et acridyle sont des exemples de substituant hétéroaryle.

Le terme « hétéroatome » fait référence ici à un atome au moins divalent, différent du carbone. N; O; S; et Se sont des exemples d'hétéroatome.

Le terme « cycloalkyle » fait référence à un substituant hydrocarboné cyclique saturé ou partiellement insaturé ayant de 3 à 12 atomes de carbone. Les substituants cyclopropyle; cyclobutyle; cyclopentyle; cyclopentènyle; cyclopentadiényle; cyclohexyle; cyclohexènyle; cycloheptyle; bicyclo[2.2.1]heptyle ; cyclooctyle; bicyclo[2.2.2]octyle ; adamantyle ; et perhydronapthyle sont des exemples de substituant cycloalkyle.

Le terme «hétérocyclyle» fait référence à un substituant hydrocarboné cyclique saturé ou partiellement insaturé ayant de 1 à 13 atomes de carbone et de 1 à 4 hétéroatomes. De préférence, le substituant hydrocarboné cyclique saturé ou partiellement insaturé sera monocyclique et comportera 4 ou 5 atomes de carbone et 1 à 3 hétéroatomes.

Le terme « substitué » fait référence à un ou plusieurs substituants différents de H, par exemple halogène; alkyle; alkyle halogéné ; aryle; aryle substitué par un ou plusieurs groupements alcoxy; hétéroaryle, cycloalkyle; hétérocyclyle ; alkylène; alkynyle; OH; O-alkyle; O-alkylène; O-aryle; O-hétéroaryle; NH₂; NH-alkyle; NH-aryle; NH-hétéroaryle; N-alkyle-alkyle'; SH; S-alkyle; S-aryle; S(O₂)H; S(O₂)-alkyle; S(O₂)-aryle; SO₃H; SO₃-alkyle; SO₃-aryle; CHO ; C(O)-alkyle; C(O)-aryle; C(O)OH ; C(O)O-alkyle; C(O)O-aryle; OC(O)-alkyle; OC(O)-aryle ; C(O)NH₂; C(O)NH-alkyle; C(O)NH-aryle; NHCHO ; NHC(O)-alkyle; NHC(O)-aryle; NH-cycloalkyle; NH-hétérocyclyle. Les produits selon l'invention peuvent être préparés à partir de méthodes conventionnelles de chimie organique non couvertes par les revendications mais utiles à la compréhension de l'invention. Le schéma 1 ci-dessous est illustratif de la méthode utilisée pour la préparation de l'exemple 1 concernant les pyroles substitués.

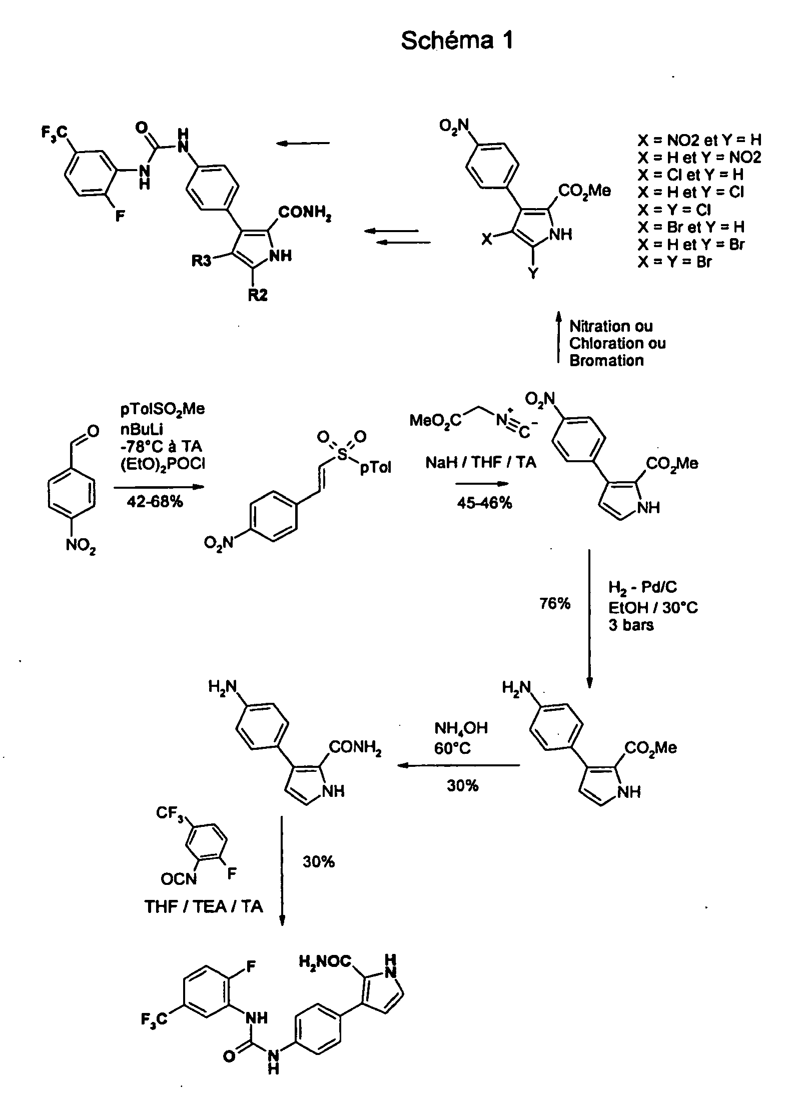

Les schémas ci-dessous sont illustratifs des méthodes utilisées pour la préparation des exemples concernant les imidazoles substitués.

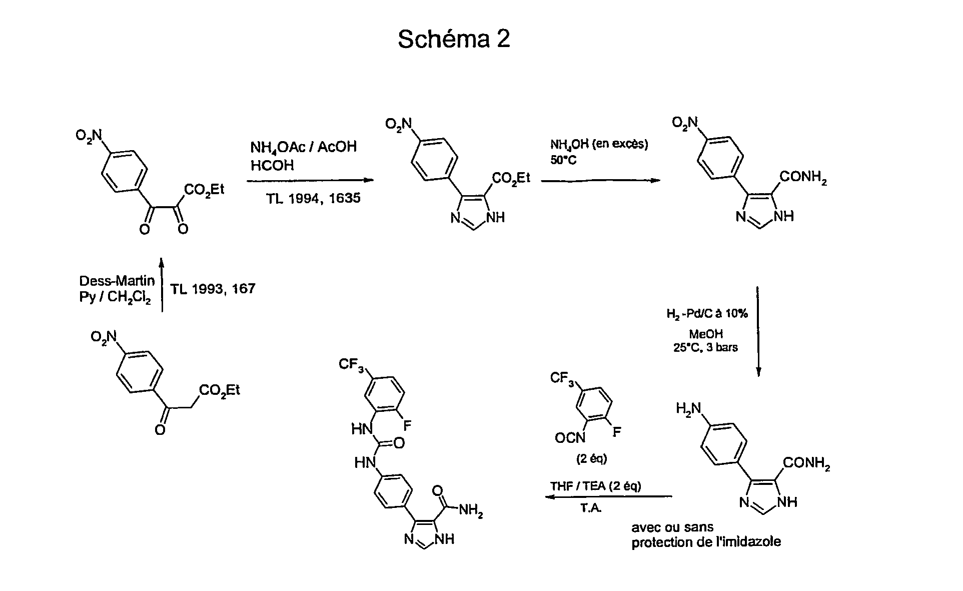

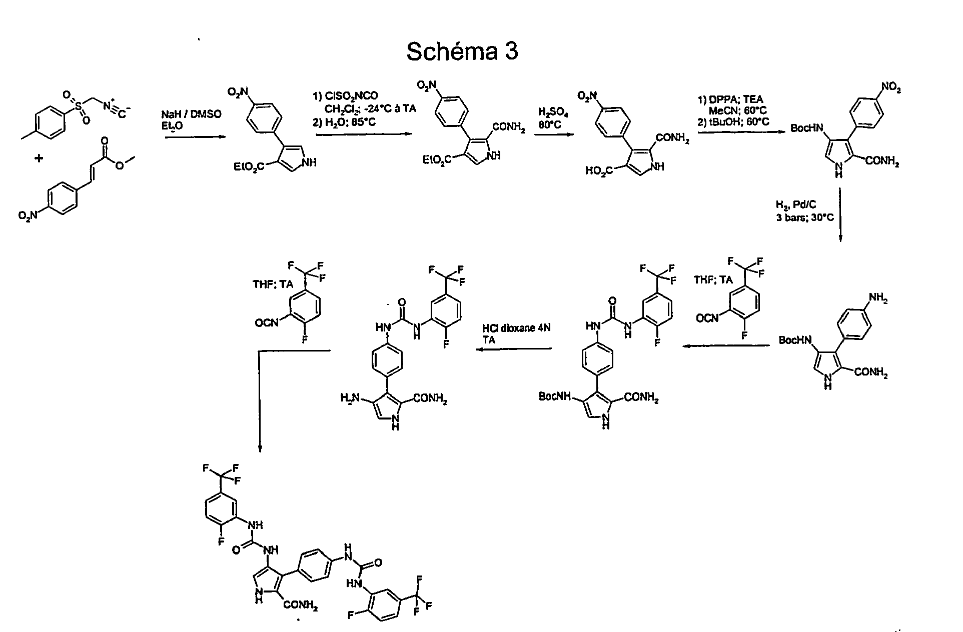

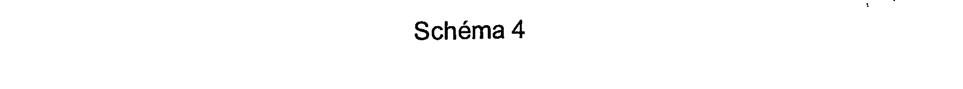

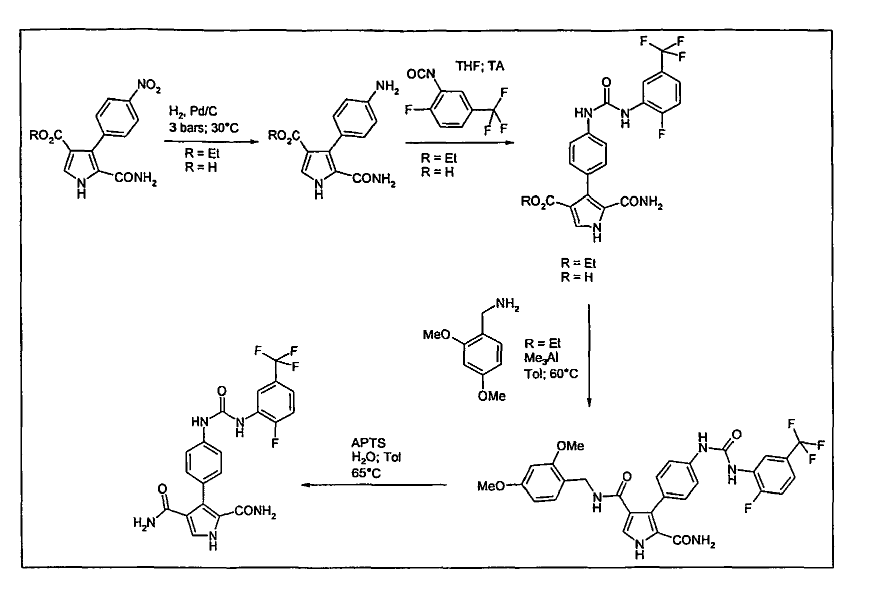

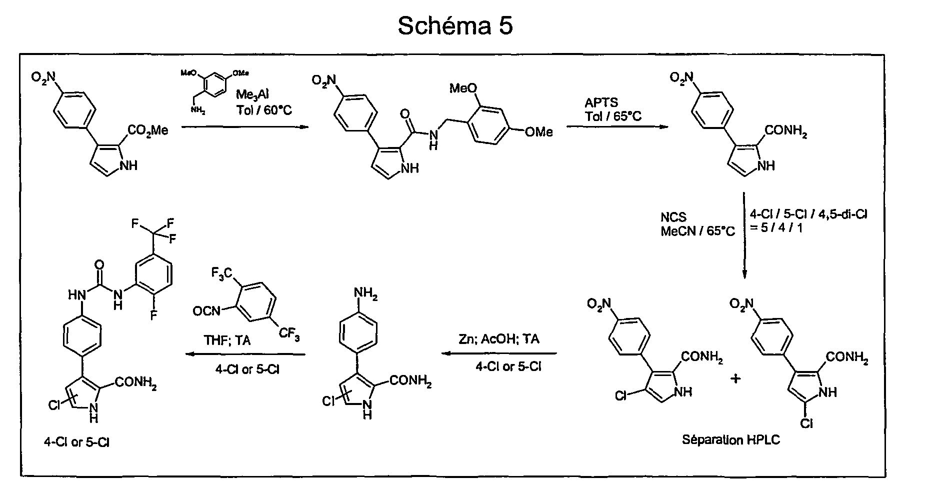

II est entendu pour l'homme du métier que, pour la mise en oeuvre des procédés selon l'invention décrits précédemment, il peut être nécessaire d'introduire des groupements protecteurs des fonctions amino, carboxyle et alcool afin d'éviter des réactions secondaires. Ces groupes sont ceux qui permettent d'être éliminés sans toucher au reste de la molécule. Comme exemples de groupes protecteurs de la fonction amino, on peut citer le carbamate de *tert*-butyle qui peut être régénéré au moyen d'acide trifluoroacétique ou d'iodotriméthylsilane, l'acétyle qui peut être régénéré en milieu acide (acide chlorhydrique par exemple). Comme groupes protecteurs de la fonction carboxyle, on peut citer les esters (méthoxyméthylester, benzylester par exemple). Comme groupes protecteurs de la fonction alcool, on peut citer les esters (benzoylester par exemple) qui peuvent être régénérés en milieu acide ou par hydrogénation catalytique. D'autres groupes protecteurs utilisables sont décrits par T. W. GREENE et coll. dans Protective Groups in Organic Synthesis, third edition, 1999, Wiley-Interscience.

Les composés de formule (I) sont isolés et peuvent être purifiés par les méthodes connues habituelles, par exemple par cristallisation, chromatographie ou extraction.

Les énantiomères, diastéréoisomères des composés de formule (I) font également partie de l'invention.

Les composés de formule (I) comportant un reste basique peuvent être éventuellement transformés en sels d'addition avec un acide minéral ou organique, par action d'un tel acide au sein d'un solvant, par exemple organique tel un alcool, une cétone, un éther ou un solvant chloré.

Les composés de formule (I) comportant un reste acide peuvent être éventuellement transformés en sels métalliques ou en sels d'addition avec des bases azotées selon les méthodes connues en soi. Ces sels peuvent être obtenus par action d'une base métallique (alcaline ou alcalinoterreuse par exemple), de l'ammoniac, d'une amine ou d'un sel d'amine sur un composé de formule (I), dans un solvant. Le sel formé est séparé par les méthodes habituelles.

Ces sels font également partie de l'invention.

Lorsqu'un produit selon l'invention présente au moins une fonction basique libre, des sels pharmaceutiquement acceptables peuvent être préparés par réaction entre ledit produit et un acide minéral ou organique. Des sels pharmaceutiquement acceptables incluent les chlorures, nitrates, sulfates, hydrogénosulfates, pyrosulfates, bisulfates, sulfites, bisulfites, phosphates, monohydrogénophosphates, dihydrogénophosphates, métaphosphates, pyrophosphates, acétates, propionates, acrylates, 4-hydroxybutyrates, caprylates, caproates, décanoates, oxalates, malonates, succinates, glutarates, adipates, pimélates, maléates, fumarates, citrates, tartrates, lactates, phénylacétates, mandélates, sébacates, subérates, benzoates, phtalates, méthanesulfonates, p-toluène sulfonate, propanesulfonates, xylènesulfonates, salicylates, cinnamates, glutamates, aspartates, glucuronates, galacturonates.

Lorsqu'un produit selon l'invention présente au moins une fonction acide libre, des sels pharmaceutiquement acceptables peuvent être préparés par réaction entre ledit produit et une base minérale ou organique. Des bases pharmaceutiquement acceptables incluent des hydroxydes de cations de métaux alcalins ou alcalino-terreux tels que Li, Na, K, Mg, Ca, des composés aminés basiques tels qu'ammoniac, arginine, histidine, pipéridine, morpholine, pipérazine, triéthylamine.

L'invention est également décrite par les exemples suivants, donnés à titre d'illustration de l'invention.

Les analyses LC/MS ont été réalisées sur un appareil Micromass modèle LCT relié à un appareil HP 1100. L'abondance des produits a été mesurée à l'aide d'un détecteur à barrette de diodes HP G1315A sur une gamme d'onde de 200-600 nm et un détecteur à dispersion de lumière Sedex 65. L'acquisition des spectres de masses Mass spectra a été réalisée sur une gamme de 180 à 800. Les données ont été analysées en utilisant le logiciel Micromass MassLynx. La séparation a été effectuée sur une colonne Hypersil BDS C18, 3 µm (50 x 4.6 mm), en éluant par un gradient linéaire de 5 à 90% d'acétonitrile contenant 0,05% (v/v) d'acide trifluoroacétique (TFA) dans l'eau contenant 0,05% (v/v) TFA en 3,5 mn à un débit de 1 mL/mn. Le temps total d'analyse, incluant la période de rééquilibration de la colonne, est de 7 mn.

Les spectres MS ont été réalisés en électrospray (ES⁺) sur un appareil Platform II (Micromass). Les principaux ions observés sont décrits.

Les points de fusion ont été mesurés en capillaire, sur un appareil Mettler FP62, gamme 30°C à 300°C, montée de 2°C par minute.

### Purification par LC/MS:

Les produits peuvent être purifiés par LC/MS en utilisant un système Waters FractionsLynx composé d'une pompe à gradient Waters modèle 600, d'une pompe de régénération Waters modèle 515, d'une pompe de dilution Waters Reagent Manager, d'un auto-injecteur Waters modèle 2700, de deux vannes Rheodyne modèle LabPro, d'un détecteur à barrette de diodes Waters modèle 996, d'un spectromètre de masse Waters modèle ZMD et d'un collecteur de fractions Gilson modèle 204. Le système était controlé par le logiciel Waters FractionLynx. La séparation a été effectuée alternativement sur deux colonnes Waters Symmetry (C₁₈, 5µM, 19x50 mm, référence catalogue 186000210), une colonne étant en cours de régénération par un mélange eau/acétonitrile 95/5 (v/v) contenant 0,07% (v/v) d'acide trifluoroacétique, pendant que l'autre colonne était en cours de séparation. L'élution des colonnes a été effectuée en utilisant un gradient linéaire de 5 à 95% d'acétonitrile contenant 0,07% (v/v) d'acide trifluoroacétique dans l'eau contenant 0,07% (v/v) d'acide trifluoroacétique, à un débit de 10 mL/mn. A la sortie de la colonne de séparation, un millième de l'effluent est séparé par un LC Packing Accurate, dilué à l'alcool méthylique à un débit de 0,5 mL/mn et envoyé vers les détecteurs, à raison de 75% vers le détecteur à barrette de diodes, et les 25% restants vers le spectromètre de masse. Le reste de l'effluent (999/1000) est envoyé vers le collecteur de fractions où le flux est éliminé tant que la masse du produit attendu n'est pas détectée par le logiciel FractionLynx. Les formules moléculaires des produits attendus sont fournies au logiciel FractionLynx qui déclenche la collecte du produit quand le signal de masse détecté correspond à l'ion [M+H]⁺ et/ou au [M+Na]⁺. Dans certains cas, dépendant des résultats de LC/MS analytique, quand un ion intense correspondant à [M+2H]⁺⁺ a été détecté, la valeur correspondant à la moitié de la masse moléculaire calculée (MW/2) est aussi fournie au logiciel FractionLynx. Dans ces conditions, la collecte est aussi déclenchée quand le signal de masse de l'ion [M+2H]⁺⁺ et/ou [M+Na+H]⁺⁺ sont détectés. Les produits ont été collectés en tube de verre tarés. Après collecte, les solvants ont été évaporés, dans un évaporateur centrifuge Savant AES 2000 ou Genevac HT8 et les masses de produits ont été déterminées par pesée des tubes après évaporation des solvants.

Analyse EI-Cl : introduction directe (DCI=dépôt échantillon sur filament)

Spectromètre de masse Finnigan SSQ7000 ; domaine de masse m/z=29-900 ; énergie des électrons 70eV ;température de la source 70 °C ;gaz réactant Cl ammoniac ; EI=Ionisation par impact d'électrons ; CI= Ionisation chimique.

Analyse electrospray : (electrospray positif : ES⁺ ; electrospay négatif : ES⁻)

Couplage LC-MS-DAD-ELSD :

MS : Waters-Micromass Platform II; LC : Agilent HP 1100 ; colonne Hypersil GOLD Thermo C18; 3X50 mm, 2.5 µm; éluant: gradient Eau (avec Acide Formique 0,1%) + acetonitrile ; UV : DAD (λ=200-400nm).

MS : Waters-Micromass ZQ; LC : Agilent HP 1100 ; colonne Xbridge Waters C18, 3X50 mm, 2.5 µm; éluant: gradient Eau (avec Acide Formique 0,1%) + acetonitrile ; UV : λ=254nm.

Spectre RMN 1H à 400 MHz sur spectromètre BRUKER AVANCE DRX-400 avec les déplacements chimiques (δ en ppm) - dans le solvant diméthylsulfoxide - d6 (DMSO-d6) référencé à 2,50 ppm à la température de 303K.

### Exemple 1

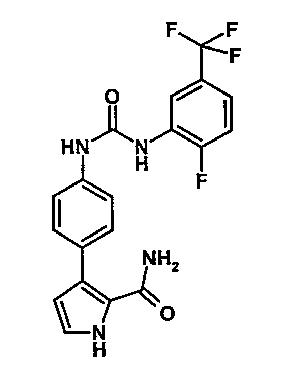

3-{4-[3-(2-Fluoro-5-trifluorométhyl-phényl)-uréido]-phényl}-1H-pyrrole-2-carboxamide

A 0,490 g (2,435 mmol) de 3-(4-amino-phényl)-1H-pyrrole-2-carboxamide en solution dans 35 cm³ de tétrahydrofurane, on ajoute à une température voisine de 20°C, sous atmosphère d'argon, 0,704 cm³ de 2-fluoro-5-trifluorométhyl-phényl isocyanate et 0,678 cm³ de triéthylamine. Après 16 heures d'agitation à une température voisine de 20°C, le mélange réactionnel est concentré à sec sous pression réduite (2,7 kPa) pour donner un résidu qui est repris dans 40 cm³ d'acétate d'éthyle. La phase organique est lavée avec 3 fois 20 cm³ d'eau et 30 cm³ d'une solution aqueuse saturée en chlorure de sodium, séchée sur sulfate de magnésium anhydre, filtrée et concentrée à sec sous pression réduite (2,7 kPa) pour donner 1,1 g d'un solide jaune qui est purifié par chromatographie-flash [éluant : dichlorométhane / méthanol / acétonitrile (90 / 5 / 5 puis 1 / 1 / 0 en volumes)]. Après concentration des fractions sous pression réduite, on obtient 0,3 g de 3-{4-[3-(2-fluoro-5-trifluorométhyl-phényl)-uréido]-phényl}-1H-pyrrole-2-carboxamide, sous forme d'un solide jaune fondant à 236°C ; R.M.N. 1H (400 MHz, DMSO d6, δ en ppm) : 6,15 (t, J = 2,5 Hz : 1H) ; 6,89 (t, J = 2,5 Hz : 1H) ; de 6,10 à 7,30 (mt très étalé : 2H) ; de 7,34 à 7,42 (mt : 3H) ; de 7,44 à 7,54 (mt : 3H) ; 8,63 (dd, J = 8,5 et 2,5 Hz : 1H) ; 8,90 (d large, J = 3 Hz : 1H) ; 9,21 (s large : 1H) ; 11,4 (s large : 1H).

Le 3-(4-amino-phényl)-1H-pyrrole-2-carboxamide peut être préparé de la manière suivante :
Une suspension de 0,3 g (1,387 mmol) de 3-(4-amino-phényl)-1H-pyrrole-2-carboxylate de méthyle dans 50 cm³ d'une solution aqueuse d'ammoniaque à 22% est chauffée en autoclave à une température voisine de 50°C, sous 12 bars, pendant 20 heures. Après arrêt du chauffage puis retour à température et pression ambiantes, le mélange réactionnel est concentré à sec sous pression réduite (2,7 kPa) pour donner 0,31 g d'un solide orange qui est purifié par chromatographie-flash [éluant: dichlorométhane / méthanol / acétonitrile (96 / 2 / 2 en volumes)]. Après concentration des fractions sous pression réduite, on obtient 0,085 g de 3-(4-amino-phényl)-1H-pyrrole-2-carboxamide, sous forme d'un solide jaune; M.W.=201, IE: m/z = 201 M^{+.}, m/z = 184 [M-NH₃]^{+.}, m/z=155 [M-H₂CONH₂]⁺.

Le 3-(4-amino-phényl)-1H-pyrrole-2-carboxylate de méthyle peut être préparé de la manière suivante :
A une suspension de 0,1 g (0,942 mmol) de palladium sur charbon à 10% dans 35 cm³ de méthanol, on ajoute à une température voisine de 20°C, 1 g (4,061 mmol) de 3-(4-nitro-phényl)-1H-pyrrole-2-carboxylate de méthyle. Après 20 heures d'hydrogénation en autoclave sous 3 bars d'hydrogène, à une température voisine de 35°C, le mélange réactionnel est filtré, le catalyseur est rincé avec 2 fois 5 cm³ de méthanol puis le filtrat est concentré à sec sous pression réduite (2,7 kPa) pour donner 0,67 g de 3-(4-aminophényl)-1H-pyrrole-2-carboxylate de méthyle, sous forme d'un solide orange; M.W.=216, IE: m/z = 216 M^{+.}, m/z = 184 [M-CH₃OH]^{+.}, m/z=155 [M-H₂COOCH₃]⁺.

Le 3-(4-nitro-phényl)-1H-pyrrole-2-carboxylate de méthyle peut être préparé de la manière suivante :
A une suspension de 1,62 g (40,63 mmol) d'hydrure de sodium à 60% dans de l'huile minérale dans 100 cm³ de tétrahydrofurane, on ajoute goutte à goutte en 25 minutes à une température voisine de 20°C, sous atmosphère d'argon, un mélange de 5,87 g (19,35 mmol) de 1-[(E)-2-(4-méthyl-phényl)-sulfonyl-vinyl]-4-nitro-benzene et 3,7 cm³ (38,7 mmol) d'isocyanoacétate de méthyle en solution dans 150 cm³ de tétrahydrofurane. Après 5 heures d'agitation à une température voisine de 20°C, le mélange réactionnel est repris dans un mélange de 100 cm³ d'eau et 200 cm³ d'acétate d'éthyle. La phase organique est lavée avec 2 fois 100 cm³ d'eau et 150 cm³ d'une solution aqueuse saturée en chlorure de sodium, séchée sur sulfate de magnésium anhydre, filtrée et concentrée à sec sous pression réduite (2,7 kPa) pour donner 5,3 g d'une huile brune qui est purifié par chromatographie-flash [éluant : dichlorométhane]. Après concentration des fractions sous pression réduite, on obtient 2,12 g de 3-(4-nitro-phényl)-1H-pyrrole-2-carboxylate, sous forme d'un solide jaune ; M.W.=246, IE: m/z = 246 M^{+.}, m/z = 214 [M-CH₃OH]⁺.

Le 1-[(E)-2-(4-méthyl-phényl)-sulfonyl-vinyl]-4-nitro-benzene peut être préparé de la manière suivante :
A 15 g (85,47 mmol) de méthyl p-tolyl sulfone en solution dans 450 cm³ de tétrahydrofurane, on ajoute à une température voisine de 0°C, sous atmosphère d'argon, 117,5 cm³ (188 mmol) d'une solution de n-butyllithium à 1,06M dans l'hexane. Après 30 minutes d'agitation à une température voisine de 0°C, on ajoute, sous atmosphère d'argon, 13 cm³ (85,47 mmol) de diéthylchlorophosphate en solution dans 70 cm³ de tétrahydrofurane. Après 30 minutes d'agitation à une température voisine de 0°C, on refroidit le mélange réactionnel à une température voisine de -78°C puis on ajoute, sous atmosphère d'argon, 13,05 g (85,47 mmol) de 4-nitrobenzaldéhyde en solution dans 130 cm³ de tétrahydrofurane. Après 1 heure d'agitation à une température voisine de -78°C, on laisse remonter la température du mélange réactionnel à une température voisine de 20°C, on ajoute 100 cm³ d'eau et on concentre sous pression réduite (2,7 kPa) pour donner un solide beige. Ce solide est agité dans 250 cm³ d'eau pendant 20 minutes, filtré, lavé avec 3 fois 40 cm³ d'eau, séché à l'étuve sous pression réduite (2,7 kPa) à 35°C pendant 5 heures pour donner 25,43 g de 1-[(E)-2-(4-méthyl-phényl)-sulfonyl-vinyl]-4-nitro-benzene, sous forme d'un solide beige fondant à 176°C; M.W.=303, IE: m/z = 303 M^{+.}, m/z = 239 [M-SO₂]^{+.}, m/z=139 [CH₃-Ph-SO]⁺.

### Exemple 2

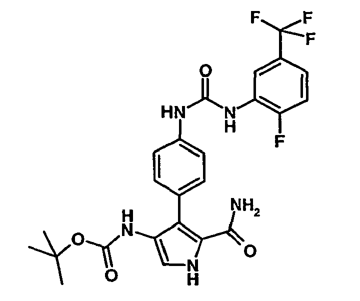

4-*tert*-Butyloxycarbonylamino-3-{4-[3-(2-fluoro-5-trifluorométhyl-phényl)-uréido]-phényl}-1H-pyrrole-2-carboxamide

A 0,096 g (0,303 mmol) de 4-*tert*-butyloxycarbonylamino-3-(4-amino-phényl)-1H-pyrrole-2-carboxamide en solution dans 11 cm³ de tétrahydrofurane, on ajoute à une température voisine de 20°C, sous atmosphère d'argon, 0,044 cm³ de 2-fluoro-5-trifluorométhyl-phényl isocyanate. Après 1 heure d'agitation à une température voisine de 20°C, le mélange réactionnel est dilué avec 40 cm³ d'acétate d'éthyle puis lavé par 3 fois 15 cm³ d'eau et 15 cm³ d'une solution aqueuse saturée en chlorure de sodium, séché sur sulfate de magnésium anhydre, filtré et concentré à sec sous pression réduite (2,7 kPa) pour donner 0,181 g d'un solide jaune qui est purifié par chromatographie-flash [éluant : dichlorométhane / méthanol / acétonitrile (90 / 5 / 5 en volumes)]. Après concentration des fractions sous pression réduite, on obtient 0,15 g de 4-*tert*-butyloxycarbonylamino-3-{4-[3-(2-fluoro-5-trifluorométhyl-phényl)-uréido]-phényl}-1H-pyrrole-2-carboxamide, sous forme d'un solide jaune; R.M.N. 1H (400 MHz, DMSO d6, δ en ppm) 1,35 (s large, 9H) ; 5,70 (m étalé, 1H) ; 6,88 (s large, 1H) ; 7,05 (m étalé, 1H) ; 7,26 (d, J = 8,5 Hz, 2H) ; 7,39 (m, 1H) ; 7,49 (m partiellement masqué, 1H) ; 7,49 (d, J = 8,5 Hz, 2H) ; 7,70 (s large, 1H) ; 8,62 (dd, J = 2,5 et 7,5 Hz, 1H) ; 9,03 (s large, 1H) ; 9,36 (s, 1H) ; 11,3 (s large, 1H): ; ES⁺: m/z=522: [M+H]⁺ ; ES^{-:} m/z=520: [M-H]⁻.

Le 4-*tert*-butyloxycarbonylamino-3-(4-amino-phényl)-1H-pyrrole-2-carboxamide peut être préparé de la manière suivante :
A une suspension de 0,024 g (0,222 mmol) de palladium sur charbon à 10% dans 63 cm³ de méthanol, on ajoute à une température voisine de 20°C, 0,331 g (0,956 mmol) de 4-*tert*-butyloxycarbonylamino-3-(4-nitro-phényl)-1H-pyrrole-2-carboxamide. Après 5 heures d'hydrogénation en autoclave sous 4 bar d'hydrogène, à une température voisine de 40°C, le mélange réactionnel est filtré. Le catalyseur est rincé avec 2 fois 15 cm³ de méthanol puis le filtrat est concentré à sec sous pression réduite (2,7 kPa) pour donner 0,3 g de 4-*tert*-butyloxycarbonylamino-3-(4-amino-phényl)-1H-pyrrole-2-carboxamide, sous forme d'un solide blanc fondant vers 142°C; El: m/z=316 M^{+.} ; m/z=260 [M-tBu]^{+.}; m/z=243 [M-OtBu]⁺; m/z=199 [243-CONH₂]⁺; m/z=57: [tBu]⁺ pic de base.

Le 4-*tert*-butyloxycarbonylamino-3-(4-nitro-phényl)-1H-pyrrole-2-carboxamide peut être préparé de la manière suivante :
A 0,45 g (1,635 mmol) d'acide 5-carbamoyl-4-(4-nitro-phényl)-1H-pyrrole-3-carboxylique en suspension dans 15 cm³ d'acétonitrile, on ajoute à une température voisine de 5°C, sous atmosphère d'argon, 0,241 cm³ de triéthylamine puis 0,370 cm³ d'azoture de diphénoxyphosphoryle. On laisse le milieu réactionnel évoluer en 30 minutes jusqu'à une température voisine de 20°C puis on chauffe 2,5 heures à une température voisine de 60°C. Le mélange réactionnel est concentré à sec sous pression réduite (2,7 kPa) pour donner une huile brune qui est repris dans 25 cm³ de *tert*-butanol. Après 2 heures d'agitation à une température voisine de 60°C, le mélange réactionnel est concentré à sec sous pression réduite (2,7 kPa) pour donner 0,56 g d'un solide ocre qui est purifié par chromatographie-flash [éluant : dichlorométhane / méthanol / acétonitrile (96 / 2 / 2 en volumes)]. Après concentration des fractions sous pression réduite, on obtient 0,331 g de 4-*tert*-butyloxycarbonylamino-3-(4-nitro-phényl)-1H-pyrrole-2-carboxamide, sous forme d'un solide jaune; R.M.N. 1H (400 MHz, DMSO d6, δ en ppm) : ; EI: m/z=346 M^{+.} ; m/z=290 [M-tBu]^{+.}; m/z=273 [M-OtBu]⁺; m/z=229 [273-CONH₂]⁺; m/z=57 [tBu]⁺ pic de base.

L'acide 5-carbamoyl-4-(4-nitro-phényl)-1H-pyrrole-3-carboxylique peut être préparé de la manière suivante :
Une solution de 2,8 g (9,232 mmol) de 5-carbamoyl-4-(4-nitro-phényl)-1H-pyrrole-3-carboxylate d'éthyle dans 33,6 cm³ (0,614 mol) d'acide sulfurique concentré est chauffée à une température voisine de 80°C pendant 2 heures. Le mélange réactionnel est versé lentement et sous agitation sur 350 cm³ d'eau glacée. Après 30 minutes d'agitation, le mélange réactionnel est filtré sur verre fritté N°3. Le solide est lavé avec 4 fois 100 cm³ d'eau glacée puis séché sous pression réduite (2,7 kPa), à une température voisine de 45°C pendant 24 heures, pour donner 2,25 g d'acide 5-carbamoyl-4-(4-nitrophényl)-1H-pyrrole-3-carboxylique, sous forme d'un solide beige; ES⁺: m/z=276: [M+H]⁺ ; ES^{-:} m/z=274: [M-H]⁻.

Le 5-carbamoyl-4-(4-nitro-phényl)-1H-pyrrole-3-carboxylate d'éthyle peut être préparé de la manière suivante :
A 18 g (69,16 mmol) de 4-(4-nitro-phényl)-1H-pyrrole-3-carboxylate d'éthyle en suspension dans 300 cm³ de dichlorométhane, on ajoute à une température voisine de -24°C, sous atmosphère d'argon, 8,1 cm³ (91,29 mmol) d'isocyanate de chlorosulfonyle en solution dans 100 cm³ de dichlorométhane. Après 30 minutes d'agitation à une température voisine de - 24°C, on laisse le milieu réactionnel évoluer en 2 h jusqu'à une température voisine de 20°C. Après avoir amorcé la cristallisation à l'aide d'une baguette de verre, le mélange réactionnel est agité à une température voisine de 20°C pendant 30 minutes. Le mélange réactionnel est ensuite refroidi à une température voisine de 5°C puis filtré sur verre fritté N°3. Le solide est lavé avec 3 fois 40 cm³ de dichlorométhane puis séché sous pression réduite (2,7 kPa), à une température voisine de 40°C pendant 3 heures. Le solide est alors mis en suspension dans 500 cm³ d'eau ; le mélange résultant est ensuite chauffé à une température voisine de 40°C pendant 3 heures puis refroidi à une température voisine de 5°C pendant 1 heure avant filtration sur verre fritté N°3. Le solide est lavé successivement avec 2 fois 50 cm³ d'eau et 2 fois 30 cm³ d'éther de pétrole puis séché sous pression réduite (2,7 kPa), à une température voisine de 40°C pendant 16 heures pour donner 15,4 g de 5-carbamoyl-4-(4-nitro-phényl)-1H-pyrrole-3-carboxylate d'éthyle, sous forme d'un solide beige; EI: m/z=303: M^{+.} ; m/z=274 [M-CH₂CH₃]^{+.}; m/z=258 [M-OCH₂CH₃]⁺; m/z=241 [258-OH]⁺ pic de base.

Le 4-(4-nitro-phényl)-1H-pyrrole-3-carboxylate d'éthyle peut être préparé de la manière suivante :
A une suspension de 0,512 g (12,8 mmol) d'hydrure de sodium à 60% dans de l'huile minérale dans 20 cm³ d'éther diéthylique, on ajoute goutte à goutte, à une température voisine de 20°C, sous atmosphère d'argon, un mélange de 2,212 g (10 mmol) de 4-nitrocinnamate d'éthyle et 1,991 g (10,2 mmol) de tosyl-méthyl isocyanate en solution dans un mélange de 18 cm³ de diméthylsulfoxyde et 36 cm³ d'éther diéthylique. Après 1 heure d'agitation à reflux, le mélange réactionnel est repris dans un mélange de 70 cm³ d'eau, 20 cm³ d'une solution aqueuse saturée en chlorure de sodium et 100 cm³ d'acétate d'éthyle. La phase aqueuse est extraite par 50 cm³ d'éther diéthylique et 2 fois 75 cm³ de dichlorométhane. Toutes les phases organiques sont réunies, séchées sur sulfate de sodium anhydre, filtrées et concentrées à sec sous pression réduite (2,7 kPa) pour donner une huile noire qui est reprise dans un mélange de 75 cm³ d'eau et 50 cm³ d'acétate d'éthyle. La phase aqueuse est extraite par 2 fois 50 cm³ d'acétate d'éthyle. Toutes les phases organiques sont réunies, séchées sur sulfate de sodium anhydre, filtrées et concentrées à sec sous pression réduite (2,7 kPa) pour donner 2,82 g d'un solide noir qui est purifié par chromatographie-flash [éluant : cyclohexane / acétate d'éthyle (3 / 2 en volumes)]. Après concentration des fractions sous pression réduite, on obtient 1,48 g d'un solide orange qui est purifié par chromatographie-flash [éluant : dichlorométhane]. Après concentration des fractions sous pression réduite, on obtient 0,78 g de 4-(4-nitro-phényl)-1H-pyrrole-3-carboxylate d'éthyle, sous forme d'un solide jaune ; IE: m/z = 260 (M^{+.}) pic de base, m/z= 215 (M - C₂H₅O⁺), m/z=169 (215-NO₂).

### Exemple 3

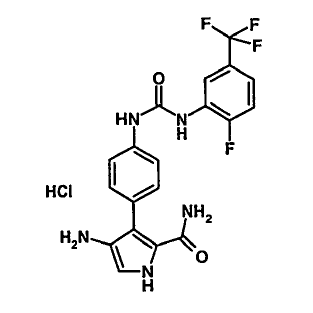

Chlorhydrate du 4-amino-3-{4-[3-(2-fluoro-5-trifluorométhyl-phényl)-uréido]-phényl}-1H-pyrrole-2-carboxamide

A 0,15 g (0,288 mmol) de 4-*tert*-butyloxycarbonylamino-3-{4-[3-(2-fluoro-5-trifluorométhyl-phényl)-uréido]-phényl}-1H-pyrrole-2-carboxamide en suspension dans 1 cm³ de dioxane, on ajoute à une température voisine de 20°C, 0,72 cm³ d'une solution d'acide chlorhydrique 4 N dans le dioxane. Après 5 heures d'agitation à une température voisine de 20°C, le mélange réactionnel est concentré à sec sous pression réduite (2,7 kPa) pour donner un résidu qui est purifié par chromatographie-flash [éluant : dichlorométhane / méthanol / acétonitrile (gradient de 90 / 5 / 5 à 80 / 10 / 10 en volumes)]. Après concentration des fractions sous pression réduite, on obtient 85 mg d'un solide jaune qui est ensuite agité dans 4 cm³ de pentane pendant 16h. Après filtration sur verre fritté N°3, le solide est lavé avec 2 fois 1 cm³ de pentane puis séché sous pression réduite (2,7 kPa), à une température voisine de 40°C pendant 4 heures pour donner 47 mg de chlorhydrate du 4-amino-3-{4-[3-(2-fluoro-5-trifluorométhyl-phényl)-uréido]-phényl}-1H-pyrrole-2-carboxamide, sous forme d'un solide jaune fondant vers 174°C ; R.M.N. 1H (400 MHz, DMSO d6, δ en ppm) de 5,30 à 8,00 (m très étalé, 2H) ; 6,34 (d, J = 2,5 Hz, 1H) ; 7,27 (d, J = 8,5 Hz, 2H) ; 7,39 (m, 1H) ; 7,50 (m partiellement masqué, 1H) ; 7,53 (d, J = 8,5 Hz, 2H) ; 8,63 (d large, J = 7,0 Hz, 1H) ; 8,96 (s large, 1H) ; 9,29 (s, 1H) ; 10,7 (s large, 1H): ; ES⁺: m/z=422: [M+H]⁺; ES^{-:} m/z=420: [M-H]⁻.

Le 4-*tert*-butyloxycarbonylamino-3-{4-[3-(2-fluoro-5-trifluorométhyl-phényl)-uréido]-phényl}-1H-pyrrole-2-carboxamide peut être préparé comme décrit dans l'exemple 2.

### Exemple 4

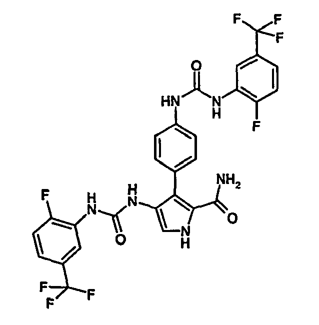

4-[3-(2-Fluoro-5-trifluorométhyl-phényl)-uréido]-3-{4-(3-(2-fluoro-5-trifluorométhyl-phényl)-uréido]-phényl}-1H-pyrrole-2-carboxamide

A 0,107 g (0,254 mmol) de chlorhydrate du 4-amino-3-{4-[3-(2-fluoro-5-trifluorométhyl-phényl)-uréido]-phényl}-1H-pyrrole-2-carboxamide en solution dans 10 cm³ de tétrahydrofurane, on ajoute à une température voisine de 20°C, sous atmosphère d'argon, 0,037 cm³ de 2-fluoro-5-trifluorométhylphényl isocyanate et 0,035 cm³ de triéthylamine. Après 1 heure d'agitation à une température voisine de 20°C, le mélange réactionnel est dilué avec 10 cm³ d'acétate d'éthyle puis lavé successivement avec 2 fois 15 cm³ d'eau et 15 cm³ d'une solution aqueuse saturée en chlorure de sodium, séché sur sulfate de magnésium anhydre, filtrée et concentré à sec sous pression réduite (2,7 kPa) pour donner 0,27 g d'un solide jaune qui est purifié par chromatographie-flash [éluant : cyclohexane / acétate d'éthyle (7 / 3 en volumes) puis dichlorométhane / méthanol / acétonitrile (90 / 5 / 5 en volumes)]. Après concentration des fractions sous pression réduite, on obtient 0,079 g de 4-[3-(2-fluoro-5-trifluorométhyl-phényl)-uréido]-3-{4-[3-(2-fluoro-5-trifluorométhyl-phényl)-uréido]-phényl}-1H-pyrrole-2-carboxamide, sous forme d'un solide blanc fondant entre 186 et 190°C ; R.M.N. 1H (400 MHz, DMSO d6, δ en ppm) 5,62 (m très étalé, 1H) ; 7,02 (m très étalé, 1H) ; 7,24 (d, J = 3,0 Hz, 1H) ; 7,30 (d, J = 8,5 Hz, 2H) ; 7,32 (m partiellement masqué, 1H) ; 7,39 (m, 1H) ; 7,42 (dd, J = 8,5 et 11,5 Hz, 1H) ; 7,51 (dd, J = 8,5 et 11,5 Hz, 1H) ; 7,61 (d, J = 8,5 Hz, 2H) ; 8,05 (s, 1H) ; 8,64 (m, 2H) ; 9,06 (s large, 1H) ; 9,12 (s large, 1H) ; 9,45 (s large, 1H) ; 11,3 (s large, 1H): ; ES⁺: m/z=627: [M+H]⁺.

Le chlorhydrate du 4-amino-3-{4-[3-(2-fluoro-5-trifluorométhyl-phényl)-uréido]-phényl}-1H-pyrrole-2-carboxamide peut être préparé comme décrit dans l'exemple 3.

### Exemple 5

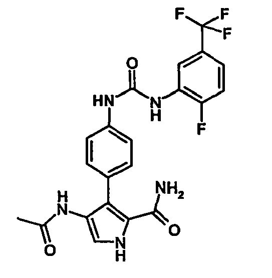

4-Acétylamino-3-{4-[3-(2-fluoro-5-trifluorométhyl-phényl)-uréido]-phényl}-1H-pyrrole-2-carboxamide

A 0,15 g (0,328 mmol) de chlorhydrate du 4-amino-3-{4-[3-(2-fluoro-5-trifluorométhyl-phényl)-uréido]-phényl}-1H-pyrrole-2-carboxamide en solution dans 4,5 cm³ de tétrahydrofurane, on ajoute à une température voisine de 20°C, sous atmosphère d'argon, 0,023 cm³ de chlorure d'acétyle, 0,091 cm³ de triéthylamine et 8 mg de 4-diméthylaminopyridine. Après 16 heures d'agitation à une température voisine de 20°C, le mélange réactionnel est dilué avec 15 cm³ de dichlorométhane puis lavé avec 2 fois 10 cm³ d'une solution aqueuse saturée en chlorure de sodium, séché sur sulfate de magnésium anhydre, filtrée et concentré à sec sous pression réduite (2,7 kPa) pour donner un solide qui est recristallisé à chaud dans 5 cm³ de dichlorométhane. Le solide recristallisé est filtré sur verre fritté N°3, lavé avec 2 fois 3 cm³ de dichlorométhane puis séché sous pression réduite (2,7 kPa), à une température voisine de 40°C pendant 5 heures pour donner 0,084 g de 4-acétylamino-3-{4-[3-(2-fluoro-5-trifluorométhyl-phényl)-uréido]-phényl}-1H-pyrrole-2-carboxamide, sous forme d'un solide blanc fondant à 228,5°C ; ES⁺: m/z=464: [M+H]⁺.

R.M.N. 1H (400 MHz, DMSO d6, δ en ppm) 1,87 (s, 3H) ; 5,62 (m étalé, 1H) ; 7,03 (m étalé, 1H) ; 7,10 (d, J = 3,0 Hz, 1H) ; 7,25 (d, J = 8,5 Hz, 2H) ; 7,39 (m, 1H) ; 7,50 (m partiellement masqué, 1H) ; 7,54 (d, J = 8,5 Hz, 2H) ; 8,62 (dd, J = 2,5 et 7,5 Hz, 1H) ; 8,70 (s, 1H) ; 9,02 (s large, 1H) ; 9,36 (s large, 1H) ; 11,3 (s large, 1H) ;

Le chlorhydrate du 4-amino-3-{4-[3-(2-fluoro-5-trifluorométhyl-phényl)-uréido]-phényl}-1H-pyrrole-2-carboxamide peut être préparé comme décrit dans l'exemple 3.

### Exemple 6

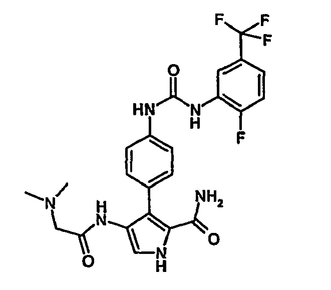

4-(2-Diméthylamino-acétylamino)-3-{4-[3-(2-fluoro-5-trifluorométhyl-phényl)-uréido]-phényl}-1H-pyrrole-2-carboxamide

A 0,15 g (0,328 mmol) de chlorhydrate du 4-amino-3-{4-[3-(2-fluoro-5-trifluorométhyl-phényl)-uréido]-phényl}-1H-pyrrole-2-carboxamide en solution dans 4,5 cm³ de tétrahydrofurane, on ajoute à une température voisine de 20°C, sous atmosphère d'argon, 0,052 g de chlorhydrate du chlorure de diméthylamino acétyle, 0,091 cm³ de triéthylamine et 8 mg de 4-diméthylaminopyridine. Après 16 heures d'agitation à une température voisine de 20°C, le mélange réactionnel est dilué avec 15 cm³ de dichlorométhane puis lavé avec 2 fois 10 cm³ d'une solution aqueuse saturée en chlorure de sodium, séché sur sulfate de magnésium anhydre, filtrée et concentré à sec sous pression réduite (2,7 kPa) pour donner 148 mg d'un solide beige qui est purifié par chromatographie-flash [éluant : dichlorométhane / méthanol / acétonitrile (82 / 9 / 9 en volumes)]. Après concentration des fractions sous pression réduite, on obtient 0,107 g de 4-(2-diméthylamino-acétylamino)-3-{4-[3-(2-fluoro-5-trifluorométhyl-phényl)-uréido]-phényl}-1H-pyrrole-2-carboxamide, sous forme d'un solide jaune fondant à 178°C ; ES⁺: m/z=507: [M+H]⁺.

R.M.N. 1H (400 MHz, DMSO d6, δ en ppm) 2,14 (s, 6H) ; 2,91 (s, 2H) ; 5,79 (m étalé, 1H) ; 7,06 (m étalé, 1H) ; de 7,25 à 7,31 (m, 3H) ; 7,39 (m, 1H) ; 7,50 (m, 1H) ; 7,58 (d, J = 8,5 Hz, 2H) ; 8,61 (m, 1H) ; 8,68 (s, 1H) ; 9,11 (s large, 1H) ; 9,49 (s large, 1H) ; 11,35 (s large, 1H);

Le chlorhydrate du 4-amino-3-{4-[3-(2-fluoro-5-trifluorométhyl-phényl)-uréido]-phényl}-1H-pyrrole-2-carboxamide peut être préparé comme décrit dans l'exemple 3.

### Exemple 7

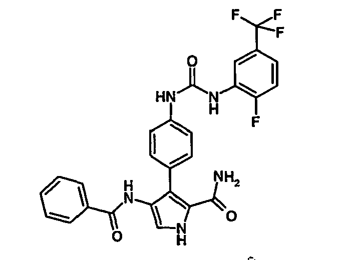

4-Benzoylamino-3-{4-[3-(2-fluoro-5-trifluorométhyl-phényl)-uréido]-phényl}-1H-pyrrole-2-carboxamide

A 0,15 g (0,328 mmol) de chlorhydrate du 4-amino-3-{4-[3-(2-fluoro-5-trifluorométhyl-phényl)-uréido]-phényl}-1H-pyrrole-2-carboxamide en solution dans 4,5 cm³ de tétrahydrofurane, on ajoute à une température voisine de 20°C, sous atmosphère d'argon, 0,038 cm³ de chlorure de benzoyle et 0,091 cm³ de triéthylamine. Après 4 heures d'agitation à une température voisine de 20°C, le mélange réactionnel est dilué avec 15 cm³ de dichlorométhane puis lavé avec 2 fois 10 cm³ d'une solution aqueuse saturée en chlorure de sodium, séché sur sulfate de magnésium anhydre, filtrée et concentré à sec sous pression réduite (2,7 kPa) pour donner un solide qui est recristallisé à chaud dans 5 cm³ de méthanol. Le solide recristallisé est filtré sur verre fritté N°3, lavé avec 2 fois 2 cm³ de méthanol puis séché sous pression réduite (2,7 kPa), à une température voisine de 35°C pendant 5 heures pour donner 0,075 g de 4-benzoylamino-3-{4-[3-(2-fluoro-5-trifluorométhyl-phényl)-uréido]-phényl}-1H-pyrrole-2-carboxamide, sous forme d'un solide blanc fondant à 239,7°C ; ES⁺: m/z=526: [M+H]⁺.

Le chlorhydrate du 4-amino-3-{4-[3-(2-fluoro-5-trifluorométhyl-phényl)-uréido]-phényl}-1H-pyrrole-2-carboxamide peut être préparé comme décrit dans l'exemple 3.

### Exemple 8

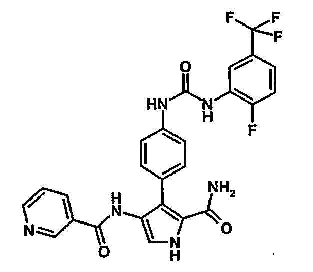

4-Nicotinoylamino-3-{4-[3-(2-fluoro-5-trifluorométhyl-phényl)-uréido]-phényl}-1H-pyrrole-2-carboxamide

A 0,15 g (0,328 mmol) de chlorhydrate du 4-amino-3-{4-[3-(2-fluoro-5-trifluorométhyl-phényl)-uréido]-phényl}-1H-pyrrole-2-carboxamide en solution dans 4,5 cm³ de tétrahydrofurane, on ajoute à une température voisine de 20°C, sous atmosphère d'argon, 0,058 g de chlorhydrate du chlorure de nicotinoyle, 0,091 cm³ de triéthylamine et 8 mg de 4-diméthylaminopyridine. Après 16 heures d'agitation à reflux, le mélange réactionnel est dilué avec 15 cm³ de dichlorométhane puis lavé avec 2 fois 10 cm³ d'une solution aqueuse saturée en chlorure de sodium, séché sur sulfate de magnésium anhydre, filtrée et concentré à sec sous pression réduite (2,7 kPa) pour donner 148 mg d'un solide beige qui est purifié par chromatographie-flash [éluant : dichlorométhane / méthanol / acétonitrile (82 / 9 / 9 en volumes)]. Après concentration des fractions sous pression réduite, on obtient 0,01 g de 4-nicotinoylamino-3-{4-[3-(2-fluoro-5-trifluorométhyl-phényl)-uréido]-phényl}-1H-pyrrole-2-carboxamide, sous forme d'un solide jaune ; R.M.N. 1H (400 MHz, DMSO d6, δ en ppm) 5,85 (m étalé, 1H) ; 7,12 (d, J = 3,0 Hz, 1H) ; 7,33 (d, J = 8,5 Hz, 2H) ; 7,38 (m, 1H) ; de 7,45 à 7,53 (m, 4H) ; 8,10 (m, 1H) ; 8,61 (m, 1H) ; 8,68 (dd, J = 1,5 et 5,0 Hz, 1H) ; 8,92 (d, J = 2,0 Hz, 1H) ; 8,93 (m, 1H) ; 9,25 (s, 1H) ; 9,52 (s, 1H) ; 11,5 (s large, 1H) ; ES⁺: m/z=527: [M+H]⁺.

Le chlorhydrate du 4-amino-3-{4-[3-(2-fluoro-5-trifluorométhyl-phényl)-uréido]-phényl}-1H-pyrrole-2-carboxamide peut être préparé comme décrit dans l'exemple 3.

### Exemple 9

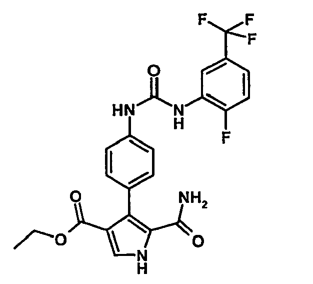

5-Carbamoyl-4-{4-[3-(2-fluoro-5-trifluorométhyl-phényl)-uréido]-phényl}-1H-pyrrole-3-carboxylate d'éthyle

A 0,23 g (0,842 mmol) de 5-carbamoyl-4-(4-amino-phényl)-1H-pyrrole-3-carboxylate d'éthyle en solution dans 30 cm³ de tétrahydrofurane, on ajoute à une température voisine de 20°C, sous atmosphère d'argon, 0,122 cm³ (0,842 mmol) de 2-fluoro-5-trifluorométhyl-phényl isocyanate. Après 2 heures d'agitation à une température voisine de 20°C, le mélange réactionnel est dilué avec 40 cm³ d'acétate d'éthyle puis lavé successivement avec 2 fois 30 cm³ d'eau et 30 cm³ d'une solution aqueuse saturée en chlorure de sodium, séché sur sulfate de magnésium anhydre, filtrée et concentré à sec sous pression réduite (2,7 kPa) pour donner 0,431 g d'un solide jaune qui est purifié par chromatographie-flash [éluant : dichlorométhane / méthanol / acétonitrile (94 / 3 / 3 en volumes)]. Après concentration des fractions sous pression réduite, on obtient 0,345 g d'un solide jaune qui est ensuite agité dans 5 cm³ de dichlorométhane pendant 1 heure. Après filtration sur verre fritté N°3, le solide est lavé avec 2 fois 3 cm³ de dichlorométhane puis séché sous pression réduite (2,7 kPa), à une température voisine de 30°C pendant 4 heures pour donner 0,333 g de 5-carbamoyl-4-{4-[3-(2-fluoro-5-trifluorométhyl-phényl)-uréido]-phényl}-1H-pyrrole-3-carboxylate d'éthyle, sous forme d'un solide jaune fondant à 179°C ; R.M.N. 1H (400 MHz, DMSO d6, δ en ppm) 1,05 (t, J = 7,0 Hz, 3H) ; 3,99 (q, J = 7,0 Hz, 2H) ; 5,45 (m étalé, 1H) ; 7,22 (d, J = 8,5 Hz, 2H) ; 7,25 (m partiellement masqué, 1H) ; 7,39 (m, 1H) ; 7,47 (s, 1H) ; 7,49 (m partiellement masqué, 1H) ; 7,51 (d, J = 8,5 Hz, 2H) ; 8,62 (dd, J = 2,5 et 7,5 Hz, 1H) ; 9,01 (s large, 1H) ; 9,36 (s, 1H) ; 12,1 (s large, 1H): ; ES⁺: m/z=479: [M+H]⁺; ES^{-:} m/z=477: [M-H]⁻.

Le 5-carbamoyl-4-(4-amino-phényl)-1H-pyrrole-3-carboxylate d'éthyle peut être préparé de la manière suivante :
A une suspension de 0,204 g (1,912 mmol) de palladium sur charbon à 10% dans 140 cm³ de méthanol, on ajoute à une température voisine de 20°C, 2,5 g (8,243 mmol) de 5-carbamoyl-4-(4-nitro-phényl)-1H-pyrrole-3-carboxylate d'éthyle. Après 4 heures d'hydrogénation en autoclave sous 4 bar d'hydrogène, à une température voisine de 35°C, le mélange réactionnel est filtré; le catalyseur est ensuite rincé avec 2 fois 20 cm³ de méthanol puis le filtrat est concentré à sec sous pression réduite (2,7 kPa) pour donner un résidu qui est engagé une seconde fois dans une réaction d'hydrogénation dans les mêmes conditions que précédemment. On obtient ainsi, après un traitement similaire à celui décrit ci-dessus, 1,93 g de 5-carbamoyl-4-(4-amino-phényl)-1H-pyrrole-3-carboxylate d'éthyle, sous forme d'un solide jaune; El: m/z=273: M^{+.} pic de base; m/z=256 [M-NH₃]^{+.}; m/z=228 [M-CONH₃]⁺.

Le 5-carbamoyl-4-(4-nitro-phényl)-1H-pyrrole-3-carboxylate d'éthyle peut être préparé comme décrit dans l'exemple 2.

Les produits des exemples 10 à 37 peuvent être préparés par analogie à l'exemple 9 à partir du 5-carbamoyl-4-(4-amino-phényl)-1H-pyrrole-3-carboxylate d'éthyle et de l'aryl isocyanate correspondant, les LCMS ont été faites sur un spectromètre LCT de Micromass, avec une colonne Highpurity C18 50 x 4,6 mm particules de 5 µm de chez Thermo, le gradient passant à 90 % en acétonitrile en 5 mn (conditions initiales: 95 % eau avec 0,5 % de TFA - 5 % acétonitrile) :

### Exemple 10

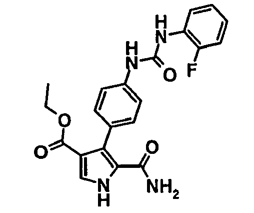

5-Carbamoyl-4-{4-[3-(2-fluoro-phényl)-uréido]-phényl}-1H-pyrrole-3-carboxylate d'éthyle

ES⁺: m/z = 411 [M+H]⁺, temps de rétention 4,3 min.

### Exemple 11

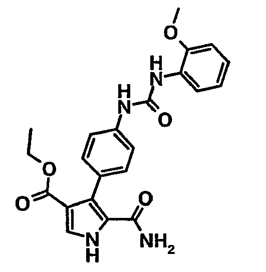

5-Carbamoyl-4-{4-[3-(2-méthoxy-phényl)-uréido]-phényl}-1H-pyrrole-3-carboxylate d'éthyle

ES⁺: m/z = 423 [M+H]⁺, temps de rétention 4,3 min.

### Exemple 12

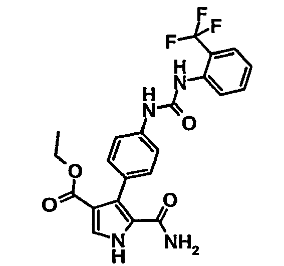

5-Carbamoyl-4-{4-[3-(2-trifluorométhyl-phényl)-uréido]-phényl}-1H-pyrrole-3-carboxylate d'éthyle

ES⁺: m/z = 461 [M+H]⁺ , temps de rétention 4,5 min.

### Exemple 13

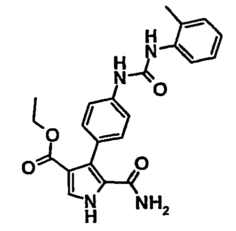

5-Carbamoyl-4-[4-(3-o-tolyl-uréido)-phényl]-1H-pyrrole-3-carboxylate d'éthyle ES⁺: m/z = 407 [M+H]⁺ , temps de rétention 4,2 min.

### Exemple 14

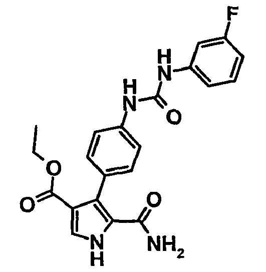

5-Carbamoyl-4-{4-[3-(3-fluoro-phényl)-uréido]-phényl}-1H-pyrrole-3-carboxylate d'éthyle

ES⁺: m/z = 411 [M+H]⁺ , temps de rétention 4,3 min.

### Exemple 15

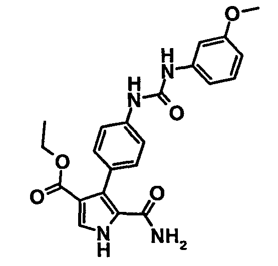

5-Carbamoyl-4-{4-[3-(3-méthoxy-phényl)-uréido]-phényl}-1H-pyrrole-3-carboxylate d'éthyle

ES⁺: m/z = 423 [M+H]⁺ , temps de rétention 4,2 min.

### Exemple 16

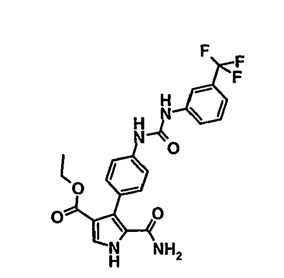

5-Carbamoyl-4-{4-[3-(3-trifluorométhyl-phényl)-uréido]-phényl}-1H-pyrrole-3-carboxylate d'éthyle

ES⁺: m/z = 461 [M+H]⁺ , temps de rétention 4,7 min.

### Exemple 17

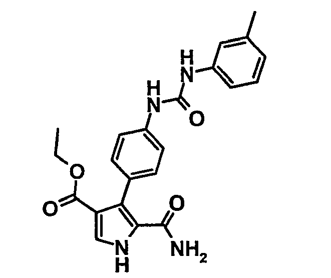

5-Carbamoyl-4-[4-(3-m-tolyl-uréido)-phényl]-1H-pyrrole-3-carboxylate d'éthyle ES⁺: m/z = 407 [M+H]⁺, temps de rétention 4,4 min.

### Exemple 18

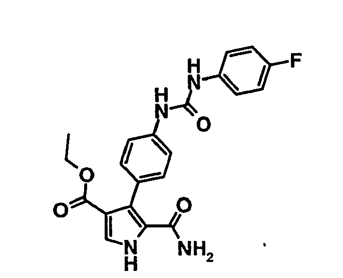

5-Carbamoyl-4-{4-[3-(4-fluoro-phényl)uréido]-phényl}-1H-pyrrole-3-carboxylate d'éthyle

ES⁺: m/z = 411 [M+H]⁺, temps de rétention 4,2 min.

### Exemple 19

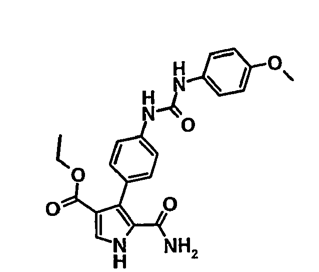

5-Carbamoyl-4-{4-[3-(4-méthoxy-phényl)-uréido]-phényl}-1H-pyrrole-3-carboxylate d'éthyle

ES⁺: m/z = 423 [M+H]⁺, temps de rétention 4,1 min.

### Exemple 20

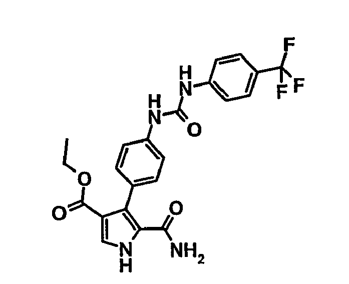

5-Carbamoyl-4-{4-[3-(4-trifluorométhyl-phényl)-uréido]-phényl}-1H-pyrrole-3-carboxylate d'éthyle

ES⁺: m/z = 461 [M+H]⁺, temps de rétention 4,8 min.

### Exemple 21

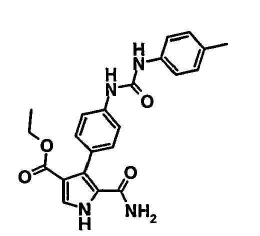

5-Carbamoyl-4-[4-(3-p-tolyl-uréido)-phényl]-1H-pyrrole-3-carboxylate d'éthyle ES⁺: m/z = 407 [M+H]⁺, temps de rétention 4,4 min.

### Exemple 22

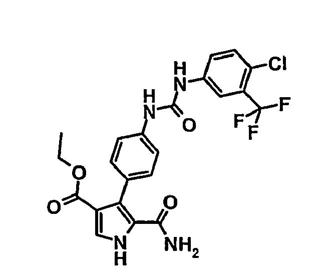

5-Carbamoyl-4-{4-[3-(4-chloro-3-trifluorométhyl-phényl)-uréido]-phényl}-1H-pyrrole-3-carboxylate d'éthyle

ES⁺: m/z = 495 [M+H]⁺, temps de rétention 5 min.

### Exemple 23

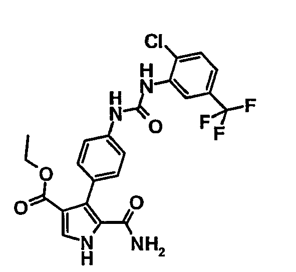

5-Carbamoyl-4-{4-[3-(2-chloro-5-trifluorométhyl-phényl)-uréido]-phényl}-1H-pyrrole-3-carboxylate d'éthyle

ES⁺: m/z = 495 [M+H]⁺, temps de rétention 5 min.

### Exemple 24

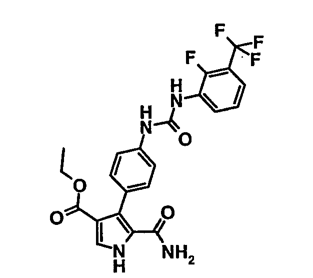

5-Carbamoyl-4-{4-[3-(2-fluoro-3-trifluorométhyl-phényl)-uréido]-phényl}-1H-pyrrole-3-carboxylate d'éthyle

ES⁺: m/z = 479 [M+H]⁺, temps de rétention 4,8 min.

### Exemple 25

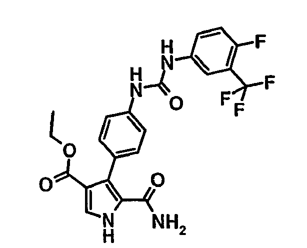

5-Carbamoyl-4-{4-[3-(4-fluoro-3-trifluorométhyl-phényl)-uréido]-phényl}-1H-pyrrole-3-carboxylate d'éthyle

ES⁺: m/z = 479 [M+H]⁺, temps de rétention 4,8 min.

### Exemple 26

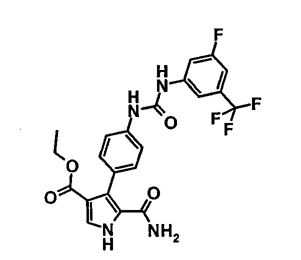

5-Carbamoyl-4-{4-[3-(3-fluoro-5-trifluorométhyl-phényl)-uréido]-phényl}-1H-pyrrole-3-carboxylate d'éthyle

ES⁺: m/z = 479 [M+H]⁺, temps de rétention 4,9 min.

### Exemple 27

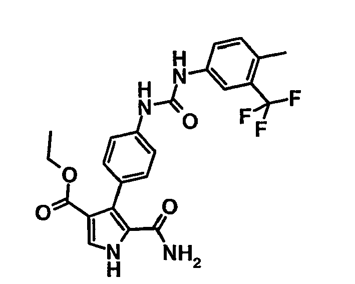

5-Carbamoyl-4-{4-[3-(4-méthyl-3-trifluorométhyl-phényl)-uréido]-phényl}-1H-pyrrole-3-carboxylate d'éthyle

ES⁺: m/z = 475 [M+H]⁺, temps de rétention 4,9 min.

### Exemple 28

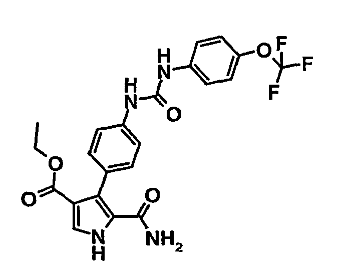

5-Carbamoyl-4-{4-[3-(4-trifluorométhoxy-phényl)-uréido]-phényl}-1H-pyrrole-3-carboxylate d'éthyle

ES⁺: m/z = 477 [M+H]⁺, temps de rétention 4,8 min.

### Exemple 29

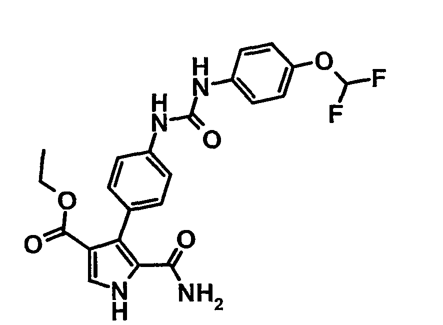

5-Carbamoyl-4-{4-[3-(4-difluorométhoxy-phényl)-uréido]-phényl}-1H-pyrrole-3-carboxylate d'éthyle

ES⁺: m/z = 459 [M+H]⁺, temps de rétention 4,4 min.

### Exemple 30

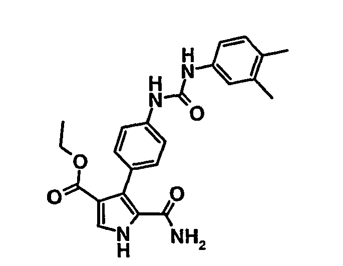

5-Carbamoyl-4-{4-[3-(3,4-diméthyl-phényl)-uréido]-phényl}-1H-pyrrole-3-carboxylate d'éthyle

ES⁺: m/z = 421 [M+H]⁺, temps de rétention 4,5 min.

### Exemple 31

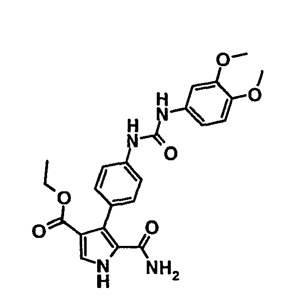

5-Carbamoyl-4-{4-[3-(3,4-diméthoxy-phényl)-uréido]-phényl}-1H-pyrrole-3-carboxylate d'éthyle

ES⁺: m/z = 453 [M+H]⁺, temps de rétention 3,8 min.

### Exemple 32

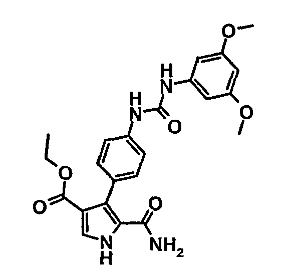

5-Carbamoyl-4-{4-[3-(3,5-diméthoxy-phényl)-uréido]-phényl}-1H-pyrrole-3-carboxylate d'éthyle

ES⁺: m/z = 453 [M+H]⁺, temps de rétention 4,2 min.

### Exemple 33

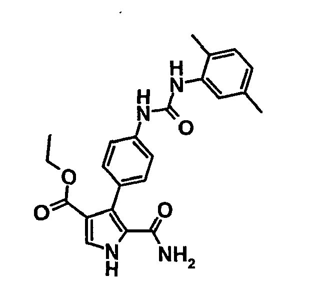

5-Carbamoyl-4-{4-[3-(2,5-diméthyl-phényl)-uréido]-phényl}-1H-pyrrole-3-carboxylate d'éthyle

ES⁺: m/z = 421 [M+H]⁺ , temps de rétention 4,5 min.

### Exemple 34

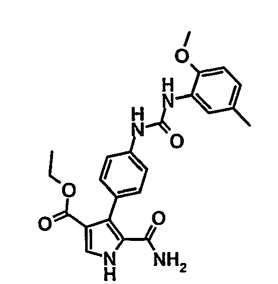

5-Carbamoyl-4-{4-[3-(2-méthoxy-5-méthyl-phényl)-uréido]-phényl}-1H-pyrrole-3-carboxylate d'éthyle

ES⁺: m/z = 437 [M+H]⁺ , temps de rétention 4,6 min.

### Exemple 35

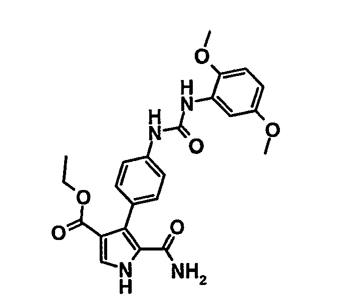

5-Carbamoyl-4-{4-[3-(2,5-diméthoxy-phényl)-uréido]-phényl}-1H-pyrrole-3-carboxylate d'éthyle

ES⁺: m/z = 453 [M+H]⁺ , temps de rétention 4,3 min.

### Exemple 36

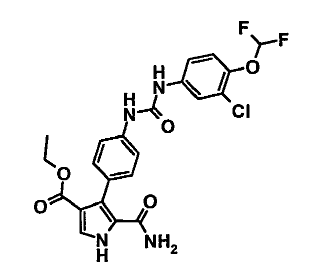

5-Carbamoyl-4-{4-[3-(3-chloro-4-difluorométhoxy-phényl)-uréido]-phényl}-1H-pyrrole-3-carboxylate d'éthyle

ES⁺: m/z = 493 [M+H]⁺, temps de rétention 4,7 min.

### Exemple 37

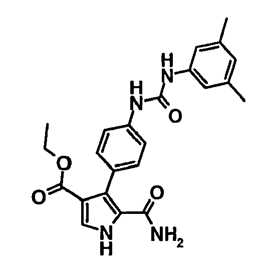

5-Carbamoyl-4-{4-[3-(3,5-diméthyl-phényl)-uréido]-phényl}-1H-pyrrole-3-carboxylate d'éthyle

ES⁺: m/z = 421 [M+H]⁺ , temps de rétention 4,6 min.

### Exemple 38

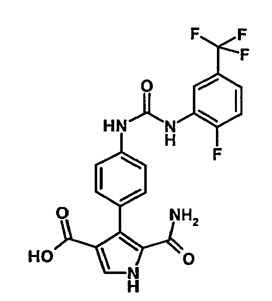

Acide 5-carbamoyl-4-{4-[3-(2-fluoro-5-trifluorométhyl-phényl)-uréido]-phényl}-1H-pyrrole-3-carboxylique

A 0,14 g (0,497 mmol) de chlorhydrate de l'acide 5-carbamoyl-4-(4-amino-phényl)-1H-pyrrole-3-carboxylique en solution dans 18 cm³ de tétrahydrofurane, on ajoute à une température voisine de 20°C, sous atmosphère d'argon, 0,072 cm³ (0,497 mmol) de 2-fluoro-5-trifluorométhyl-phényl isocyanate et 0,07 cm³ (0,497 mmol) de triéthylamine. Après 5 heures d'agitation à une température voisine de 20°C, le mélange réactionnel est concentré à sec sous pression réduite (2,7 kPa) pour donner un résidu qui est purifié par chromatographie-flash [éluant : dichlorométhane / méthanol / acétonitrile (gradient de 90 / 5 / 5 à 80 / 10 / 10 en volumes)]. Après concentration des fractions sous pression réduite, on obtient un solide qui est ensuite agité dans 5 cm³ de pentane pendant 3 heures. Après filtration sur verre fritté N°3, le solide est lavé avec 2 fois 1 cm³ de pentane puis séché sous pression réduite (2,7 kPa), à une température voisine de 30°C pendant 15 heures pour donner 0,105 g d'acide 5-carbamoyl-4-{4-[3-(2-fluoro-5-trifluorométhyl-phényl)-uréido]-phényl}-1H-pyrrole-3-carboxylique, sous forme d'un solide jaune fondant à 194°C ; R.M.N. 1H (400 MHz, DMSO d6, δ en ppm) : 5,37 (m étalé, 1H) ; 7,23 (d, J = 8,5 Hz, 2H) ; 7,23 (m masqué, 1H) ; 7,39 (m, 1H) ; 7,42 (d, J = 3,0 Hz, 1H) ; 7,49 (d, J = 8,5 Hz, 2H) ; 7,50 (m partiellement masqué, 1H) ; 8,64 (dd, J = 2,5 et 7,5 Hz, 1H) ; 8,97 (d, J = 2,0 Hz, 1H) ; 9,35 (s, 1H) ; 11,6 (m étalé, 1H) ; 12,05 (s large, 1H); ES⁺: m/z=451: [M+H]⁺ ; ES^{-:} m/z=449: [M-H]⁻.

Le chlorhydrate de l'acide 5-carbamoyl-4-(4-amino-phényl)-1H-pyrrole-3-carboxylique peut être préparé de la manière suivante :
A une suspension de 0,135 g (0,126 mmol) de palladium sur charbon à 10% dans 20 cm³ de méthanol, on ajoute à une température voisine de 20°C, 0,15 g (0,545 mmol) d'acide 5-carbamoyl-4-(4-nitro-phényl)-1H-pyrrole-3-carboxylique. Après 3 heures d'hydrogénation en autoclave sous 3 bar d'hydrogène, à une température voisine de 30°C, le mélange réactionnel est repris avec 5 cm³ d'une solution d'acide chlorhydrique 4N dans le dioxane puis filtré. Le catalyseur est rincé avec 2 fois 2 cm³ d'une solution d'acide chlorhydrique 4N dans le dioxane puis le filtrat est concentré à sec sous pression réduite (2,7 kPa) pour donner 0,141 g de chlorhydrate de l'acide 5-carbamoyl-4-(4-amino-phényl)-1H-pyrrole-3-carboxylique, sous forme d'un solide jaune; ES⁺: m/z=246: [M+H]⁺

L'acide 5-carbamoyl-4-(4-nitro-phényl)-1H-pyrrole-3-carboxylique peut être préparé comme décrit dans l'exemple 2.

### Exemple 39

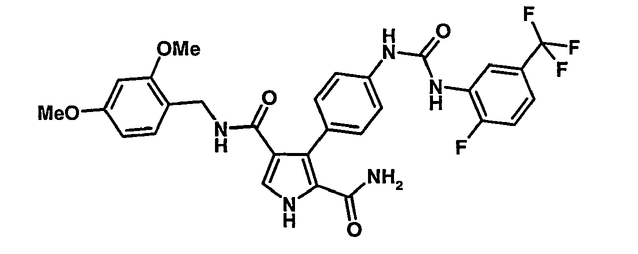

4-(2,4-Diméthoxy-benzylamino)carbonyl-3-{4-[3-(2-fluoro-5-trifluorométhyl-phényl)-uréido]-phényl}-1H-pyrrole-2-carboxamide

A 0,321 cm³ (2,09 mmol) de 2,4-diméthoxy-benzylamine en solution dans 10 cm³ de toluène, on ajoute à une température voisine de 20°C, sous atmosphère d'argon, 1,36 cm³ (2,717 mmol) d'une solution de triméthylaluminium 2M dans le toluène puis 0,5 g (1,045 mmol) de 5-carbamoyl-4-{4-[3-(2-fluoro-5-trifluorométhyl-phényl)-uréido]-phényl}-1H-pyrrole-3-carboxylate d'éthyle. Après 16 heures d'agitation à une température voisine de 60°C, le mélange réactionnel est dilué avec 50 cm³ d'acétate d'éthyle, puis lavé successivement avec 2 fois 30 cm³ d'une solution aqueuse saturée en chlorure d'ammonium et 2 fois 25 cm³ d'eau. La phase organique obtenue est séchée sur sulfate de magnésium anhydre, filtrée et concentrée à sec sous pression réduite (2,7 kPa) pour donner 0,52 g d'un solide jaune qui est purifié par chromatographie-flash [éluant : dichlorométhane / méthanol / acétonitrile (90 / 5 / 5 en volumes)]. Après concentration des fractions sous pression réduite, on obtient 0,170 g de 4-(2,4-diméthoxybenzylamino)carbonyl-3-{4-[3-(2-fluoro-5-trifluorométhyl-phényl)-uréido]-phényl}-1H-pyrrole-2-carboxamide, sous forme d'un solide jaune fondant à une température supérieure à 260°C ; R.M.N. 1H (400 MHz, DMSO d6, δ en ppm) : 3,68 (s, 3H) ; 3,70 (s, 3H) ; 4,12 (d, J = 6,0 Hz, 2H) ; 5,34 (m étalé, 1H) ; 6,40 (dd, J = 2,5 et 8,5 Hz, 1H) ; 6,46 (d, J = 2,5 Hz, 1H) ; 6,86 (d, J = 8,5 Hz, 1H) ; 6,99 (t, J = 6,0 Hz, 1H) ; 7,17 (m étalé, 1H) ; 7,23 (d, J = 8,5 Hz, 2H) ; 7,39 (m, 1H) ; 7,43 (d, J = 2,5 Hz, 1H) ; 7,50 (m partiellement masqué, 1H) ; 7,51 (d, J = 8,5 Hz, 2H) ; 8,64 (dd, J = 2,5 et 7,5 Hz, 1H) ; 9,00 (s large, 1H) ; 9,36 (s large, 1H) ; 11,85 (s large, 1H); ES⁺: m/z=600: [M+H]⁺.

Le 5-carbamoyl-4-{4-[3-(2-fluoro-5-trifluorométhyl-phényl)-uréido]-phényl}-1H-pyrrole-3-carboxylate d'éthyle peut être préparé comme décrit dans l'exemple 9.

### Exemple 40

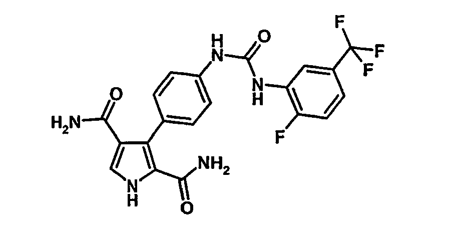

3-{4-[3-(2-Fluoro-5-trifluorométhyl-phényl)-uréido]-phényl}-1H-pyrrole-2,4-dicarboxamide

A 0,15 g (0,25 mmol) de 4-(2,4-diméthoxy-benzylamino)carbonyl-3-{4-[3-(2-fluoro-5-trifluorométhyl-phényl)-uréido]-phényl}-1H-pyrrole-2-carboxamide en solution dans 3 cm³ de toluène, on ajoute à une température voisine de 20°C, sous atmosphère d'argon, 0,108 g d'acide p-toluène sulfonique monohydrate. Après 48 heures d'agitation à une température voisine de 65°C, le mélange réactionnel est dilué avec 0,25 cm³ de méthanol puis le pH est ajusté à une valeur comprise entre 12 et 13 par addition d'une solution aqueuse d'hydroxyde de sodium 1N. Le milieu réactionnel est extrait avec 3 fois 15 cm³ d'acétate d'éthyle. La phase organique est lavée successivement avec 20 cm³ d'eau et 20 cm³ d'une solution aqueuse saturée en chlorure sodium, puis est séchée sur sulfate de magnésium anhydre, filtrée et concentrée à sec sous pression réduite (2,7 kPa) pour donner 0,175 g d'un solide jaune qui est purifié par chromatographie-flash [éluant : dichlorométhane / méthanol / acétonitrile (90 / 5 / 5 en volumes)]. Après concentration des fractions sous pression réduite, on obtient 0,047 g de 3-{4-[3-(2-fluoro-5-trifluorométhyl-phényl)-uréido]-phényl}-1H-pyrrole-2,4-di-carboxamide, sous forme d'un solide blanc fondant à 196°C ; R.M.N. 1H (400 MHz, DMSO d6, δ en ppm) : 5,32 (m étalé, 1H) ; 6,41 à 6,77 (m étalé, 2H) ; 7,15 (m étalé, 1H) ; 7,24 (d, J = 8,5 Hz, 2H) ; 7,39 (m, 1H) ; 7,45 (s large, 1H) ; 7,50 (m partiellement masqué, 1H) ; 7,52 (d, J = 8,5 Hz, 2H) ; 8,62 (dd, J = 2,0 et 7,5 Hz, 1H) ; 9,11 (s large, 1H) ; 9,46 (s large, 1H) ; 11,85 (s large, 1H); ES⁺: m/z=450: [M+H]⁺ .

Le 4-(2,4-diméthoxy-benzylamino)carbonyl-3-{4-[3-(2-fluoro-5-trifluorométhylphényl)-uréido]-phényl}-1H-pyrrole-2-carboxamide peut être préparé comme décrit dans l'exemple 39.

### Exemple 41

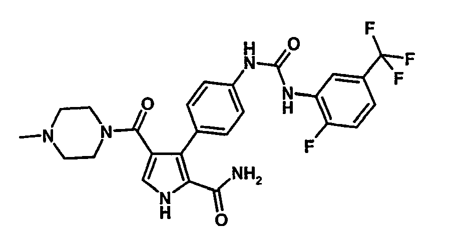

3-{4-[3-(2-Fluoro-5-trifluorométhyl-phényl)-uréido]-phényl}-4-(4-méthyl-pipérazine-1-carbonyl)-1H-pyrrole-2-carboxamide

On opère d'une façon analogue à celle décrite à l'exemple 39 mais à partir de N-méthylpipérazine et de 5-carbamoyl-4-{4-[3-(2-fluoro-5-trifluorométhyl-phényl)-uréido]-phényl}-1H-pyrrole-3-carboxylate d'éthyle. Après un traitement similaire, on obtient le 3-{4-[3-(2-fluoro-5-trifluorométhyl-phényl)-uréido]-phényl}-4-(4-méthyl-pipérazine-1-carbonyl)-1H-pyrrole-2-carboxamide ; ES⁺: m/z = 533 [M+H]⁺.

Le 5-carbamoyl-4-{4-[3-(2-fluoro-5-trifluorométhyl-phényl)-uréido]-phényl}-1H-pyrrole-3-carboxylate d'éthyle peut être préparé comme décrit dans l'exemple 9.

### Exemple 42

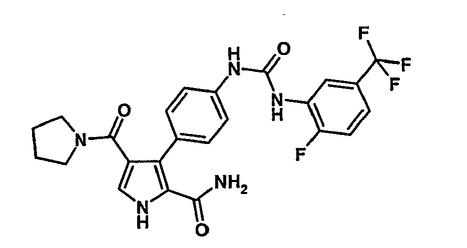

3-{4-[3-(2-Fluoro-5-trifluorométhyl-phényl)-uréido]-phényl}-4-(pyrrolidine-1-carbonyl)-1H-pyrrole-2-carboxamide

On opère d'une façon analogue à celle décrite à l'exemple 39 mais à partir de pyrrolidine et de 5-carbamoyl-4-{4-[3-(2-fluoro-5-trifluorométhyl-phényl)-uréido]-phényl}-1H-pyrrole-3-carboxylate d'éthyle. Après un traitement similaire, on obtient le 3-{4-[3-(2-fluoro-5-trifluorométhyl-phényl)-uréido]-phényl}-4-(pyrrolidine-1-carbonyl)-1H-pyrrole-2-carboxamide; ES⁺: m/z = 504 [M+H]⁺.

Le 5-carbamoyl-4-{4-[3-(2-fluoro-5-trifluorométhyl-phényl)-uréido]-phényl}-1H-pyrrole-3-carboxylate d'éthyle peut être préparé comme décrit dans l'exemple 9.

### Exemple 43

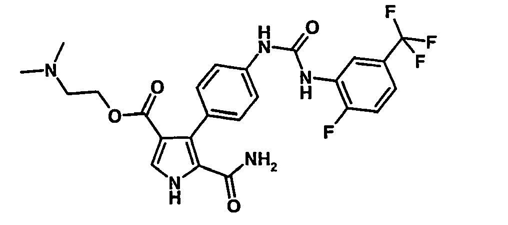

5-Carbamoyl-4-{4-[3-(2-fluoro-5-trifluorométhyl-phényl)-uréido]-phényl}-1H-pyrrole-3-carboxylate de 2-diméthylamino-éthyle

A 0,12 g (0,266 mmol) de 5-carbamoyl-4-{4-[3-(2-fluoro-5-trifluorométhyl-phényl)-uréido]-phényl}-1H-pyrrole-3-carboxylate d'éthyle en solution dans 4 cm³ de diméthylformamide, on ajoute à une température voisine de 20°C, sous atmosphère d'argon, 0,06 cm³ de 2-diméthylamino-éthanol, 0,085 cm³ de triéthylamine, 112 mg de chlorhydrate 1-(3-diméthylaminopropyl)-3-éthylcarbodiimide et 80 mg d'hydrate de 1-hydroxybenzotriazole . Après 3 heures d'agitation à une température voisine de 70°C, le mélange réactionnel est coulé sur 40 cm³ d'eau distillé puis agité 1 heure à une température voisine de 20°C. Le mélange réactionnel est filtré sur verre fritté N°3, le solide est lavé avec 2 fois 5 cm³ d'eau puis séché à l'air pour donner 0,065 g d'un solide blanc qui est purifié par chromatographie-flash [éluant : dichlorométhane / méthanol / ammoniaque aqueux 28% (12 / 2,25 / 0,38 en volumes)]. Après concentration des fractions sous pression réduite, on obtient 0,04 g de 5-carbamoyl-4-{4-[3-(2-fluoro-5-trifluorométhyl-phényl)-uréido]-phényl}-1H-pyrrole-3-carboxylate de 2-diméthylamino-éthyle, sous forme d'un solide blanc ; ES⁺: m/z = 522 [M+H]⁺, temps de rétention 6,61 min.

Le 5-carbamoyl-4-{4-[3-(2-fluoro-5-trifluorométhyl-phényl)-uréido]-phényl}-1H-pyrrole-3-carboxylate d'éthyle peut être préparé comme décrit dans l'exemple 9.

### Exemple 44

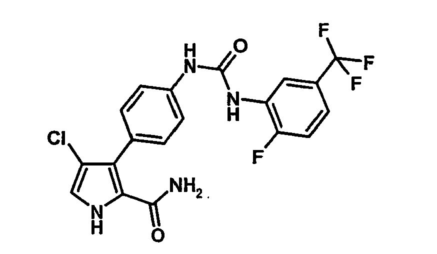

4-Chloro-3-{4-[3-(2-fluoro-5-trifluorométhyl-phényl)-uréido]-phényl}-1H-pyrrole-2-carboxamide

A 0,03 g (0,127 mmol) de 4-chloro-3-(4-amino-phényl)-1H-pyrrole-2-carboxamide en solution dans 2,7 cm³ de tétrahydrofurane, on ajoute à une température voisine de 20°C, sous atmosphère d'argon, 0,019 cm³ (0,134 mmol) de 2-fluoro-5-trifluorométhyl-phényl isocyanate. Après 2 heures d'agitation à une température voisine de 20°C, le mélange réactionnel est concentré à sec sous pression réduite (2,7 kPa) pour donner un résidu qui est recristallisé dans 4 cm³ d'acétonitrile. Après filtration sur verre fritté N°3, le solide est lavé avec 2 cm³ d'acétonitrile puis séché sous pression réduite (2,7 kPa), à une température voisine de 45°C pendant 2 heures pour donner 0,037 g de 4-chloro-3-{4-[3-(2-fluoro-5-trifluorométhyl-phényl)-uréido]-phényl}-1H-pyrrole-2-carboxamide, sous forme d'un solide blanc; R.M.N. 1H (400 MHz, DMSO d6, δ en ppm) : 5,83 (m étalé, 1H) ; 7,08 (s, 1H) ; 7,18 (m étalé, 1H) ; 7,28 (d, J = 8,5 Hz, 2H) ; 7,38 (m, 1H) ; 7,50 (m partiellement masqué, 1H) ; 7,55 (d, J = 8,5 Hz, 2H) ; 8,62 (dd, J = 2,0 et 7,5 Hz, 1H) ; 9,04 (s large, 1H) ; 9,39 (s large, 1H) ; 11,8 (s large, 1H); ES⁺: m/z=441: [M+H]⁺ ; ES^{-:} m/z=439: [M-H]⁻.

Le 4-chloro-3-(4-amino-phényl)-1H-pyrrole-2-carboxamide peut être préparé de la manière suivante :
A 0,136 g (0,512 mmol) de 4-chloro-3-(4-nitro-phényl)-1H-pyrrole-2-carboxamide en solution dans 2,8 cm³ d'acide acétique, on ajoute à une température voisine de 20°C, sous atmosphère d'argon, 134 mg (2,048 mmol) de zinc en poudre. Après 2 heures d'agitation à une température voisine de 20°C, le mélange réactionnel est dilué avec 20 cm³ d'acétate d'éthyle et 15 cm³ d'eau puis le pH est ajusté à une valeur comprise entre 8 et 9 par addition d'une solution aqueuse d'hydroxyde de sodium 1N. La phase aqueuse est extraite avec 2 fois 15 cm³ d'acétate d'éthyle. , Les phases organiques réunies sont séchées sur sulfate de magnésium anhydre, filtrées et concentrés à sec sous pression réduite (2,7 kPa) pour donner 0,145 g d'un solide orange qui est purifié par chromatographie-flash [éluant : dichlorométhane / méthanol / acétonitrile (90 / 5 / 5 en volumes)]. Après concentration des fractions sous pression réduite, on obtient 0,03 g de 4-chloro-3-(4-amino-phényl)-1H-pyrrole-2-carboxamide, sous forme d'un solide beige; El: m/z=235: M^{+.} ; m/z=218 [M-NH2]^{+.}; m/z=155 [M-Cl-CONH₃]⁺; m/z=44 [CONH₂]⁺ pic de base.

Les 4-chloro-3-(4-nitro-phényl)-1H-pyrrole-2-carboxamide et 5-chloro-3-(4-nitro-phényl)-1H-pyrrole-2-carboxamide peuvent être préparés de la manière suivante :
A 0,495 g (2,141 mmol) de 3-(4-nitro-phényl)-1H-pyrrole-2-carboxamide en solution dans 13,7 cm³ d'acétonitrile, on ajoute à une température voisine de 20°C, sous atmosphère d'argon, 286 mg (1,868 mmol) de N-chlorosuccinimide en poudre. Après 16 heures d'agitation à une température voisine de 65°C, le mélange réactionnel est dilué avec 40 cm³ d'acétate d'éthyle puis lavé successivement avec 3 fois 20 cm³ d'eau et 20 cm³ d'une solution aqueuse saturée en chlorure sodium. La phase organique résutante est puis séchée sur sulfate de magnésium anhydre, filtrée et concentré à sec sous pression réduite (2,7 kPa) pour donner 0,45 g d'un solide rouge qui est purifié par chromatographie-flash [éluant : dichlorométhane / méthanol / acétonitrile (98 / 1 / 1 en volumes)]. Après concentration des fractions sous pression réduite, on obtient 0,3 g d'un mélange de 4-chloro-3-(4-nitro-phényl)-1H-pyrrole-2-carboxamide et 5-chloro-3-(4-nitro-phényl)-1H-pyrrole-2-carboxamide dans les proportions respectives 55 / 45, sous forme d'un solide orange qui est purifié par LC-MS HPLC préparative :

| **Technique** | **Détection** | **Phase mobile A** | **Phase mobile B** |
|---|---|---|---|
| LC-MS préparative | Barette de diode | H₂O + 0,1%TFA pH=1 | acétonitrile |

| **Gradient** | | |
|---|---|---|
| Temps min | Débit ml/min | % acétonitrile |
| 0 | 20 | 25 |
| 1 | 20 | 25 |
| 3 | 30 | 25 |
| 11 | 30 | 40 |
| 12 | 30 | 100 |
| 12.80 | 20 | 100 |

• Les deux isomères sont séparés par chromatographie :

| **Technique** | **Détection** | **Phase stationnaire** | **Phase mobile** | **Débit** |
|---|---|---|---|---|
| Prochrom 2 | UV | WHELK01.SS | 70% Heptane; | 92 ml/min |
| Colonne AXXIAL | 254nm | MIXTE,700 G 350 x 80 mm, 10 µm | 28% CH₂Cl₂; 2% MeOH; 0.1 % TEA | |

• Pureté chimique

| **Technique** | **Détection** | **Phase stationnaire** | **Phase mobile** | **Débit** |
|---|---|---|---|---|
| Platform | TIC | Thermo hypersil gold 50 x 3 mm, 3 µm | Gradient 7 min A: H₂O + HCOOH 0,1 % B; acétonitrile | 0,8 ml/min |

• Contrôles analytiques, Pureté HPLC sur phase inverse :

| **Technique** | **Détection** | **Phase stationnaire** | **Phase mobile** | **Débit** |
|---|---|---|---|---|
| Agilent 1 | UV 265 nm | Whelk01.SS 10µm 250 x 4.6 mm | 70% Heptane; 28% CH₂Cl₂; 2% MeOH; 0.1% TEA | 1 ml/min |

| | **Isomère 5-Cl** | **Isomère 4-Cl** |
|---|---|---|
| **Temps de rétention** | 16, 23 min | 18,59 min |
| **Pureté** | > 99% | 98% |

On obtient ainsi 0,136 g de 4-chloro-3-(4-nitro-phényl)-1H-pyrrole-2-carboxamide, sous forme d'un solide jaune ; El: m/z=265: M^{+.} pic de base; m/z=248 [M-NH₃]^{+.}; m/z=218 [248-NO]⁺. et 0,120 mg de 5-chloro-3-(4-nitro-phényl)-1H-pyrrole-2-carboxamide, sous forme d'un solide jaune ; El: m/z=265: M^{+.} pic de base; m/z=248 [M-NH₃]^{+.}; m/z=218 [248-NO]⁺.

Le 3-(4-nitro-phényl)-1H-pyrrole-2-carboxamide peut être préparé de la manière suivante :
A 1,96 g (5,139 mmol) de 2-(2,4-diméthoxy-benzylamino)carbonyl-3-(4-nitro-phényl)-1H-pyrrole en solution dans 55 cm³ de toluène, on ajoute à une température voisine de 20°C, sous atmosphère d'argon, 2,224 g (11,69 mmol) d'acide p-toluène sulfonique monohydrate. Après 4,5 heures d'agitation à une température voisine de 65°C, le mélange réactionnel est dilué avec 70 cm³ d'acétate d'éthyle puis lavé successivement avec 3 fois 50 cm³ d'eau et 50 cm³ d'une solution aqueuse saturée en chlorure sodium. La phase organique résultante est séchée sur sulfate de magnésium anhydre, filtrée et concentrée à sec sous pression réduite (2,7 kPa) pour donner un solide jaune qui est ensuite agité dans 15 cm³ d'acétonitrile pendant 16 heures. Après filtration sur verre fritté N°3, le solide est lavé avec 2 fois 4 cm³ d'acétonitrile glacé puis séché sous pression réduite (2,7 kPa) à une température voisine de 35°C pendant 5 heures pour donner 0,5 g de 3-(4-nitro-phényl)-1H-pyrrole-2-carboxamide, sous forme d'un solide ocre; El: m/z=231: M^{+.} pic de base; m/z=214 [M-NH3]^{+.}; m/z=184 [M-HNO₂]⁺.

Le 2-(2,4-diméthoxy-benzylamino)carbonyl-3-(4-nitro-phényl)-1H-pyrrole peut être préparé de la manière suivante :
A 2,435 cm³ (15,84 mmol) de 2,4-diméthoxy-benzylamine en solution dans 65 cm³ de toluène, on ajoute à une température voisine de 20°C, sous atmosphère d'argon, 10,3 cm³ (20,59 mmol) d'une solution de triméthylaluminium 2M dans le toluène puis 1,95 g (7,92 mmol) de 3-(4-nitro-phényl)-1H-pyrrole-2-carboxylate de méthyle. Après 1 heure d'agitation à une température voisine de 20°C puis 16 heures d'agitation à une température voisine de 60°C, le mélange réactionnel est dilué avec 170 cm³ d'acétate d'éthyle. La solution résultante est lavée successivement avec 3 fois 70 cm³ d'une solution aqueuse saturée en chlorure d'ammonium et 2 fois 50 cm³ d'eau puis séchée sur sulfate de magnésium anhydre, filtrée et concentrée à sec sous pression réduite (2,7 kPa) pour donner un solide jaune qui est ensuite agité dans 20 cm³ d'acétonitrile à une température voisine de 5°C pendant 30 minutes. Après filtration sur verre fritté N°3, le solide est lavé avec 10 cm³ d'acétonitrile glacé puis séché sous pression réduite (2,7 kPa) à une température voisine de 35°C pendant 3 heures pour donner 1,96 g de 2-(2,4-diméthoxy-benzylamino)carbonyl-3-(4-nitro-phényl)-1H-pyrrole, sous forme d'un solide jaune ; ES⁺: m/z=382: [M+H]⁺ ; Eus^{-:} m/z=380: [M-H]⁻.

Le 3-(4-nitro-phényl)-1H-pyrrole-2-carboxylate de méthyle peut être préparé comme décrit dans l'exemple 1.

### Exemple 45

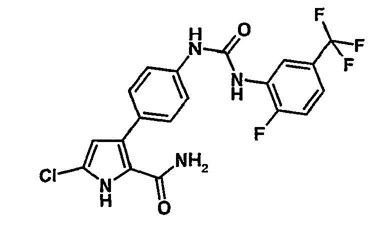

5-Chloro-3-{4-[3-2-fluoro-5-trifluorométhyl-phényl)-uréido]-phényl}-1H-pyrrole-2-carboxamide

A 0,055 g (0,233 mmol) de 5-chloro-3-(4-amino-phényl)-1H-pyrrole-2-carboxamide en solution dans 5 cm³ de tétrahydrofurane, on ajoute à une température voisine de 20°C, sous atmosphère d'argon, 0,035 cm³ (0,245 mmol) de 2-fluoro-5-trifluorométhyl-phényl isocyanate. Après 2 heures d'agitation à une température voisine de 20°C, le mélange réactionnel est concentré à sec sous pression réduite (2,7 kPa) pour donner un résidu qui est recristallisé dans 4 cm³ d'acétonitrile. Après filtration sur verre fritté N°3, le solide est lavé avec 2 cm³ d'acétonitrile puis séché sous pression réduite (2,7 kPa), à une température voisine de 35°C pendant 4 heures pour donner 0,076 g de 5-chloro-3-{4-[3-(2-fluoro-5-trifluorométhyl-phényl)-uréido]-phényl}-1H-pyrrole-2-carboxamide, sous forme d'un solide jaune; R.M.N. 1H (400 MHz, DMSO d6, δ en ppm) : 6,16 (s, 1H) ; 6,45 (m étalé, 1H) ; 7,12 (m étalé, 1H) ; 7,38 (m partiellement masqué, 1H) ; 7,40 (d, J = 9,0 Hz, 2H) ; 7,45 (d, J = 9,0 Hz, 2H) ; 7,49 (dd, J = 9,0 et 11,0 Hz, 1H) ; 8,63 (dd, J = 2,0 et 7,5 Hz, 1H) ; 8,90 (d, J = 2,5 Hz, 1H) ; 9,22 (s, 1H); 12,1 (m étalé, 1H); ES⁺: m/z=291: [M+Na]⁺ .

Le 5-chloro-3-(4-amino-phényl)-1H-pyrrole-2-carboxamide peut être préparé de la manière suivante :
A 0,12 g (0,452 mmol) de 5-chloro-3-(4-nitro-phényl)-1H-pyrrole-2-carboxamide en solution dans 2,36 cm³ d'acide acétique, on ajoute à une température voisine de 20°C, sous atmosphère d'argon, 118 mg (1,806 mmol) de zinc en poudre. Après 2 heures d'agitation à une température voisine de 20°C, le mélange réactionnel est dilué avec 30 cm³ d'acétate d'éthyle et 15 cm³ d'eau puis le pH est ajusté à une valeur comprise entre 8 et 9 par addition d'une solution aqueuse d'hydroxyde de sodium 1N. La phase aqueuse est extraite avec 2 fois 20 cm³ d'acétate d'éthyle. Les phases organiques sont réunies, séchées sur sulfate de magnésium anhydre, filtrées et concentrés à sec sous pression réduite (2,7 kPa) pour donner 0,103 g d'un solide orange qui est purifié par chromatographie-flash [éluant : dichlorométhane / méthanol / acétonitrile (90 / 5 / 5 en volumes)]. Après concentration des fractions sous pression réduite, on obtient 0,055 g de 5-chloro-3-(4-amino-phényl)-1H-pyrrole-2-carboxamide, sous forme d'un solide jaune; El: m/z=235: M^{+.} ; m/z=218 [M-NH2]^{+.}; m/z=155 [M-CI-CONH₃]⁺; m/z=44 [CONH₂]⁺ pic de base.

Le 3-(4-nitro-phényl)-1H-pyrrole-2-carboxylate de méthyle peut être préparé comme décrit dans l'exemple 44.

### Exemple 46

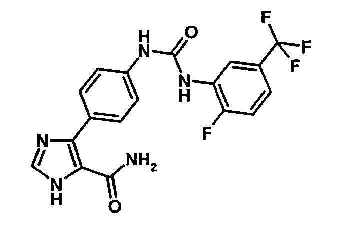

5-{4-[3-(2-Fluoro-5-trifluorométhyl-phényl)-uréido]-phényl}-3H-imidazole-4-carboxamide

A 0,055 g (0,272 mmol) de 5-(4-amino-phényl)-3H-imidazole-4-carboxamide en solution dans 4 cm³ de tétrahydrofurane, on ajoute à une température voisine de 20°C, sous atmosphère d'argon, 0,040 cm³ (0,272 mmol) de 2-fluoro-5-trifluorométhyl-phényl isocyanate. Après 16 heures d'agitation à une température voisine de 20°C, le mélange réactionnel est filtré sur verre fritté N°3. Le solide obtenu est lavé successivement avec 3 fois 2 cm³ de tétrahydrofurane et 3 fois 2 cm³ de pentane puis est séché sous pression réduite (2,7 kPa) pour donner 0,058 g de 5-{4-[3-(2-fluoro-5-trifluorométhyl-phényl)-uréido]-phényl}-3H-imidazole-4-carboxamide, sous forme d'un solide beige fondant à 325°C; R.M.N. 1H (400 MHz, DMSO d6, δ en ppm) 6,98 (m étalé, 1H) ; 7,30 (m étalé, 1H) ; 7,40 (m, 1H) ; 7,49 (m partiellement masqué, 1H) ; 7,51 (d, J = 8,5 Hz, 2H) ; 7,71 (s, 1H) ; 7,82 (d, J = 8,5 Hz, 2H) ; 8,62 (dd, J = 2,0 et 7,5 Hz, 1H) ; 9,00 (s large, 1H) ; 9,37 (s large, 1H) ; 12,65 (m étalé, 1H); El: m/z=407: M^{+.}; m/z=205 [C₈H₃NOF₄]^{+.} pic de base; m/z=179 [C₇H₅NF₄]⁺.

Le 5-(4-amino-phényl)-3H-imidazole-4-carboxamide peut être préparé de la manière suivante :
A une suspension de 0,009 g (0,081 mmol) de palladium sur charbon à 10% dans 20 cm³ de méthanol, on ajoute à une température voisine de 20°C, 0,04 g (0,172 mmol) de 5-(4-nitro-phényl)-3H-imidazole-4-carboxamide. Après 3 heures d'hydrogénation en autoclave sous 3 bar d'hydrogène, à une température voisine de 25°C, le mélange réactionnel est filtré. Le catalyseur est rincé avec 2 fois 2 cm³ de méthanol puis le filtrat est concentré à sec sous pression réduite (2,7 kPa), pour donner 0,034 g de 5-(4-amino-phényl)-3H-imidazole-4-carboxamide, sous forme d'un solide marron; El: m/z=202: M^{+.}; CI: m/z=203: [M+H]⁺ ; ES⁺: m/z=203: [M+H]⁺; ES^{-:} m/z=201: [M-H]⁻.

Le 5-(4-nitro-phényl)-3H-imidazole-4-carboxamide peut être préparé de la manière suivante :
Une solution de 0,05 g (0,192 mmol) de 5-(4-nitro-phényl)-3H-imidazole-4-carboxylate d'éthyle dans 4 cm³ d'une solution d'ammoniaque 7 N dans le méthanol et 8 cm³ d'une solution aqueuse d'ammoniaque à 28% est chauffée en autoclave à une température voisine de 80°C, sous 12 bar, pendant 20 heures. Après arrêt du chauffage puis retour à température et pression ambiante, le mélange réactionnel est concentré à sec, sous pression réduite (2,7 kPa), pour donner 0,068 g d'un solide qui est purifié par chromatographie-flash [éluant : acétate d'éthyle / méthanol (95 / 5 en volumes)]. Après concentration des fractions sous pression réduite, on obtient 0,012 g de 5-(4-nitro-phényl)-3H-imidazole-4-carboxamide, sous forme d'un solide blanc; El: m/z=232: M^{+.}; m/z=231 [M-H]⁻ pic de base ; Cl: m/z=233: [M+H]⁺; m/z=250 [M+NH₄]⁺ pic de base.

Le 5-(4-nitro-phényl)-3H-imidazole-4-carboxylate d'éthyle peut être préparé de la manière suivante :
A 4,572g (59,32 mmol) d'acétate d'ammonium en solution dans 60 cm³ d'acide acétique, on ajoute à une température voisine de 20°C, sous atmosphère d'argon, 1,493 g (5,93 mmol) de 3-(4-nitro-phényl)-2,3-dioxo-propioniate d'éthyle et 32,06 g (356 mmol) de formaldéhyde. Après 4 heures d'agitation à une température voisine de 65°C, le mélange réactionnel est filtré sur verre fritté N°3. Le solide obtenu est lavé avec 50 cm³ de méthanol puis le filtrat est concentré à sec sous pression réduite (2,7 kPa) pour donner un résidu qui est repris avec 30 cm³ de dichlorométhane. La solution résultante est lavée successivement avec 30 cm³ d'une solution aqueuse saturée en carbonate de sodium, 30 cm³ d'eau et 30 cm³ d'une solution aqueuse saturée en chlorure de sodium puis est séchée sur sulfate de magnésium anhydre, filtrée et concentrée à sec sous pression réduite (2,7 kPa), pour donner un solide jaune qui est purifié par chromatographie-flash [éluant : acétate d'éthyle / cyclohexane (4 / 1 en volumes)]. Après concentration des fractions sous pression réduite, on obtient 0,197 g de 5-(4-nitro-phényl)-3H-imidazole-4-carboxylate d'éthyle, sous forme d'un solide jaune; El: m/z=261: M^{+.} pic de base; m/z=215 [M-HOEt]⁺.

Le 3-(4-nitro-phényl)-2,3-dioxo-propioniate d'éthyle peut être préparé comme décrit dans la littérature par Dayer, Francis; Huu Le Dao; Gold, Hellmut; Rode-Gowal, Heike; Dahn, Hans; Helvetica Chimica Acta (1974), 57(7), 2201-9,

### Détermination de l'activité des composés - Protocoles expérimentaux

### 1. FAK

L'activité inhibitrice des composés sur FAK est déterminée par une mesure de l'inhibition de l'autophosphorylation de l'enzyme en utilisant un test de fluorescence résolue dans le temps (HTRF).

L'ADNc complet de FAK humain, dont l'extrémité N-terminale a été marquée à l'histidine, a été cloné dans un vecteur d'expression baculovirus pFastBac HTc. La protéine a été exprimée et purifiée à environ 70% d'homogénéité.

L'activité kinase est déterminée en incubant l'enzyme (6.6 µg/ml) avec différentes concentrations de composé à tester dans un tampon 50 mM Hepes pH = 7,2, 10 mM MgCl₂, 100 µM Na₃VO₄ ,15 µM d'ATP pendant 1 heure à 37°C. La réaction enzymatique est stoppée par l'addition de tampon Hepes pH = 7,0 contenant 0.4 mM KF, 133 mM EDTA, 0.1 % BSA et le marquage est effectuée, pendant 1 à 2 heures à température ambiante, par l'addition dans ce tampon d'un anticorps anti-Histidine marqué avec XL665 et d'un anticorps monoclonal phosphospécifique de la tyrosine conjugué à du cryptate d'europium (Eu-K). Les caractéristiques des deux fluorophores sont disponibles dans G. Mathis et al., Anticancer Research, 1997, 17, pages 3011-3014. Le transfert d'énergie entre le cryptate d'europium excité vers le XL665 accepteur est proportionnel au degré d'autophosphorylation de FAK. Le signal de longue durée spécifique de XL-665 est mesuré dans un compteur de plaques Packard Discovery. Tous les essais sont effectués en double exemplaire et la moyenne des deux essais est calculée. L'inhibition de l'activité d'autophosphorylation de FAK avec des composés de l'invention est exprimée en pourcentage d'inhibition par rapport à un contrôle dont l'activité est mesurée en l'absence de composé test. Pour le calcul du % d'inhibition, le ratio [signal à 665 nm/signal à 620 nm] est considéré.

### 2. KDR

L'effet inhibiteur des composés est déterminé dans un test de phosphorylation de substrat par l'enzyme KDR *in vitro* par une technique de scintillation (plaque 96 puits, NEN).

Le domaine cytoplasmique de l'enzyme KDR humaine a été cloné sous forme de fusion GST dans le vecteur d'expression baculovirus pFastBac. La protéine a été exprimée dans les cellules SF21 et purifiée à environ 60 % d'homogénéité.

L'activité kinase de KDR est mesurée dans 20 mM MOPS, 10 mM MgCl2, 10 mM MnCl2, 1 mM DTT, 2.5 mM EGTA, 10 mM b-glycérophosphate, pH = 7.2, en présence de 10 mM MgCl₂, 100 µM Na₃VO₄, 1 mM NaF. 10 µl du composé sont ajoutés à 70 µl de tampon kinase contenant 100 ng d'enzyme KDR à 4°C. La réaction est lancée en ajoutant 20 µl de solution contenant 2 µg de substrat (fragment SH2-SH3 de la PLC□ exprimée sous forme de protéine de fusion GST), 2 µCi □ ³³P[ATP] et 2 µM ATP froid. Après 1 heure d'incubation à 37°C, la réaction est stoppée en ajoutant 1 volume (100 µl) de 200 mM EDTA. Le tampon d'incubation est retiré, et les puits sont lavés trois fois avec 300 µl de PBS. La radioactivité est mesurée dans chaque puits en utilisant un compteur de radioactivité Top Count NXT (Packard).

Le bruit de fond est déterminé par la mesure de la radioactivité dans quatre puits différents contenant l'ATP radioactif et le substrat seul.

Un contrôle d'activité totale est mesuré dans quatre puits différents contenant tous les réactifs (γ³³P-[ATP], KDR et substrat PLCγ) mais en l'absence de composé.

L'inhibition de l'activité KDR avec le composé de l'invention est exprimée en pourcentage d'inhibition de l'activité contrôle déterminée en l'absence de composé.

Le composé SU5614 (Calbiochem) (1 µM) est inclus dans chaque plaque comme contrôle d'inhibition.

### 3. Tie2

La séquence codante de Tie2 humain correspondant aux acides aminés du domaine intracellulaire 776-1124 a été générée par PCR en utilisant le cDNA isolé de placenta humain comme modèle. Cette séquence a été introduite dans un vecteur d'expression *baculovirus* pFastBacGT sous forme de protéine de fusion GST.

L'effet inhibiteur des molécules est déterminé dans un test de phosphorylation de PLC par Tie2 en présence de GST-Tie2 purifiée à environ 80% d'homogénéité. Le substrat est composé des fragments SH2-SH3 de la PLC exprimée sous forme de protéine de fusion GST.

L'activité kinase de Tie2 est mesurée dans un tampon MOPS 20mM pH 7.2, contenant 10 mM MgCl₂, 10 mM MnCl₂, 1 mM DTT, 10 mM de glycérophosphate. Dans une plaque 96 puits FlashPlate maintenue sur glace, on dépose un mélange réactionnel composé de 70 µl de tampon kinase contenant 100 ng d'enzyme GST-Tie2 par puits. Ensuite 10 µl de la molécule à tester diluée dans du DMSO à une concentration de 10 % maximum sont ajoutés. Pour une concentration donnée, chaque mesure est effectuée en quatre exemplaires. La réaction est initiée en ajoutant 20 µl de solution contenant 2 µg de GST-PLC, 2 µM d'ATP froid et 1 µCi d'³³P[ATP]. Après 1 heure d'incubation à 37°C, la réaction est stoppée en ajoutant 1 volume (100µl) d'EDTA à 200 mM. Après élimination du tampon d'incubation, les puits sont lavés trois fois avec 300 µl de PBS. La radioactivité est mesurée sur un MicroBeta1450 Wallac.

L'inhibition de l'activité Tie2 est calculée et exprimée en pourcentage d'inhibition par rapport à l'activité contrôle déterminée en l'absence de composé.

**Résultats : Tableau 1 :**

| | | FAK | KDR | TIE2 |
|---|---|---|---|---|
| Structure | exemple | IC 50 (nM) | IC 50 (nM) | IC 50 (nM) |
| 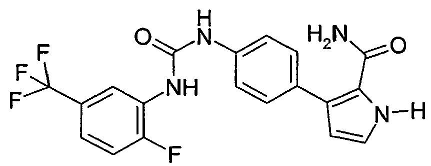 | **1** | 2381 | 115 | 13 |
| 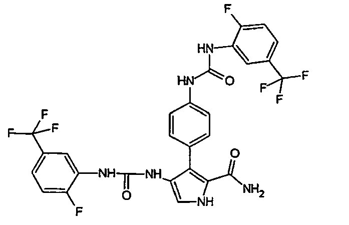 | **4** | - | 352 | 111 |
| 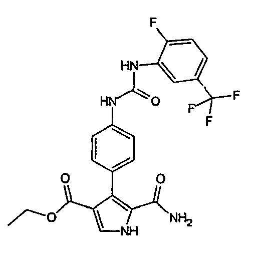 | **9** | - | 23 | 4 |
| 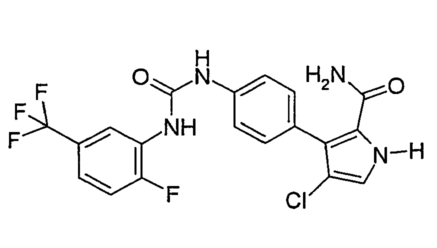 | **44** | - | 27 | 7 |

## Revendications

1. Produit répondant à la formule (I) suivante : 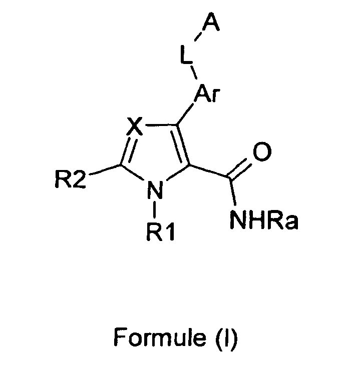 dans laquelle :
1) Ar-L-A est: 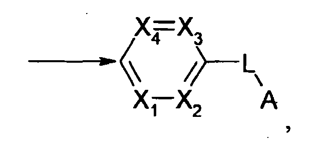 dans lequel chaque X1, X2, X3 et X4 est N ou CH ;
2) L est NH-CO-NH;
3) A est phényle, pyrazole ou isoxazolyl éventuellement substitué ;
4) Ra est H ;
5) R1 est H ;
6) X est choisi parmi CR3 et N ;
7) R2 est un atome d'hydrogène
8) R3 est sélectionné dans le groupe constitué par : H, halogène, R'2, CN, O(R'2), OC(O)(R'2), OC(O)N(R'2)(R'3), OS(O₂)(R'2), N(R'2)(R'3), N=C(R'2)(R'3), N(R'2)C(O)(R'3), N(R'2)C(O)O(R'3), N(R'4)C(O)N(R'2)(R'3), N(R'4)C(S)N(R'2)(R'3), N(R'2)S(O₂)(R'3) C(O)(R'2), C(O)O(R'2), C(O)N(R'2)(R'3), C(=N(R'3))(R'2), C(=N(OR'3))(R'2), S(R'2), S(O)(R'2), S(O₂)(R'2), S(O₂)O(R'2), S(O₂)N(R'2)(R'3) ; dans lequel chaque R'2, R'3, R'4 est indépendamment sélectionné dans le groupe constitué par H, alkyle, alkylène, alkynyle, aryle, hétéroaryle, cycloalkyle, hétérocyclyle, alkyle substitué, alkylène substitué, alkynyle substitué, aryle substitué, hétéroaryle substitué, cycloalkyle substitué, hétérocyclyle substitué; dans lequel, lorsque R'2 et R'3 sont chacun différents de H et simultanément présents sur R2 ou sur R3, ils peuvent être liés entre eux pour former un cycle contenant de 0 à 3 hétéroatomes choisis parmi O, S et N et **caractérisé en ce qu'**au moins l'un des R2 ou R3 est un halogène.

2. Produit selon la revendication 1, **caractérisé en ce que** X est CR3.

3. Produit selon l'une quelconque des revendications 1 à 2, **caractérisé en ce que** R3 est sélectionné dans le groupe constitué par H, halogène, NH₂, N(R'2)C(O)(R'3), N(R'2)C(O)O(R'3), N(R'4)C(O)N(R'2)(R'3), C(O)O(R'2), C(O)N(R'2)(R'3); dans lequel chaque R'2, R'3 est indépendamment sélectionné dans le groupe constitué par H, alkyle, alkylène, alkynyle, aryle, hétéroaryle, cycloalkyle, hétérocyclyle, alkyle substitué, alkylène substitué, alkynyle substitué, aryle substitué, hétéroaryle substitué, cycloalkyle substitué, hétérocyclyle substitué; dans lequel, lorsque R'2 et R'3 sont chacun différents de H et simultanément présents sur R2 ou sur R3, ils peuvent être liés entre eux pour former un cycle contenant de 0 à 3 hétéroatomes choisis parmi O, S et N.

4. Produit selon la revendication 3, **caractérisé en ce que** R3 est sélectionné dans le groupe constitué par H, halogène, NH₂, NH-COO-alkyle, - NH-CO-NH-aryle où l'aryle est non substitué ou substitué par un ou plusieurs halogène ou alkyle halogéné, -NH-CO-alkyle, -NH-CO-alkyleN(alkyle)(alkyle'), -COOH, -COO-alkyle, -COO-alkyle-N(alkyle)(alkyle'), -CO-NH-alkyle-aryle où l'aryle est non substitué ou substitué par un ou plusieurs alcoxy, -CONH₂, -CO-hétérocyclyle où l'hétérocyclycle est non substitué ou substitué par un ou plusieurs alkyles.

5. Produit selon la revendication 1, **caractérisé en ce que** A est substitué par un premier substituant sélectionné dans le groupe constitué par alkyle, alkyle halogéné, alkylène, alkynyle, aryle, hétéroaryle, O-alkyle, O-Aryle, O-hétéroaryle, S-alkyle, S-alkyle substitué, S-Aryle, S-hétéroaryle, chacun étant éventuellement substitué par un substituant choisi parmi (C1-C3)alkyle, halogène, O-(C1-C3)alkyle.

6. Produit selon la revendication 5, **caractérisé en ce que** A est substitué par un deuxième substituant sélectionné dans le groupe constitué par F, CI, Br, I, OH, SH, SO₃M, COOM, CN, NO₂, CON(R8)(R9), N(R8)CO(R9), (C1-C3)alkyle-OH, (C1-C3)alkyle-N(R8)(R9), (C1-C3)alkyle-(R10), (C1-C3)alkyle-COOH, N(R8)(R9) ; dans lequel R8 et R9 sont indépendamment choisis parmi H, (C1-C3)alkyle, (C1-C3)alkyle halogéné, (C1-C3)alkyleOH, (C1-C3)alkyleNH₂, (C1-C3)alkyleCOOM, (C1-C3)alkyleSO₃M ; dans lequel lorsque R8 et R9 sont simultanément différents de H, ils peuvent être liés pour former un cycle de 5 à 7 chaînons contenant de 0 à 3 hétéroatomes choisis parmi N, O et S; dans lequel M est H ou un cation de métal alcalin choisi parmi Li, Na et K ; et dans lequel R10 est H ou un hétérocycle non aromatique éventuellement substitué comprenant de 2 à 7 atomes de carbone, et de 1 à 3 hétéroatomes choisis parmi N, O et S.

7. Produit selon l'une quelconque des revendications 5 à 6, **caractérisé en ce que** A est phényle substitué par halogène, (C1-C4)alkyle, (C1-C3)alkyle halogéné, O-(C1-C4)alkyle, S-(C1-C4)alkyle, O-(C1-C4)alkyle halogéné, et S-(C1-C4)alkyle halogéné, et lorsque A est disubstitué, les deux substituants peuvent être liés entre eux pour former un cycle de 5 à 7 chaînons contenant de 0 à 3 hétéroatomes choisis parmi O, N et S.

8. Produit selon l'une quelconque des revendications 5 à 7, **caractérisé en ce que** A est substitué par un ou plusieurs substituants, identiques ou différents, indépendamment sélectionnés dans le groupe constitué par F, CI, Br, I, OH, SH, SO₃M, COOM, CN, NO₂, CON(R8)(R9), N(R8)CO(R9), (C1-C3)alkyle-OH, (C1-C3)alkyle-N(R8)(R9), (C1-C3)alkyle-(R10), (C1-C3)alkyle-COOH, N(R8)(R9), (C1-C6)alkyle, (C2-C6)alkylène, (C2-C6)alkynyle, aryle, hétéroaryle, O-(C1-C6)alkyle, O-Aryle, O-hétéroaryle, S-(C1-C6)alkyle, S-Aryle, S-hétéroaryle, chacun étant éventuellement substitué par un ou plusieurs substituants choisis parmi (C1-C3)alkyle, halogène, O-(C1-C3)alkyle; dans lequel R8 et R9 sont indépendamment choisis parmi H, (C1-C3)alkyle, (C1-C3)alkyleOH, (C1-C3)alkyleNH₂, (C1-C3)alkyleCOOM, (C1-C3)alkyleSO₃M ; dans lequel lorsque R8 et R9 sont simultanément différents de H, ils peuvent être liés pour former un cycle de 5 à 7 chaînons comportant de 0 à 3 hétéroatomes choisis parmi O, N et S; dans lequel M est H ou un cation de métal alcalin choisi parmi Li, Na et K ; et dans lequel R10 est H ou un hétérocycle non aromatique éventuellement substitué, comprenant 2 à 7 atomes de carbone, et 1 à 3 hétéroatomes choisis parmi N, O et S.

9. Produit selon la revendication 1, **caractérisé en ce qu'**il est choisi parmi :
3-{4-[3-(2-Fluoro-5-trifluorométhyl-phényl)-uréido]-phényl}-1H-pyrrole-2-carboxamide,
4-*tert*-Butyloxycarbonylamino-3-{4-[3-(2-fluoro-5-trifluorométhyl-phényl)-uréido]-phényl}-1H-pyrrole-2-carboxamide,
Chlorhydrate du 4-amino-3-{4-[3-(2-fluoro-5-trifluorométhyl-phényl)-uréido]-phényl}-1H-pyrrole-2-carboxamide,
4-[3-(2-Fluoro-5-trifluorométhyl-phényl)-uréido]-3-{4-[3-(2-fluoro-5-trifluorométhyl-phényl)-uréido]-phényl}-1H-pyrrole-2-carboxam ide,
4-Acétylamino-3-{4-[3-(2-fluoro-5-trifluorométhyl-phényl)-uréido]-phényl}-1H-pyrrole-2-carboxamide,
4-(2-Diméthylamino-acétylamino)-3-{4-[3-(2-fluoro-5-trifluorométhyl-phényl)-uréido]-phényl}-1H-pyrrole-2-carboxamide,
4-Benzoylamino-3-{4-[3-(2-fluoro-5-trifluorométhyl-phényl)-uréido]-phényl}-1H-pyrrole-2-carboxamide,
4-Nicotinoylamino-3-{4-[3-(2-fluoro-5-trifluorométhyl-phényl)-uréido]-phényl}-1H-pyrrole-2-carboxamide,
5-Carbamoyl-4-{4-[3-(2-fluoro-5-trifluorométhyl-phényl)-uréido]-phényl}-1H-pyrrole-3-carboxylate d'éthyle,
5-Carbamoyl-4-{4-[3-(2-fluoro-phényl)-uréido]-phényl}-1H-pyrrole-3-carboxylate d'éthyle,
5-Carbamoyl-4-{4-[3-(2-méthoxy-phényl)-uréido]-phényl}-1H-pyrrole-3-carboxylate d'éthyle,
5-Carbamoyl-4-{4-[3-(2-trifluorométhyl-phényl)-uréido]-phényl}-1H-pyrrole-3-carboxylate d'éthyle,
5-Carbamoyl-4-[4-(3-o-tolyl-uréido)-phényl]-1H-pyrrole-3-carboxylate d'éthyle,
5-Carbamoyl-4-{4-[3-(3-fluoro-phényl)-uréido]-phényl}-1H-pyrrole-3-carboxylate d'éthyle,
5-Carbamoyl-4-{4-[3-(3-méthoxy-phényl)-uréido]-phényl}-1H-pyrrole-3-carboxylate d'éthyle,
5-Carbamoyl-4-{4-[3-(3-trifluorométhyl-phényl)-uréido]-phényl}-1H-pyrrole-3-carboxylate d'éthyle,
5-Carbamoyl-4-[4-(3-m-tolyl-uréido)-phényl]-1H-pyrrole-3-carboxylate d'éthyle,
5-Carbamoyl-4-{4-[3-(4-fluoro-phényl)-uréido]-phényl}-1H-pyrrole-3-carboxylate d'éthyle,
5-Carbamoyl-4-{4-[3-(4-méthoxy-phényl)-uréido]-phényl}-1H-pyrrole-3-carboxylate d'éthyle,
5-Carbamoyl-4-{4-[3-(4-trifluorométhyl-phényl)-uréido]-phényl}-1H-pyrrole-3-carboxylate d'éthyle,
5-Carbamoyl-4-[4-(3-p-tolyl-uréido)-phényl]-1H-pyrrole-3-carboxylate d'éthyle,
5-Carbamoyl-4-{4-[3-(4-chloro-3-trifluorométhyl-phényl)-uréido]-phényl}-1H-pyrrole-3-carboxylate d'éthyle,
5-Carbamoyl-4-{4-[3-(2-chloro-5-trifluorométhyl-phényl)-uréido]-phényl}-1H-pyrrole-3-carboxylate d'éthyle,
5-Carbamoyl-4-{4-[3-(2-fluoro-3-trifluorométhyl-phényl)-uréido]-phényl}-1H-pyrrole-3-carboxylate d'éthyle,
5-Carbamoyl-4-{4-[3-(4-fluoro-3-trifluorométhyl-phényl)-uréido]-phényl}-1H-pyrrole-3-carboxylate d'éthyle,
5-Carbamoyl-4-{4-[3-(3-fluoro-5-trifluorométhyl-phényl)-uréido]-phényl)-1H-pyrrole-3-carboxylate d'éthyle,
5-Carbamoyl-4-{4-[3-(4-méthyl-3-trifluorométhyl-phényl)-uréido]-phényl}-1H-pyrrole-3-carboxylate d'éthyle,
5-Carbamoyl-4-{4-[3-(4-trifluorométhoxy-phényl)-uréido]-phényl}-1H-pyrrole-3-carboxylate d'éthyle,
5-Carbamoyl-4-{4-[3-(4-difluorométhoxy-phényl)-uréido]-phényl}-1H-pyrrole-3-carboxylate d'éthyle,
5-Carbamoyl-4-{4-[3-(3,4-diméthyl-phényl)-uréido]-phényl}-1H-pyrrole-3-carboxylate d'éthyle,
5-Carbamoyl-4-{4-[3-(3,4-diméthoxy-phényl)-uréido]-phényl}-1H-pyrrole-3-carboxylate d'éthyle,
5-Carbamoyl-4-{4-[3-(3,5-diméthoxy-phényl)-uréido]-phényl}-1H-pyrrole-3-carboxylate d'éthyle,
5-Carbamoyl-4-{4-[3-(2,5-diméthyl-phényl)-uréido]-phényl}-1H-pyrrole-3-carboxylate d'éthyle,
5-Carbamoyl-4-{4-[3-(2-méthoxy-5-méthyl-phényl)-uréido]-phényl}-1H-pyrrole-3-carboxylate d'éthyle,
5-Carbamoyl-4-{4-[3-(2,5-diméthoxy-phényl)-uréido]-phényl}-1H-pyrrole-3-carboxylate d'éthyle,
5-Carbamoyl-4-{4-[3-(3-chloro-4-difluorométhoxy-phényl)-uréido]-phényl}-1H-pyrrole-3-carboxylate d'éthyle,
5-Carbamoyl-4-{4-[3-(3,5-diméthyl-phényl)-uréido]-phényl}-1H-pyrrole-3-carboxylate d'éthyle,
Acide 5-carbamoyl-4-{4-[3-(2-fluoro-5-trifluorométhyl-phényl)-uréido]-phényl}-1H-pyrrole-3-carboxylique,
4-(2,4-Diméthoxy-benzylamino)carbonyl-3-{4-[3-(2-fluoro-5-trifluorométhyl-phényl)-uréido]-phényl}-1H-pyrrole-2-carboxamide,
3-{4-[3-(2-Fluoro-5-trifluorométhyl-phényl)-uréido]-phényl}-1H-pyrrole-2,4-di-carboxamide,
3-{4-[3-(2-Fluoro-5-trifluorométhyl-phényl)-uréido]-phényl}-4-(4-méthylpipérazine-1-carbonyl)-1H-pyrrole-2-carboxamide,
3-{4-[3-(2-Fluoro-5-trifluorométhyl-phényl)-uréido]-phényl}-4-(pyrrolidine-1-carbonyl)-1H-pyrrole-2-carboxamide,
5-Carbamoyl-4-{4-[3-(2-fluoro-5-trifluorométhyl-phényl)-uréido]-phényl}-1H-pyrrole-3-carboxylate de 2-diméthylamino-éthyle,
4-Chloro-3-{4-[3-(2-fluoro-5-trifluorométhyl-phényl)-uréido]-phényl}-1H-pyrrole-2-carboxamide,
5-Chloro-3-{4-[3-(2-fluoro-5-trifluorométhyl-phényl)-uréido]-phényl}-1H-pyrrole-2-carboxamide,
5-{4-[3-(2-Fluoro-5-trifluorométhyl-phényl)-uréido]-phényl}-3H-imidazole-4-carboxamide.

10. Produit selon l'une quelconque des revendications précédentes, **caractérisé en ce qu'**il est sous forme :
1) non chirale, ou
2) racémique, ou
3) enrichie en un stéréo-isomère, ou
4) enrichie en un énantiomère ;
et **en ce qu'**il est éventuellement salifié.

11. Médicament, **caractérisé en ce qu'**il comprend un produit de formule (I) selon l'une quelconque des revendication 1 à 9, ou un sel d'addition de ce composé à un acide pharmaceutiquement acceptable, ou encore un hydrate ou un solvate du produit de formule (I).

12. Composition pharmaceutique comprenant un produit selon l'une quelconque des revendications précédentes, en combinaison avec un excipient pharmaceutiquement acceptable.

13. Utilisation d'un produit selon l'une quelconque des revendications 1 à 9, pour la fabrication d'un médicament utile pour traiter un état pathologique.

14. Utilisation selon la revendication 13, **caractérisée en ce que** l'état pathologique est le cancer.

## Claims

1. Product corresponding to formula (I) below: 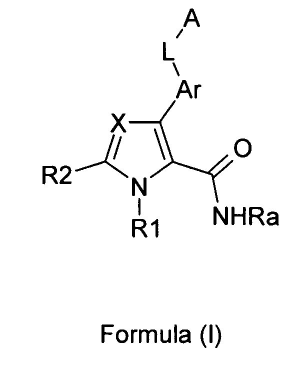 in which:
1) Ar-L-A is: 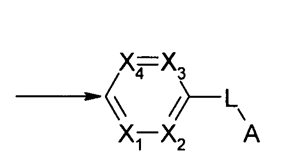 in which X1, X2, X3 and X4 is N or CH;
2) L is NH-CO-NH;
3) A is optionally substituted phenyl, pyrazolyl or isoxazolyl;
4) Ra is H;
5) R1 is H;
6) X is chosen from CR3 and N;
7) R2 is a hydrogen atom;
8) R3 is selected from the group consisting of: H, halogen, R'2, CN, O(R'2), OC(O)(R'2), OC(O)N(R'2)(R'3), OS(O₂)(R'2), N(R'2)(R'3), N=C(R'2)(R'3), N(R'2)C(O)(R'3), N(R'2)C(O)O(R'3), N(R'4)C(O)N(R'2)(R'3), N(R'4)C(S)N(R'2)(R'3), N(R'2)S(O₂)(R'3) C(O)(R'2), C(O)O(R'2), C(O)N(R'2)(R'3), C(= N(R'3))(R'2), C(=N(OR'3))(R'2), S(R'2), S(O)(R'2), S(O₂)(R'2) S(O₂)O(R'2), S(O₂)N(R'2)(R'3); in which each R'2, R'3, R'4 is independently selected from the group consisting of H, alkyl, alkylene, alkynyl, aryl, heteroaryl, cycloalkyl, heterocyclyl, substituted alkyl, substituted alkylene, substituted alkynyl, substituted aryl, substituted heteroaryl, substituted cycloalkyl and substituted heterocyclyl; in which, when R'2 and R'3 are each other than H and simultaneously present on R2 or on R3, they may be linked together to form a ring containing from 0 to 3 hetero atoms chosen from O, S and N and **characterized in that** at least one from among R2 and R3 is a halogen.

2. Product according to Claim 1, **characterized in that** X is CR3.

3. Product according to either of Claims 1 and 2, **characterized in that** R3 is selected from the group consisting of H, halogen, NH₂, N(R'2)C(O)(R'3), N(R'2)C(O)O(R'3), N(R'4)C(O)N(R'2)(R'3), C(O)O(R'2) and C(O)N(R'2)(R'3); in which each R'2, R'3 is independently selected from the group consisting of H, alkyl, alkylene, alkynyl, aryl, heteroaryl, cycloalkyl, heterocyclyl, substituted alkyl, substituted alkylene, substituted alkynyl, substituted aryl, substituted heteroaryl, substituted cycloalkyl and substituted heterocyclyl; in which, when R'2 and R'3 are each other than H and simultaneously present on R2 or on R3, they may be linked together to form a ring containing from 0 to 3 hetero atoms chosen from O, S and N.

4. Product according to Claim 3, **characterized in that** R3 is selected from the group consisting of H, halogen, NH₂, NH-COO-alkyl and -NH-CO-NH-aryl in which the aryl is unsubstituted or substituted with one or more halogen or haloalkyl, -NH-CO-alkyl, -NH-CO-alkyl-N(alkyl)(alkyl'), -COOH, -COO-alkyl, -COO-alkyl-N(alkyl)(alkyl') or -CO-NH-alkyl-aryl in which the aryl is unsubstituted or substituted with one or more alkoxy, -CONH₂ or -CO-heterocyclyl in which the heterocyclyl is unsubstituted or substituted with one or more alkyls.

5. Product according to Claim 1, **characterized in that** A is substituted with a first substituent selected from the group consisting of alkyl, haloalkyl, alkylene, alkynyl, aryl, heteroaryl, O-alkyl, O-Aryl, O-heteroaryl, S-alkyl, substituted S-alkyl, S-Aryl and S-heteroaryl, each being optionally substituted with a substituent chosen from (C1-C3)alkyl, halogen and O-(C1-C3)alkyl.

6. Product according to Claim 5, **characterized in that** A is substituted with a second substituent chosen from the group consisting of F, Cl, Br, I, OH, SH, SO₃M, COOM, CN, NO₂, CON(R8)(R9), N(R8)CO(R9), (C1-C3)alkyl-OH, (C1-C3)alkyl-N(R8)(R9), (C1-C3)alkyl-(R10), (C1-C3)alkyl-COOH and N(R8)(R9); in which R8 and R9 are independently chosen from H, (C1-C3)alkyl, (C1-C3)haloalkyl, (C1-C3)alkylOH, (C1-C3)alkylNH₂, (C1-C3)alkylCOOM and (C1-C3)alkylSO₃M; in which, when R8 and R9 are simultaneously other than H, they may be linked together to form a 5- to 7-membered ring containing from 0 to 3 hetero atoms chosen from N, O and S; in which M is H or a cation of an alkali metal chosen from Li, Na and K; and in which R10 is H or an optionally substituted non-aromatic heterocycle containing from 2 to 7 carbon atoms and 1 to 3 hetero atoms chosen from N, O and S.

7. Product according either of Claims 5 and 6, **characterized in that** A is phenyl substituted with halogen, (C1-C4)alkyl, (C1-C3)haloalkyl, O-(C1-C4)alkyl, S-(C1-C4)alkyl, O-(C1-C4)haloalkyl and S-(C1-C4)haloalkyl, and when A is disubstituted, the two substituents may be linked together to form a 5- to 7-membered ring containing from 0 to 3 hetero atoms chosen from O, N and S.

8. Product according to any one of Claims 5 to 7, **characterized in that** A is substituted with one or more substituents, which may be identical or different, independently selected from the group consisting of F, Cl, Br, I, OH, SH, SO₃M, COOM, CN, NO₂, CON(R8)(R9), N(R8)CO(R9), (C1-C3)alkyl-OH, (C1-C3)alkyl-N(R8)(R9), (C1-C3)alkyl-(R10), (C1-C3)alkyl-COOH, N(R8)(R9), (C1-C6)alkyl, (C2-C6)alkylene, (C2-C6)alkynyl, aryl, heteroaryl, O-(C1-C6)alkyl, O-Aryl, O-heteroaryl, S-(C1-C6)alkyl, S-Aryl and S-heteroaryl, each being optionally substituted with one or more substituents chosen from (C1-C3)alkyl, halogen and O-(C1-C3)alkyl; in which R8 and R9 are independently chosen from H, (C1-C3)alkyl, (C1-C3)alkylOH, (C1-C₃)alkylNH₂, (C1-C3)alkylCOOM and (C1-C3)alkylSO₃M; in which, when R8 and R9 are simultaneously other than H, they may be linked together to form a 5- to 7-membered ring containing from 0 to 3 hetero atoms chosen from O, N and S; in which M is H or a cation of an alkali metal chosen from Li, Na and K; and in which R10 is H or an optionally substituted non-aromatic heterocycle, containing 2 to 7 carbon atoms and 1 to 3 hetero atoms chosen from N, O and S.

9. Product according to Claim 1, **characterized in that** it is chosen from:
3-{4-[3-(2-Fluoro-5-trifluoromethylphenyl)ureido]phenyl}-1H-pyrrole-2-carboxamide,
4-*tert*-Butyloxycarbonylamino-3-{4-[3-(2-fluoro-5-trifluoromethylphenyl)ureido]-phenyl}-1H-pyrrole-2-carboxamide,
4-Amino-3-{4-[3-(2-fluoro-5-trifluoromethylphenyl)ureido]phenyl}-1H-pyrrole-2-carboxamide hydrochloride,
4-[3-(2-Fluoro-5-trifluoromethylphenyl)ureido]-3-{4-[3-(2-fluoro-5-trifluoromethylphenyl)ureido]phenyl}-1H-pyrrole-2-carboxamide,
4-Acetylamino-3-{4-[3-(2-fluoro-5-trifluoromethylphenyl)ureido]phenyl}-1H-pyrrole-2-carboxamide,
4-(2-Dimethylaminoacetylamino)-3-{4-[3-(2-fluoro-5-trifluoromethylphenyl)-ureido]phenyl}-1H-pyrrole-2-carboxamide,
4-Benzoylamino-3-{4-[3-(2-fluoro-5-trifluoromethylphenyl)ureido]phenyl}-1H-pyrrole-2-carboxamide,
4-Nicotinoylamino-3-{4-[3-(2-fluoro-5-trifluoromethylphenyl)ureido]phenyl}-1H-pyrrole-2-carboxamide,
Ethyl 5-carbamoyl-4-{4-[3-(2-fluoro-5-trifluoromethylphenyl)ureido]phenyl}-1H-pyrrole-3-carboxylate,
Ethyl 5-carbamoyl-4-{4-[3-(2-fluorophenyl)ureido]phenyl}-1H-pyrrole-3-carboxylate,
Ethyl 5-carbamoyl-4-{4-[3-(2-methoxyphenyl)ureido]phenyl}-1H-pyrrole-3-carboxylate,
Ethyl 5-carbamoyl-4-{4-[3-(2-trifluoromethylphenyl)ureido]phenyl}-1H-pyrrole-3-carboxylate,
Ethyl 5-carbamoyl-4-[4-(3-o-tolylureido)phenyl]-1H-pyrrole-3-carboxylate,
Ethyl 5-carbamoyl-4-{4-[3-(3-fluorophenyl)ureido]phenyl}-1H-pyrrole-3-carboxylate,
Ethyl 5-carbamoyl-4-{4-[3-(3-methoxyphenyl)ureido]phenyl}-1H-pyrrole-3-carboxylate,
Ethyl 5-carbamoyl-4-{4-[3-(3-trifluoromethylphenyl)ureido]phenyl}-1H-pyrrole-3-carboxylate,
Ethyl 5-carbamoyl-4-[4-(3-m-tolylureido)phenyl]-1H-pyrrole-3-carboxylate,
Ethyl 5-carbamoyl-4-{4-[3-(4-fluorophenyl)ureido]phenyl}-1H-pyrrole-3-carboxylate,
Ethyl 5-carbamoyl-4-{4-[3-(4-methoxyphenyl)ureido]phenyl}-1H-pyrrole-3-carboxylate,
Ethyl 5-carbamoyl-4-{4-[3-(4-trifluoromethylphenyl)ureido]phenyl}-1H-pyrrole-3-carboxylate,
Ethyl 5-carbamoyl-4-[4-(3-p-tolylureido)phenyl]-1H-pyrrole-3-carboxylate,
Ethyl 5-carbamoyl-4-{4-[3-(4-chloro-3-trifluoromethylphenyl)ureido]phenyl}-1H-pyrrole-3-carboxylate,
Ethyl 5-carbamoyl-4-{4-[3-(2-chloro-5-trifluoromethylphenyl)ureido]phenyl}-1H-pyrrole-3-carboxylate,
Ethyl 5-carbamoyl-4-{4-[3-(2-fluoro-3-trifluoromethylphenyl)ureido]phenyl}-1H-pyrrole-3-carboxylate,
Ethyl 5-carbamoyl-4-{4-[3-(4-fluoro-3-trifluoromethylphenyl)ureido]phenyl}-1H-pyrrole-3-carboxylate,
Ethyl 5-carbamoyl-4-{4-[3-(3-fluoro-5-trifluoromethylphenyl)ureido]phenyl}-1H-pyrrole-3-carboxylate,
Ethyl 5-carbamoyl-4-{4-[3-(4-methyl-3-trifluoromethylphenyl)ureido]phenyl}-1H-pyrrole-3-carboxylate,
Ethyl 5-carbamoyl-4-{4-[3-(4-trifluoromethoxyphenyl)ureido]phenyl}-1H-pyrrole-3-carboxylate,
Ethyl 5-carbamoyl-4-{4-[3-(4-difluoromethoxyphenyl)ureido]phenyl}-1H-pyrrole-3-carboxylate,
Ethyl 5-carbamoyl-4-{4-[3-(3,4-dimethylphenyl)ureido]phenyll-1H-pyrrole-3-carboxylate,
Ethyl 5-carbamoyl-4-{4-[3-(3,4-dimethoxyphenyl)ureido]phenyl}-1H-pyrrole-3-carboxylate,
Ethyl 5-carbamoyl-4-{4-[3-(3,5-dimethoxyphenyl)ureido]phenyl}-1H-pyrrole-3-carboxylate,
Ethyl 5-carbamoyl-4-{4-[3-(2,5-dimethylphenyl)ureido]phenyl}-1H-pyrrole-3-carboxylate,
Ethyl 5-carbamoyl-4-{4-[3-(2-methoxy-5-methylphenyl)ureido]phenyl}-1H-pyrrole-3-carboxylate,
Ethyl 5-carbamoyl-4-{4-[3-(2,5-dimethoxyphenyl)ureido]phenyl}-1H-pyrrole-3-carboxylate,
Ethyl 5-carbamoyl-4-{4-[3-(3-chloro-4-difluoromethoxyphenyl)ureido]phenyl}-1H-pyrrole-3-carboxylate,
Ethyl 5-carbamoyl-4-{4-[3-(3,5-dimethylphenyl)ureido]phenyl}-1H-pyrrole-3-carboxylate,
5-Carbamoyl-4-{4-[3-(2-fluoro-5-trifluoromethylphenyl)ureido]phenyl}-1H-pyrrole-3-carboxylic acid,
4-(2,4-Dimethoxybenzylamino)carbonyl-3-{4-[3-(2-fluoro-5-trifluoromethylphenyl)ureido]phenyl}-1H-pyrrole-2-carboxamide,
3-{4-[3-(2-Fluoro-5-trifluoromethylphenyl)ureido]phenyl}-1H-pyrrole-2,4-dicarboxamide,
3-{4-[3-(2-Fluoro-5-trifluoromethylphenyl)ureido]phenyl}-4-(4-methylpiperazine-1-carbonyl)-1H-pyrrole-2-carboxamide,
3-{4-[3-(2-Fluoro-5-trifluoromethylphenyl)ureido]phenyl}-4-(pyrrolidine-1-carbonyl)-1H-pyrrole-2-carboxamide,
2-Dimethylaminoethyl 5-carbamoyl-4-{4-[3-(2-fluoro-5-trifluoromethylphenyl)-ureido]phenyl}-1H-pyrrole-3-carboxylate,
4-Chloro-3-{4-[3-(2-fluoro-5-trifluoromethylphenyl)ureido]phenyl}-1H-pyrrole-2-carboxamide,
5-Chloro-3-{4-[3-(2-fluoro-5-trifluoromethylphenyl)ureido]phenyl}-1H-pyrrole-2-carboxamide,
5-{4-[3-(2-Fluoro-5-trifluoromethylphenyl)ureido]phenyl}-3H-imidazole-4-carboxamide.

10. Product according to any one of the preceding claims, **characterized in that** it is:
1) in achiral form, or
2) in racemic form, or
3) enriched in one stereoisomer, or
4) enriched in one enantiomer;
and **in that** it is optionally salified.

11. Medicament, **characterized in that** it comprises a product of formula (I) according to any one of Claims 1 to 9, or an addition salt of this compound with a pharmaceutically acceptable acid, or alternatively a hydrate or a solvate of the product of formula (I).

12. Pharmaceutical composition comprising a product according to any one of the preceding claims, in combination with a pharmaceutically acceptable excipient.

13. Use of a product according to any one of Claims 1 to 9, for the manufacture of a medicament that is useful for treating a pathological condition.

14. Use according to Claim 13, **characterized in that** the pathological condition is cancer.

## Patentansprüche

1. Produkt der folgenden Formel (I): 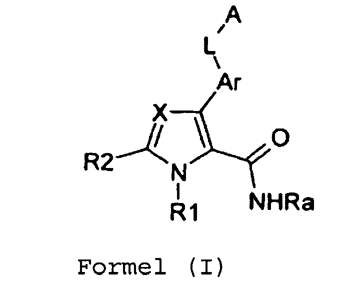 worin:
1) Ar-L-A Folgendes ist: 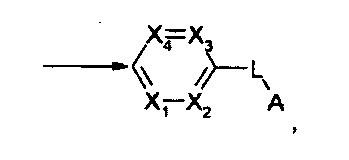 wobei X1, X2, X3 und X4 jeweils für N oder CH stehen;
2) L für NH-CO-NH steht;
3) A für gegebenenfalls substituiertes Phenyl, Pyrazol oder Isoxazolyl steht;
4) Ra für H steht;
5) R1 für H steht;
6) X aus CR3 und N ausgewählt ist;
7) R2 ein Wasserstoffatom ist
8) R3 aus der Gruppe ausgewählt wird, bestehend aus: H, Halogen, R'2, CN, O(R'2), OC(O)(R'2), OC(O)N(R'2)(R'3), OS(O₂)(R'2), N(R'2)(R'3), N=C(R'2)(R'3), N(R'2)C(O)(R'3), N(R'2)C(O)O(R'3), N(R'4)C(O)N(R'2)(R'3), N(R'4)C(S)N(R'2)(R'3), N(R'2)S(O₂)(R'3), C(O)(R'2), C(O)O(R'2), C(O)N(R'2)(R'3), C(=N(R'₃))(R'2)_{,} C(=N(OR'3))(R'2), S(R'2), S(O)(R'2), S(O₂)(R'2), S(O₂)O(R'₂), S(O₂)N(R'2)(R'3); worin R'2, R'3, R'4 jeweils unabhängig aus der Gruppe ausgewählt werden, die aus H, Alkyl, Alkylen, Alkinyl, Aryl, Heteroaryl, Cycloalkyl, Heterocyclyl, substituiertem Alkyl, substituiertem Alkylen, substituiertem Alkinyl, substituiertem Aryl, substituiertem Heteroaryl, substituiertem Cycloalkyl, substituiertem Heterocyclyl besteht; worin R'2 und R'3, wenn sie jeweils verschieden von H sind und gleichzeitig an R2 oder an R3 vorliegen, miteinander verbunden sein können und einen Ring bilden können, der 0 bis 3 Heteroatome enthält, die aus O, S und N ausgewählt sind; und **dadurch gekennzeichnet, dass** mindestens einer der Reste R2 oder R3 ein Halogen ist.

2. Produkt nach Anspruch 1, **dadurch gekennzeichnet, dass** X für CR3 steht.

3. Produkt nach einem der Ansprüche 1 bis 2, **dadurch gekennzeichnet, dass** R3 aus der Gruppe ausgewählt wird, bestehend aus H, Halogen, NH₂, N(R'2)C(O) (R'3), N(R'2)C(O)O(R'3), N(R'4)C(O)N(R'2) (R'3), C(O)O(R'2), C(O)N(R'2)(R'3); worin R'2, R'3 jeweils unabhängig aus der Gruppe ausgewählt werden, die aus H, Alkyl, Alkylen, Alkinyl, Aryl, Heteroaryl, Cycloalkyl, Heterocyclyl, substituiertem Alkyl, substituiertem Alkylen, substituiertem Alkinyl, substituiertem Aryl, substituiertem Heteroaryl, substituiertem Cycloalkyl, substituiertem Heterocyclyl besteht; worin R'2 und R'3, wenn sie jeweils verschieden von H sind und gleichzeitig an R2 oder an R3 vorliegen, miteinander verbunden sein können und einen Ring bilden können, der von 0 bis 3 Heteroatome enthält, die aus O, S und N ausgewählt sind.

4. Produkt nach Anspruch 3, **dadurch gekennzeichnet, dass** R3 aus der Gruppe ausgewählt wird, bestehend aus H, Halogen, NH₂, NH-COO-Alkyl, -NH-CO-NH-Aryl, wobei Aryl unsubstituiert oder mit einem oder mehreren Halogen oder Halogenalkyl substituiert ist, -NH-CO-Alkyl, -NH-CO-Alkyl-N(alkyl)(alkyl'), -COOH, -COO-Alkyl, -COO-Alkyl-N(alkyl)(alkyl'), -CO-NH-Alkyl-aryl, wobei Aryl unsubstituiert oder mit einem oder mehreren Alkoxy substituiert ist, -CONH₂, -CO-Heterocyclyl, wobei Heterocyclyl unsubstituiert oder mit einem oder mehreren Alkylen substituiert ist.

5. Produkt nach Anspruch 1, worin A mit einem ersten Substituenten substituiert ist, der aus der Gruppe ausgewählt wird, bestehend aus Alkyl, Halogenalkyl, Alkylen, Alkinyl, Aryl, Heteroaryl, O-Alkyl, O-Aryl, O-Heteroaryl, S-Alkyl, substituiertem S-Alkyl, S-Aryl, S-Heteroaryl, die jeweils gegebenenfalls mit einem Substituenten substituiert sind, ausgewählt aus (C1-C3)-Alkyl, Halogen, O-(C1-C3)-Alkyl.

6. Produkt nach Anspruch 5, **dadurch gekennzeichnet, dass** A mit einem zweiten Substituenten substituiert ist, der aus der Gruppe gewählt wird, bestehend aus F, Cl, Br, I, OH, SH, SO₃M, COOM, CN, NO₂, CON(R8)(R9), N(R8)CO(R9), (C1-C3)Alkyl-OH, (Cl-C3)Alkyl-N(R8)(R9), (C1-C3)Alkyl-(R10), (C1-C3)Alkyl-COOH, N(R8)(R9); wobei R8 und R9 unabhängig ausgewählt sind aus H, (C1-C3)Alkyl, (C1-C3)Halogenalkyl, (C1-C3)AlkylOH, (C1-C3)AlkylNH₂, (C1-C3)AlkylCOOM, (C1-C3)AlkylSO₃M; worin R8 und R9, wenn sie gleichzeitig verschieden von H sind, miteinander verbunden sein können und einen Ring mit 5 bis 7 Gliedern bilden können, der 0 bis 3 Heteroatome, ausgewählt aus N, O und S, enthält; worin M für H oder ein Alkalimetallkation steht, das ausgewählt wird aus Li, Na und K; und worin R10 für H oder einen gegebenenfalls substituierten, nicht aromatischen Heterocyclus steht, der 2 bis 7 Kohlenstoffatome und 1 bis 3 Heteroatome, ausgewählt aus N, O und S, umfasst.

7. Produkt nach einem der Ansprüche 5 bis 6, **dadurch gekennzeichnet, dass** A für Phenyl steht, das mit Halogen, (C1-C4)Alkyl, (C1-C3)Halogenalkyl, O-(C1-C4)Alkyl, S-(C1-C4)Alkyl, O-(C1-C4)Halogenalkyl und S-(C1-C4)Halogenalkyl substituiert ist, und wenn A disubstituiert ist, die beiden Substituenten miteinander verbunden sein können und einen Ring mit 5 bis 7 Gliedern bilden können, der 0 bis 3 Heteroatome enthält, die aus O, N und S ausgewählt werden.

8. Produkt nach einem der Ansprüche 5 bis 7, **dadurch gekennzeichnet, dass** A mit einem oder mehreren identischen oder verschiedenen Substituenten substituiert ist, die unabhängig aus der Gruppe ausgewählt werden, bestehend aus F, Cl, Br, I, OH, SH, SO₃M, COOM, CN, NO₂, CON(R8) (R9), N(R8)CO(R9), (C1-C3)Alkyl-OH, (C1-C3)Alkyl-N(R8)(R9), (C1-C3)Alkyl-(R10), (C1-C3)Alkyl-COOH, N(R8)(R9), (C1-C6)Alkyl, (C2-C6)Alkylen, (C2-C6)Alkinyl, Aryl, Heteroaryl, O-(C1-C6)Alkyl, O-Aryl, O-Heteroaryl, S-(C1-C6)Alkyl, S-Aryl, S-Heteroaryl, die jeweils gegebenenfalls mit einem oder mehreren Substituenten substituiert sind, die ausgewählt werden aus (C1-C3)Alkyl, Halogen, O-(C1-C3)Alkyl; worin R8 und R9 unabhängig ausgewählt sind aus H, (C1-C3)Alkyl, (C1-C3)AlkylOH, (C1-C3)AlkylNH₂, (C1-C3)AlkylCOOM, (C1-C3)AlkylSO₃M; wobei R8 und R9, wenn sie gleichzeitig verschieden von H sind, miteinander verbunden sein können und einen Ring mit 5 bis 7 Gliedern bilden können, der 0 bis 3 Heteroatome, ausgewählt aus O, N und S, enthält; wobei M für H oder ein Alkalimetallkation steht, das ausgewählt wird aus Li, Na und K; und wobei R10 für H oder einen gegebenenfalls substituierten, nicht aromatischen Heterocyclus steht, der 2 bis 7 Kohlenstoffatome und 1 bis 3 Heteroatome, ausgewählt aus N, O und S, umfasst.

9. Produkt nach Anspruch 1, **dadurch gekennzeichnet, dass** es aus Folgenden ausgewählt wird:
3-{4-[3-(2-Fluor-5-trifluormethylphenyl)ureido]-phenyl}-1H-pyrrol-2-carboxamid,
4-tert.-Butyloxycarbonylamino-3-{4-[3-(2-fluor-5-trifluormethylphenyl)ureido]phenyl}-1H-pyrrol-2-carboxamid,
4-Amino-3-{4-[3-(2-fluor-5-trifluormethylphenyl)ureido]phenyl}-1H-pyrrol-2-carboxamid-hydrochlorid,
4-[3-(2-Fluor-5-trifluormethylphenyl)ureido]-3-{4-[3-(2-fluor-5-trifluormethylphenyl)ureido]phenyl}-1H-pyrrol-2-carboxamid,
4-Acetylamino-3-{4-[3-(2-fluor-5-trifluormethylphenyl)ureido]phenyl}-1H-pyrrol-2-carboxamid,
4-(2-Dimethylaminoacetylamino)-3-{4-[3-(2-fluor-5-trifluormethylphenyl)ureido]phenyl}-1H-pyrrol-2-carboxamid,
4-Benzoylamino-3-{4-[3-(2-fluor-5-trifluormethylphenyl)ureido]phenyl}-1H-pyrrol-2-carboxamid,
4-Nicotinoylamino-3-{4-[3-(2-fluor-5-trifluormethylphenyl)ureido]phenyl}-1H-pyrrol-2-carboxamid,
5-Carbamoyl-4-{4-[3-(2-fluor-5-trifluormethylphenyl)ureido]phenyl}-1H-pyrrol-3-ethylcarboxylat,
5-Carbamoyl-4-{4-[3-(2-fluorphenyl)ureido]-phenyl}-1H-pyrrol-3-ethylcarboxylat,
5-Carbamoyl-4-{4-[3-(2-methoxyphenyl)ureido]-phenyl}-1H-pyrrol-3-ethylcarboxylat,
5-Carbamoyl-4-{4-[3-(2-trifluormethylphenyl)-ureido]-phenyl}-1H-pyrrol-3-ethylcarboxylat,
5-Carbamoyl-4-[4-(3-o-tolylureido)phenyl]-1H-pyrrol-3-ethylcarboxylat,
5-Carbamoyl-4-{4-[3-(3-fluorphenyl)ureido]-phenyl}-1H-pyrrol-3-ethylcarboxylat,
5-Carbamoyl-4-{4-[3-(3-methoxyphenyl)ureido]phenyl}-1H-pyrrol-3-ethylcarboxylat,
5-Carbamoyl-4-{4-[3-(3-trifluormethylphenyl)-ureido]phenyl}-1H-pyrrol-3-ethylcarboxylat,
5-Carbamoyl-4-[4-(3-m-tolylureido)phenyl]-1H-pyrrol-3-ethylcarboxylat,
5-Carbamoyl-4-{4-[3-(4-fluorphenyl)ureido]-phenyl}-1H-pyrrol-3-ethylcarboxylat,
5-Carbamoyl-4-{4-[3-(4-methoxyphenyl)ureido]-phenyl}-1H-pyrrol-3-ethylcarboxylat,
5-Carbamoyl-4-{4-[3-(4-trifluormethylphenyl)-ureido]phenyl}-1H-pyrrol-3-ethylcarboxylat,
5-Carbamoyl-4-[4-(3-p-tolylureido)phenyl]-1H-pyrrol-3-ethylcarboxylat,
5-Carbamoyl-4-{4-[3-(4-chlor-3-trifluormethylphenyl)ureido]phenyl}-1H-pyrrol-3-ethylcarboxylat,
5-Carbamoyl-4-{4-[3-(2-chlor-5-trifluormethylphenyl)ureido]phenyl}-1H-pyrrol-3-ethylcarboxylat,
5-Carbamoyl-4-{4-[3-(2-fluor-3-trifluormethylphenyl)ureido]phenyl}-1H-pyrrol-3-ethylcarboxylat,
5-Carbamoyl-4-{4-[3-(4-fluor-3-trifluormethylphenyl)ureido]phenyl}-1H-pyrrol-3-ethylcarboxylat,
5-Carbamoyl-4-{4-[3-(3-fluor-5-trifluormethylphenyl)ureido]phenyl}-1H-pyrrol-3-ethylcarboxylat,
5-Carbamoyl-4-{4-[3-(4-methyl-3-trifluormethylphenyl)ureido]phenyl}-1H-pyrrol-3-ethylcarboxylat,
5-Carbamoyl-4-{4-[3-(4-trifluormethoxyphenyl)-ureido]phenyl}-1H-pyrrol-3-ethylcarboxylat,
5-Carbamoyl-4-{4-[3-(4-difluormethoxyphenyl)-ureido]phenyl}-1H-pyrrol-3-ethylcarboxylat,
5-Carbamoyl-4-{4-[3-(3,4-dimethylphenyl)ureido]-phenyl}-1H-pyrrol-3-ethylcarboxylat,
5-Carbamoyl-4-{4-[3-(3,4-dimethoxyphenyl)ureido]-phenyl}-1H-pyrrol-3-ethylcarboxylat,
5-Carbamoyl-4-{4-[3-(3,5-dimethoxyphenyl)ureido]-phenyl}-1H-pyrrol-3-ethylcarboxylat,
5-Carbamoyl-4-{4-[3-(2,5-dimethylphenyl)ureido]-phenyl}-1H-pyrrol-3-ethylcarboxylat,
5-Carbamoyl-4-{4-[3-(2-methoxy-5-methylphenyl)-ureido]phenyl}-1H-pyrrol-3-ethylcarboxylat,
5-Carbamoyl-4-{4-[3-(2,5-dimethoxyphenyl)ureido]phenyl}-1H-pyrrol-3-ethylcarboxylat,
5-Carbamoyl-4-{4-[3-(3-chlor-4-difluormethoxyphenyl)ureido]phenyl}-1H-pyrrol-3-ethylcarboxylat,
5-Carbamoyl-4-{4-[3-(3,5-dimethylphenyl)ureido]-phenyl}-1H-pyrrol-3-ethylcarboxylat,
5-Carbamoyl-4-{4-[3-(2-fluor-5-trifluormethylphenyl)ureido]phenyl}-1H-pyrrol-3-carbonsäure,
4-(2,4-Dimethoxybenzylamino)carbonyl-3-{4-[3-(2-fluor-5-trifluormethylphenyl)ureido]phenyl}-1H-pyrrol-2-carboxamid,
3-{4-[3-(2-Fluor-5-trifluormethylphenyl)ureido]-phenyl}-1H-pyrrol-2,4-dicarboxamid,
3-{4-[3-(2-Fluor-5-trifluormethylphenyl)ureido] - phenyl}-4-(4-methylpiperazin-1-carbonyl)-1H-pyrrol-2-carboxamid,
3-{4-[3-(2-Fluor-5-trifluormethylphenyl)ureido]-phenyl}-4-(pyrrolidin-1-carbonyl)-1H-pyrrol-2-carboxamid,
5-Carbamoyl-4-{4-[3-(2-fluor-5-trifluormethylphenyl)ureido]phenyl}-1H-pyrrol-3-(2-dimethylaminoethyl)carboxylat,
4-Chlor-3-{4-[3-(2-fluor-5-trifluormethylphenyl)ureido]phenyl}-1H-pyrrol-2-carboxamid,
5-Chlor-3-{4-[3-(2-fluor-5-trifluormethylphenyl)ureido]phenyl}-1H-pyrrol-2-carboxamid und
5-{4-[3-(2-Fluor-5-trifluormethylphenyl)ureido]-phenyl}-3H-imidazol-4-carboxamid.

10. Produkt nach einem der vorhergehenden Ansprüche, **dadurch gekennzeichnet, dass** es in folgender Form vorliegt:
1) in der achiralen Form oder
2) in der racemischen Form oder
3) an einem Stereoisomer angereichert oder
4) an einem Enantiomer angereichert;
und **dadurch**, dass es gegebenenfalls versalzt ist.

11. Arzneimittel, **dadurch gekennzeichnet, dass** es ein Produkt der Formel (I) nach einem der Ansprüche 1 bis 9 oder ein Additionssalz dieser Verbindung mit einer pharmazeutisch annehmbaren Säure oder auch ein Hydrat oder ein Solvat des Produkts der Formel (I) umfasst.

12. Pharmazeutische Zusammensetzung, die ein Produkt nach einem der vorhergehenden Ansprüche in Kombination mit einem pharmazeutisch annehmbaren Excipienten umfasst.

13. Verwendung eines Produkts nach einem der Ansprüche 1 bis 9 zur Herstellung eines Arzneimittels, das zur Behandlung eines pathologischen Zustands geeignet ist.

14. Verwendung nach Anspruch 13, **dadurch gekennzeichnet, dass** der pathologische Zustand Krebs ist.
